(19) Europäisches Patentamt
European Patent Office
Office européen des brevets

(11) EP 2 968 131 B1

(12) **EUROPEAN PATENT SPECIFICATION**

(45) Date of publication and mention of the grant of the patent:
**27.05.2020 Bulletin 2020/22**

(21) Application number: **14770805.1**

(22) Date of filing: **14.03.2014**

(51) Int Cl.:
***A61L 27/52*** *(2006.01)* ***A61L 27/18*** *(2006.01)*
***A61L 27/54*** *(2006.01)* ***A61L 26/00*** *(2006.01)*

(86) International application number:
**PCT/US2014/028798**

(87) International publication number:
**WO 2014/153038 (25.09.2014 Gazette 2014/39)**

(54) **BIOCOMPATIBLE HYDROGEL POLYMER MATRIX FOR DELIVERY OF CELLS**

BIOKOMPATIBLE HYDROGELPOLYMERMATRIX ZUR VERABREICHUNG VON ZELLEN

MATRICE POLYMÈRE D'HYDROGEL BIOCOMPATIBLE POUR L'ADMINISTRATION DE CELLULES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **14.03.2013 US 201361785477 P**

(43) Date of publication of application:
**20.01.2016 Bulletin 2016/03**

(73) Proprietor: **C.P. Medical Corporation**
**Norcross, Georgia 30093 (US)**

(72) Inventors:
- **ASKARI, Syed, H.**
  **San Jose, CA 95129 (US)**
- **HORNG, George**
  **Millbrae, CA 94030 (US)**

(74) Representative: **Bassil, Nicholas Charles**
**Kilburn & Strode LLP**
**Lacon London**
**84 Theobalds Road**
**London WC1X 8NL (GB)**

(56) References cited:
**WO-A1-2011/057131**
**WO-A2-2011/140519**
**WO-A2-2011/140519**
**US-A1- 2001 003 126**
**US-A1- 2001 003 126**
**US-A1- 2008 279 944**
**US-A1- 2009 196 928**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### CROSS-REFERENCE

### BACKGROUND OF THE INVENTION

Cell based therapies are important options for the treatment of clinical indications including diseases, tissue damage, neurological disorders, blood disorders, cancers, developmental defects, wounds and orthopedic impediments. Many cell based therapies are target specific, with cells being administered directly to a target site. When cells are not suitably retained at a target site after administration, there is both a loss of cells available for the intended treatment as well as an increased risk of cell differentiation at an alternative site. When cells are administered to a target site without sufficient protection, the cells may go through physiochemical changes such as hypertrophy, necrosis, apoptosis, or senescence. Treatment efficacy is attenuated when the administered cells are physiochemically altered or not retained at the desired target site. WO 2011/140519 describes an in vivo gelling pre-formulation and resulting biocompatible hydrogel. US 2001/003125 describes a biocompatible hydrogel for cell delivery.

### SUMMARY OF THE INVENTION

The aspects of the invention for which protection is sought are defined by the claims.

In a first aspect, provided herein is a polyglycol-based biocompatible hydrogel polymer matrix comprising a hydrogel polymer comprising at least one first monomeric unit bound through at least one amide linkage to at least one second monomeric unit, wherein the polymer forms the matrix that encapsulates:

(a) at least one cell; and
(b) a culture medium which supports the growth of the at least one cell;

wherein the hydrogel polymer is derived from the first monomeric unit of 8ARM PEG Acetate Amine (8ARM-20k-AA) and 8ARM PEG Amine (8ARM-20k-NH2); the second monomeric unit of 4ARM PEG Succinimidyl Glutaramide (4ARM-20k-SGA);
a phosphate buffer and a viscosity enhancing agent;
wherein the fully synthetic, polyglycol-based biocompatible hydrogel polymer matrix provides controlled release of the at least one cell, when implanted at a target site in an animal’s body, to the target site of the animal’s body.

In some embodiments according to the first aspect, the 8ARM PEG Acetate Amine may be 8ARM PEG Acetate Amine (hexaglycerol) HCl Salt, MW 20000 or 8ARM PEG Acetate Amine (hexaglycerol) TFA Salt, MW 20000. The cell may be selected from a mammalian cell, insect cell, protozoal cell, bacterial cell, viral cell, or fungal cell; and optionally, the mammalian cell may be a stem cell. The culture medium may comprise a growth factor. The ratio of 8ARM-20k-AA to 8ARM-20k-NH2 maybe 1:1, 70:30, or 75:25. The animal may be a human. The polyglycol-based biocompatible hydrogel polymer matrix may be bioabsorbed within about 14 to 180 days. The controlled release of the at least one cell to the target site of the animal’s body may comprise diffusion of the at least one cell from the polyglycol-based biocompatible hydrogel polymer matrix; or the controlled release of the at least one cell to the target site of the animal’s body is at least partially through degradation and bioabsorption of the polyglycol-based biocompatible hydrogel polymer matrix. The viscosity enhancing agent may be HPMC.

In a second aspect, provided herein is a fully synthetic polyglycol-based biocompatible pre-formulation, comprising:

(a) at least one fully synthetic polyglycol-based first composition comprising 8ARM PEG Acetate Amine (8ARM-20k-AA) and 8ARM PEG Amine (8ARM-20k-NH2);
(b) at least one fully synthetic polyglycol-based second composition comprising 4ARM PEG Succinimidyl Glutaramide (4ARM-20k-SGA);
(c) a phosphate buffer;
(d) a viscosity enhancing agent;
(e) at least one cell; and
(f) a culture medium that supports growth of the at least one cell;

wherein the polyglycol-based biocompatible pre-formulation at least in part polymerizes and/or gels to form a polyglycol-based biocompatible hydrogel polymer matrix encapsulating the cell in the presence of water.

In some embodiments according to the second aspect, the cell may be a mammalian cell or stem cell; and optionally, the culture medium may comprise a buffer. The polyglycol-based biocompatible pre-formulation may gel to

form a polyglycol-based biocompatible hydrogel polymer matrix in between about 20 seconds and 10 minutes. The viscosity enhancing agent may be HPMC.

In a third aspect, provided herein is a fully synthetic, polyglycol based biocompatible hydrogel polymer matrix according to the first aspect, for use in a method of treating a disease.

## BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:

**Figure 1** shows the effect of addition of degradable acetate amine 8ARM-20k-AA or 4ARM-20k-AA on degradation times. Degradations occurred in phosphate buffered saline (PBS) at 37°C.

**Figure 2** shows the effect of polymer concentration on degradation time for 75% Acetate Amine formulation and 100% Acetate Amine formulation.

**Figure 3** shows the effect of a polymer left in the air as the percent of water weight loss over time.

**Figure 4** shows a sample plot generated by the Texture Analyzer Exponent software running the firmness test. The peak force was recorded as the polymer firmness, which represents the point where the target penetration depth of 4 mm has been reached by the probe.

**Figure 5** shows a sample plot generated by the Texture Analyzer Exponent software running the elastic modulus test under compression. The modulus was calculated from the initial slope of the curve up to 10% of the maximum compression stress.

**Figure 6** shows an exemplary plot generated by the Texture Analyzer Exponent software running the adhesion test. A contact force of 100.0 g was applied for 10 seconds. The tack was measured as the peak force after lifting the probe from the sample. The adhesion energy or the work of adhesion was calculated as the area under the curve representing the tack force (points 1 to 2). The stringiness was defined as the distance traveled by the probe while influencing the tack force (points 1 and 2).

**Figure 7** shows the firmness vs. degradation time plotted as percentages for the polymer formulation: 8ARM-20k-AA/8ARM-20k-NH2 (70/30) & 4ARM-20k-SGA at 4.8% solution with 0.3% HPMC. The error bars represent the standard deviations of 3 samples. The degradation time for the polymer was 18 days.

**Figure 8** shows the chlorhexidine cumulative % elution.

**Figure 9** shows that for a polymer, the triamcinolone cumulative % elution for 60, 90 and 240 day polymers.

**Figure 10** shows that for short degradation time version of the hydrogel polymer loaded with Depo-Medrol, the methylprednisolone cumulative % elution.

**Figure 11** shows that for long degradation time version of a polymer loaded with Depo-Medrol, the methylprednisolone cumulative % elution.

**Figure 12** shows the effect of solid phosphate powder concentration on polymer gel time **(A)** and solution pH **(B).**

**Figure 13** shows the effect of sterilization on gel times for polymers of various concentrations **(A)** and **(B).**

**Figure 14** shows the storage stability of kits at 5°C, 20°C and 37°C.

## DETAILED DESCRIPTION OF THE INVENTION

Cell therapy is used for many clinical indications in multiple target sites and by several modes of cell delivery. Cells are directed to a target site via local or systemic administration. An important example of cell therapy comprises the steps of stem cell engraftment, cell differentiation, and replacement of damaged tissue, whereby the target tissue has improved function. Cell based therapies can be administered to damaged tissue following events such as myocardial infarction, infection or cancer treatment. During and after administration of a cell based therapy to a target site, the therapeutic cells may not be sufficiently protected. Cells which are not protected may undergo apoptosis, necrosis, hypertrophy, or senescence; thereby diminishing the efficacy of treatment. Cell based therapies which are delivered in a suitable environment allow for cell survival. In some instances, cells delivered in a suitable environment proliferate, differentiate, and integrate with target tissue. Cell based therapies which are retained in a suitable environment at the target site allow for proper cell functionality at the target site.

Cell based treatments administered systemically expose many regions in the body to the administered cells in addition to the target site. Diffusion of stem cells away from the target site increases the risk of undesired cell differentiation and subsequent complications. Importantly, the loss of cell retention at the target site increases the amount of administered cells necessary for therapeutic efficacy. Localized cell delivery directly to a target site limits exposure of the administered cells to the areas surrounding the target site. Localized cell delivery and cell retention enables the administration of a controlled therapeutic dose. In some instances, a therapeutic dose is controlled by extended release of the cells. In some instances, localized cell delivery treatments are more effective because dosages can be increased with less concern for adverse side effects. In further instances, extended release of the cells also reduces the number of doses necessary in the course of treatment.

A biocompatible pre-formulation to form a biocompatible hydrogel polymer matrix enables the administration and retention of cells directly to target sites. The biocompatible pre-formulation at least in part polymerizes and/or gels to form the biocompatible hydrogel polymer matrix. The biocompatible hydrogel polymer matrix comprises at least one cell. In some instances, the biocompatible hydrogel matrix comprises a biocompatible hydrogel scaffold. The biocompatible hydrogel polymer matrix provides structural and nutritional support for the cells after administration of the polymer matrix or pre-formulation to a target site. In some instances, the biocompatible hydrogel scaffold provides structural and nutritional support for the cells after administration of the polymer matrix or pre-formulation to a target site. In certain instances, the biocompatible hydrogel polymer matrix provides structural and nutritional support for the cells after administration of the polymer matrix or pre-formulation to a target site. The biocompatible hydrogel polymer matrix enables the cells to be retained at a target site for a pre-determined amount of time. In certain instances, the biocompatible hydrogel polymer matrix provides a protective and nutrient rich environment suitable for cell survival, growth or proliferation. In certain instances, the biocompatible hydrogel polymer matrix provides a protective and nutrient rich environment suitable for cell survival, proliferation, differentiation, and tissue integration. A biocompatible pre-formulation to form a biocompatible hydrogel polymer matrix further enables the controlled release of cells at target sites. In certain instances, the controlled release of cells at target sites is through diffusion, degradation of the biocompatible hydrogel polymer matrix, or any combination thereof. In some instances, the hydrogel polymer matrix is biodegradable. In certain instances, delivery, retention, and controlled release of the cells using a biocompatible hydrogel polymer matrix minimizes cell hypertrophy, senescence, apoptosis, and necrosis. In some instances, the biocompatible hydrogel polymer matrix protects the cells from the enzymes and pH conditions of the gastrointestinal tract. In some instances, the polymer matrix is configured to maintain the physiochemical properties of the cells during administration, retention, biocompatible hydrogel polymer matrix degradation, or release of the cells to the target site. In some instances, the cells remain viable during and after polymerization of the biocompatible hydrogel polymer matrix. In some instances, the cells remain viable when added to an already polymerized and/or gelled biocompatible hydrogel polymer matrix. In some instances, the cells remain viable when administered. In some instances, the cells remain viable after delivery to a target site. In some instances, the cells remain viable during release from the biocompatible hydrogel polymer matrix. In some instances, the cells remain viable during degradation of the biocompatible hydrogel polymer matrix.

A biocompatible hydrogel polymer matrix enables the delivery of cells to a target site where the cells will eventually be released from the polymer matrix by diffusion, polymer matrix degradation or any combination thereof. In some instances, polymer matrix degradation times are controlled by varying the composition of the biocompatible pre-formulation components allowing for the appropriate application and placement of the biocompatible hydrogel polymer matrix. In some instances, polymer matrix degradation times are controlled by varying the pH of the pre-formulation allowing for the appropriate application and placement of the biocompatible hydrogel polymer matrix. In some instances, polymer matrix degradation times are controlled by varying the concentrations of the biocompatible pre-formulation components allowing for the appropriate application and placement of the biocompatible hydrogel polymer matrix. In some instances, the cells are released from the biocompatible hydrogel polymer matrix in a precise and consistent manner. In certain instances, the biocompatible hydrogel polymer matrix is bioabsorbed over a defined period of time. In some instances, the biocompatible hydrogel polymer matrix provides the sustained release of cells at a target site. In certain instances, the sustained and controlled release reduces the systemic exposure to the cells. In certain instances, the controlled release allows for cell retention at a target site. In some instances, the cells are released from the biocompatible hydrogel polymer matrix over an extended period of time. In certain instances, delivery of the cells in a biocompatible hydrogel polymer matrix provides a depot of the cells (e.g., under the skin), wherein the depot releases the cells over an extended period of time (e.g., 1 day, 2 days, 3 days, 4 days, 5 days, 6 days, 7 days, 10, days, 14 days, 3 weeks, 4 weeks). In some instances, the biocompatible hydrogel polymer matrix releases the cells after a delay as a delayed burst.

The biocompatible hydrogel polymer matrix comprising at least one cell may start out as a liquid biocompatible pre-formulation which is delivered to a target site using minimally invasive techniques. The initial liquid state allows the formulation to be delivered through small catheters directed by endoscopes or other image guided techniques to the target site (e.g., bronchoscope for lung, thoracoscope for the chest cavity, laparoscope for the abdominal cavity, cystoscope for the bladder, arthroscope for joint space, etc.). Once in the body, the liquid formulation polymerizes into a biocompatible hydrogel polymer matrix. In some instances, the biocompatible hydrogel polymer matrix adheres to the tissue and the at least one cell is maintained at the target site. In some instances, the biocompatible hydrogel polymer matrix is delivered to a target site after polymerization. In some instances, polymerization times are controlled by varying the composition of the biocompatible pre-formulation components allowing for the appropriate application and placement of the biocompatible hydrogel polymer matrix. The controlled gelling allows the use of the biocompatible hydrogel polymer

matrix to deliver at least one cell directly to the affected target tissue, thereby minimizing systemic exposure. In some instances, the biocompatible hydrogel polymer matrix may polymerize outside the body. In certain instances, exposure to the cells is limited to the tissue around the target site. In some instances, the patient is not exposed systemically to the cell therapy. In certain instances, the biocompatible pre-formulation allows the cells to remain viable during and after polymerization. In some instances, the cells are combined with a biocompatible hydrogel polymer matrix after polymerization and/or gel formation. In some instances, the biocompatible hydrogel polymer matrix further polymerizes and/or gels after delivery to a target site.

Cells may also be administered via a biocompatible hydrogel polymer matrix directly on a wound or surgical site. Biocompatible pre-formulations may form a biocompatible hydrogel polymer matrix that is easily applied on the wound or surgical site and the surrounding skin. The biocompatible hydrogel polymer matrix enables the administration of cells directly to the wound or surgical site. Biocompatible pre-formulations may polymerize and/or gel prior to or after application to the wound or surgical site. In some instances, once the biocompatible pre-formulation is applied, e.g., sprayed over the wound or surgical site, in the liquid form, the biocompatible pre-formulation gels quickly and forms a solid biocompatible hydrogel polymer matrix layer over the wound or surgical site. The biocompatible hydrogel polymer matrix seals the wound or surgical site and it also sticks to the surrounding skin. The biocompatible hydrogel polymer matrix layer over the wound or surgical site acts as a barrier to keep the wound or surgical site from getting infected. In some instances, the biocompatible hydrogel polymer matrix layer in contact with the skin makes the skin surface sticky and thus allows a bandage to stick to the skin more effectively. Most importantly, the biocompatible hydrogel polymer matrix is non-toxic. After healing has taken place, the biocompatible hydrogel polymer matrix dissolves and is absorbed without producing toxic by-products. In some instances, the wound or surgical site is healed by the formation of a graft after the administration of stem cells with a biocompatible hydrogel polymer matrix. In certain instances, the biocompatible pre-formulation is applied to a wound or surgical site without the cells losing viability. In certain instances, the biocompatible hydrogel polymer matrix keeps the wound or surgical site sealed for 24-48 hours and protects it from infection, which avoids repeat visits to the hospital and thus saving costs. In certain instances, exposure to the cells is limited to the tissue around the target site. In some instances, the patient is not exposed systemically to the cell therapy.

In some instances, the biocompatible hydrogel polymer matrix is also loaded with one or more additional components, such as a buffer or a therapeutic agent. The physical and chemical nature of the biocompatible hydrogel polymer matrix is such that a large variety of cell types and additional components may be used with the biocompatible pre-formulation that forms the biocompatible hydrogel polymer matrix. In some instances, the additional components enhance the viability and functionality of the cells. In some instances, the additional components comprise activation factors. In some instances the activation factors include growth factors for cell growth stimulation and proliferation.

## ***Exemplary Biocompatible Hydrogel Components***

Provided herein are biocompatible pre-formulations, comprising at least one first compound comprising more than one nucleophilic group, at least one second compound comprising more than one electrophilic group, at least one cell, and optionally additional components. An exemplary additional component is a culture medium. In certain instances, the culture medium is a buffer. In certain instances the culture medium contains nutrients for the at least one cell. In certain instances the at least one cell is a stem cell. In certain instances, the at least one first compound is formulated in a buffer. In certain instances, the at least one second compound is formulated in a buffer. In certain instances, the at least one cell is formulated in a buffer. In certain instances, at least one biocompatible pre-formulation component is a solid. In certain instances, all components of the biocompatible pre-formulations are solids. In certain instances, at least one biocompatible pre-formulation component is a liquid. In certain instances, all biocompatible pre-formulation components are liquids. In certain instances, the biocompatible pre-formulation components form a biocompatible hydrogel polymer matrix at a target site by mixing the at least one first compound, the at least one second compound, the at least one cell, and the optional additional component in the presence of water and delivering the mixture to the target site such that the biocompatible hydrogel polymer matrix at least in part polymerizes and/or gels at the target site. In certain instances, the biocompatible pre-formulation forms a biocompatible hydrogel polymer matrix at a target site by mixing the at least one first compound, the at least one second compound, and the at least one cell in the presence of water and delivering the mixture to the target site such that the biocompatible hydrogel polymer matrix at least in part polymerizes and/or gels at the target site. In certain instances, the optional additional component, e.g. buffer, is added after the formulation is combined. In certain instances, the biocompatible pre-formulation forms a biocompatible hydrogel polymer matrix prior to application at a target site by mixing the at least one first compound, the at least one second compound, the at least one cell, and the optional additional component in the presence of water and delivering the mixture to the target site such that the biocompatible hydrogel polymer matrix at least in part polymerizes and/or gels prior to application at a target site. In certain instances, the biocompatible pre-formulation forms a biocompatible hydrogel polymer matrix prior to application at a target site by mixing the at least one first compound, the at least one second compound, and the at least one cell in the presence of water and delivering the mixture to the target site such that the biocompatible

hydrogel polymer matrix at least in part polymerizes and/or gels prior to application at a target site. In certain instances, the optional additional component, e.g. buffer, is added after the formulation is combined. In certain instances, the biocompatible pre-formulations are biodegradable. In certain instances, the biocompatible hydrogel polymer matrix comprises a biocompatible hydrogel scaffold. In certain instances, the biocompatible hydrogel scaffold comprises the at least one first compound and the at least one second compound. In certain instances, the biocompatible hydrogel scaffold comprises the at least one first compound, the at least one second compound and a buffer. In certain instances, the biocompatible hydrogel scaffold is fully synthetic.

Provided herein are biocompatible pre-formulations, comprising at least one first compound comprising more than one nucleophilic group, at least one second compound comprising more than one electrophilic group, a buffer, and optionally additional components. An exemplary additional component is at least one cell. In certain embodiments the cell is a stem cell. In certain instances, the buffer is a culture medium. In certain instances the culture medium provides nutrients to a cell. In certain instances, the at least one first compound is formulated in a buffer. In certain instances, the at least one second compound is formulated in a buffer. In certain instances, at least one biocompatible pre-formulation component is a solid. In certain instances, all biocompatible pre-formulations are solids. In certain instances, at least one biocompatible pre-formulation component is a liquid. In certain instances, all biocompatible pre-formulation components are liquids. In certain instances, the biocompatible pre-formulation forms a biocompatible hydrogel polymer matrix at a target site by mixing the at least one first compound, the at least one second compound, the buffer, and the optional additional component in the presence of water and delivering the mixture to the target site such that the biocompatible hydrogel polymer matrix at least in part polymerizes and/or gels at the target site. In certain instances, the biocompatible pre-formulation forms a biocompatible hydrogel polymer matrix at a target site by mixing the at least one first compound, the at least one second compound, and the buffer in the presence of water and delivering the mixture to the target site such that the biocompatible hydrogel polymer matrix at least in part polymerizes and/or gels at the target site. In certain instances, the optional additional component, e.g. cell, is added after the formulation is combined. In certain instances, the biocompatible pre-formulation forms a biocompatible hydrogel polymer matrix prior to application at a target site by mixing the at least one first compound, the at least one second compound, the buffer, and the optional additional component in the presence of water and delivering the mixture to the target site such that the biocompatible hydrogel polymer matrix at least in part polymerizes and/or gels prior to application at a target site. In certain instances, the biocompatible pre-formulation forms a biocompatible hydrogel polymer matrix prior to application at a target site by mixing the at least one first compound, the at least one second compound, and the buffer in the presence of water and delivering the mixture to the target site such that the biocompatible hydrogel polymer matrix at least in part polymerizes and/or gels prior to application at a target site. In certain instances, the optional additional component, e.g. cell, is added after the formulation is combined. In certain instances, the biocompatible pre-formulations are biodegradable. In certain instances, the biocompatible hydrogel polymer matrix comprises a biocompatible hydrogel scaffold. In certain instances, the biocompatible hydrogel scaffold comprises the at least one first compound, the at least one second compound and a buffer. In certain instances, the biocompatible hydrogel scaffold is fully synthetic.

In some instances, the biocompatible pre-formulation compounds comprise monomers which polymerize into polymers. In some instances, the biocompatible pre-formulation monomers polymerize to form a biocompatible hydrogel polymer matrix. In some instances, a polymer is a biocompatible hydrogel polymer matrix. In some instances, a polymer is a biocompatible hydrogel scaffold. In some instances, the biocompatible pre-formulation compounds gel to form a biocompatible hydrogel polymer matrix. In some instances, the biocompatible pre-formulation compounds gel to form a biocompatible hydrogel scaffold. In some instances, the biocompatible pre-formulation compounds polymerize and gel to form a biocompatible hydrogel polymer matrix. In some instances, the biocompatible pre-formulation compounds polymerize and gel to form a biocompatible hydrogel polymer scaffold. In some instances, the biocompatible hydrogel polymer matrix further polymerizes after hydrogel polymer matrix formation. In some instances, the biocompatible hydrogel polymer matrix gels after hydrogel polymer matrix formation. In some instances, the biocompatible hydrogel polymer matrix further polymerizes and gels after hydrogel polymer matrix formation.

In some instances, the first or second compound comprising more than one nucleophilic or electrophilic group are glycol-based. In some instances, glycol-based compounds include ethylene glycol, propylene glycol, butylene glycol, alkyl glycols of various chain lengths, and any combination or copolymers thereof. In some instances, the glycol-based compounds are polyglycol-based compounds. In some instances, the polyglycol-based compounds include, but are not limited to, polyethylene glycols (PEGs), polypropylene glycols (PPGs), polybutylene glycols (PBGs), and polyglycol copolymers. In some instances, glycol-based compounds include polyethylene glycol, polypropylene glycol, polybutylene glycol, polyalkyl glycols of various chain lengths, and any combination or copolymers thereof. In some instances, the glycol-based compounds are fully synthetic. In some instances, the polyglycol-based compounds are fully synthetic.

In some instances, the first or second compound comprising more than one nucleophilic or electrophilic group are polyol derivatives. In certain instances, the first or second compound is a dendritic polyol derivative. In some instances, the first or second compound is a glycol, trimethylolpropane, glycerol, diglycerol, pentaerythritiol, sorbitol, hexaglycerol, tripentaerythritol, or polyglycerol derivative. In certain instances, the first or second compound is a glycol, trimethylolpropane, pentaerythritol, hexaglycerol, or tripentaerythritol derivative. In some instances, the first or second compound is a trimethylolpropane, glycerol, diglycerol, pentaerythritiol, sorbitol, hexaglycerol, tripentaerythritol, or polyglycerol derivative. In some instances, the first or second compound is a pentaerythritol, di-pentaerythritol, or tripentaerythritol derivative. In certain instances, the first or second compound is a hexaglycerol (2-ethyl-2-(hydroxymethyl)-1,3-propanediol, trimethylolpropane) derivative. In some instances, the first or second compound is a sorbitol derivative. In certain instances, the first or second compound is a glycol, propyleneglycol, glycerin, diglycerin, or polyglycerin derivative.

In some instances, the first and/or second compound comprise polyethylene glycol (PEG) chains comprising one to 200 ethylene glycol subunits. In certain instances, the first and/or second compound may further comprise polypropylene glycol (PPG) chains comprising one to 200 propylene glycol subunits. The PEG or PPG chains extending from the polyols are the "arms" linking the polyol core to the nucleophilic or electrophilic groups.

***Exemplary Nucleophilic Monomers***

The biocompatible pre-formulation comprises at least one first compound comprising more than one nucleophilic group. In some instances, the first compound is a monomer configured to form a polymer matrix through the reaction of a nucleophilic group in the first compound with an electrophilic group of a second compound. In some instances, the first compound monomer is fully synthetic. In some instances, the nucleophilic group is a hydroxyl, thiol, or amino group. In preferred instances, the nucleophilic group is a thiol or amino group. In some instances, the at least one first compound is glycol-based. In some instances, glycol-based compounds include ethylene glycol, propylene glycol, butylene glycol, alkyl glycols of various chain lengths, and any combination or copolymers thereof. In some instances, glycol-based compounds are polyglycol-based compounds. In some instances, the polyglycol-based compounds include, but are not limited to, polyethylene glycols (PEGs), polypropylene glycols (PPGs), polybutylene glycols (PBGs), and polyglycol copolymers. In some instances, glycol-based compounds include polyethylene glycol, polypropylene glycol, polybutylene glycol, polyalkyl glycols of various chain lengths, and any combination or copolymers thereof. In some instances, the glycol-based compounds are fully synthetic. In some instances, the polyglycol-based compounds are fully synthetic.

In certain instances, the nucleophilic group is connected to the polyol derivative through a suitable linker. Suitable linkers include, but are not limited to, esters (e.g., acetates) or ethers. In some instances, monomers comprising ester linkers are more susceptible to biodegradation. Examples of linkers comprising a nucleophilic group include, but are not limited to, mercaptoacetate, aminoacetate (glycin) and other amino acid esters (e.g., alanine, β-alanine, lysine, ornithine), 3-mercaptopropionate, ethylamine ether, or propylamine ether. In some instances, the polyol core derivative is bound to a polyethylene glycol or polypropylene glycol subunit, which is connected to the linker comprising the nucleophilic group. The molecular weight of the first compound (the nucleophilic monomer) is about 500 to 40000. In certain instances, the molecular weight of a first compound (a nucleophilic monomer) is about 100, about 500, about 1000, about 2000, about 3000, about 4000, about 5000, about 6000, about 7000, about 8000, about 9000, about 10000, about 12000, about 15000, about 20000, about 25000, about 30000, about 35000, about 40000, about 50000, about 60000, about 70000, about 80000, about 90000, or about 100000. In some instances, the molecular weight of a first compound is about 500 to 2000. In certain instances, the molecular weight of a first compound is about 15000 to about 40000. In some instances, the first compound is water soluble.

In some instances, the first compound is a MULTIARM-(5k-50k)-polyol derivative comprising polyglycol subunits and more than two nucleophilic groups. MULTIARM refers to number of polyglycol subunits that are attached to the polyol core and these polyglycol subunits link the nucleophilic groups to the polyol core. In some instances, MULTIARM is 3ARM, 4ARM, 6ARM, 8ARM, 10ARM, 12ARM. In some instances, MULTIARM is 4ARM or 8ARM. In some instances, the first compound is MULTIARM-(5k-50k)-NH2, MULTIARM-(5k-50k)-AA, or a combination thereof. In certain instances, the first compound is 4ARM-(5k-50k)-NH2, 4ARM-(5k-50k)-AA, 8ARM-(5k-50k)-NH2, and 8ARM-(5k-50k)-AA, or a combination thereof. In some instances, the polyol derivative is a glycol, trimethylolpropane, glycerol, diglycerol, pentaerythritol, sorbitol, hexaglycerol, tripentaerythritol, or polyglycerol derivative.

Examples of the construction of monomers comprising more than one nucleophilic group are shown below with a trimethylolpropane or pentaerythritol core polyol. The compounds shown have thiol or amine electrophilic groups that are connected to variable lengths PEG subunit through acetate, propionate or ethyl ether linkers (e.g., structures below of ETTMP (A; n = 1), 4ARM-PEG-NH2 (B; n = 1), and 4ARM-PEG-AA (C; n = 1)). Monomers using other polyol cores are constructed in a similar way.

A:

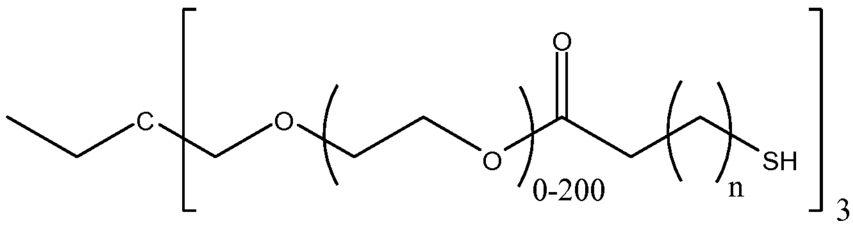

(n = 0 to 6)

B:

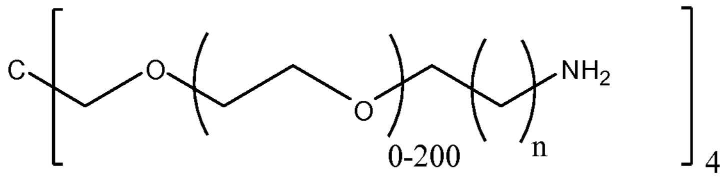

(n = 0 to 6)

C:

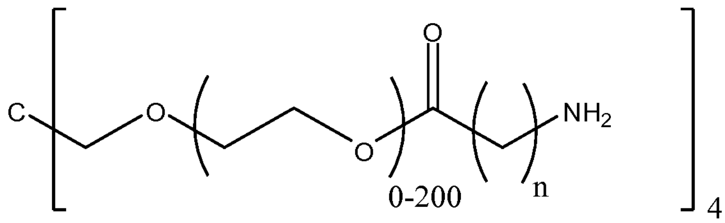

(n = 1-6)

Suitable first compounds comprising a nucleophilic group (used in the amine-ester chemistry) include, but are not limited to, pentaerythritol polyethylene glycol amine (4ARM-PEG-NH2) (molecular weight selected from about 5000 to about 40000, e.g., 5000, 10000, or 20000), pentaerythritol polyethylene glycol amino acetate (4ARM-PEG-AA) (molecular weight selected from about 5000 to about 40000, e.g., 5000, 10000, or 20000), hexaglycerin polyethylene glycol amine (8ARM-PEG-NH2) (molecular weight selected from about 5000 to about 40000, e.g., 10000, 20000, or 40000), or tripentaerythritol glycol amine (8ARM(TP)-PEG-NH2) (molecular weight selected from about 5000 to about 40000, e.g., 10000, 20000, or 40000). Within this class of compounds, 4(or 8)ARM-PEG-AA comprises ester (or acetate) groups while the 4(or 8)ARM-PEG-NH2 monomers do not comprise ester (or acetate) groups.

Other suitable first compounds comprising a nucleophilic group (used in the thiol-ester chemistry) include, but not limited to, glycol dimercaptoacetate (THIOCURE® GDMA), trimethylolpropane trimercaptoacetate (THIOCURE® TMPMA), pentaerythritol tetramercaptoacetate (THIOCURE® PETMA), glycol di-3-mercaptopropionate (THIOCURE® GDMP), trimethylolpropane tri-3-mercaptopropionate (THIOCURE® TMPMP), pentaerythritol tetra-3-mercaptopropionate (THIOCURE® PETMP), polyol-3-mercaptopropionates, polyester-3-mercaptopropionates, propyleneglycol 3-mercaptopropionate (THIOCURE® PPGMP 800), propyleneglycol 3-mercaptopropionate (THIOCURE® PPGMP 2200), ethoxylated trimethylolpropane tri-3-mercaptopropionate (THIOCURE® ETTMP-700), and ethoxylated trimethylolpropane tri-3-mercaptopropionate (THIOCURE® ETTMP-1300).

***Exemplary Electrophilic Monomers***

The biocompatible pre-formulation comprises at least one second compound comprising more than one electrophilic group. In some instances, the second compound is a monomer configured to form a polymer matrix through the reaction of an electrophilic group in the second compound with a nucleophilic group of a first compound. In some instances, the second compound monomer is fully synthetic. In some instances, the electrophilic group is an epoxide,

maleimide, succinimidyl, or an alpha-beta unsaturated ester. In preferred instances, the electrophilic group is an epoxide or succinimidyl. In some instances, the at least one second compound is glycol-based. In some instances, glycol-based compounds include ethylene glycol, propylene glycol, butylene glycol, alkyl glycols of various chain lengths, and any combination or copolymers thereof. The glycol-based compound is a polyglycol-based compound. In some instances, the polyglycol-based compounds include, but are not limited to, polyethylene glycols (PEGs), polypropylene glycols (PPGs), polybutylene glycols (PBGs), and polyglycol copolymers. In some instances, glycol-based compounds include polyethylene glycol, polypropylene glycol, polybutylene glycol, polyalkyl glycols of various chain lengths, and any combination or copolymers thereof. In some instances, the glycol-based compounds are fully synthetic. In some instances, the polyglycol-based polymer is fully synthetic.

In certain instances, the electrophilic group is connected to the polyol derivative through a suitable linker. Suitable linkers include, but are not limited to, esters, amides, or ethers. In some instances, monomers comprising ester linkers are more susceptible to biodegradation. Examples of linkers comprising an electrophilic group include, but are not limited to, succinimidyl succinate, succinimidyl glutarate, succinimidyl succinamide, succinimidyl glutaramide, or glycidyl ether. In some instances, the polyol core derivative is bound to a polyethylene glycol or polypropylene glycol subunit, which is connected to the linker comprising the electrophilic group. The molecular weight of the second compound (the electophilic monomer) is about 500 to 40000. In certain instances, the molecular weight of a second compound (an electophilic monomer) is about 100, about 500, about 1000, about 2000, about 3000, about 4000, about 5000, about 6000, about 7000, about 8000, about 9000, about 10000, about 12000, about 15000, about 20000, about 25000, about 30000, about 35000, about 40000, about 50000, about 60000, about 70000, about 80000, about 90000, or about 100000. In some instances, the molecular weight of a second compound is about 500 to 2000. In certain instances, the molecular weight of a second compound is about 15000 to about 40000. In some instances, the second compound is water soluble.

In some instances, the second compound is a MULTIARM-(5k-50k)-polyol derivative comprising polyglycol subunits and more than two electrophilic groups. MULTIARM refers to number of polyglycol subunits that are attached to the polyol core and these polyglycol subunits link the nucleophilic groups to the polyol core. In some instances, MULTIARM is 3ARM, 4ARM, 6ARM, 8ARM, 10ARM, 12ARM or any combination thereof. In some instances, MULTIARM is 4ARM or 8ARM. In certain instances, the second compound is selected from MULTIARM-(5-50k)-SG, MULTIARM-(5-50k)-SGA, MULTIARM-(5-50k)-SS, MULTIARM-(5-50k)-SSA, and a combination thereof. In certain instances, the second compound is selected from 4ARM-(5-50k)-SG, 4ARM-(5-50k)-SGA, 4ARM-(5-50k)-SS, 8ARM-(5-50k)-SG, 8ARM-(5-50k)-SGA and 8ARM-(5-50k)-SS, and a combination thereof. In some instances, the polyol derivative is a glycol, trimethylolpropane, glycerol, diglycerol, pentaerythritol, sorbitol, hexaglycerol, tripentaerythritol, or polyglycerol derivative.

Examples of the construction of monomers comprising more than one electrophilic group are shown below with a pentaerythritol core polyol. The compounds shown have a succinimidyl electrophilic group, a glutarate or glutaramide linker, and a variable lengths PEG subunit (e.g., structures below of 4ARM-PEG-SG (D; n = 3) and 4ARM-PEG-SGA (E; n = 3)). Monomers using other polyol cores or different linkers (e.g., succinate (SS) or succinamide (SSA) are constructed in a similar way.

D:

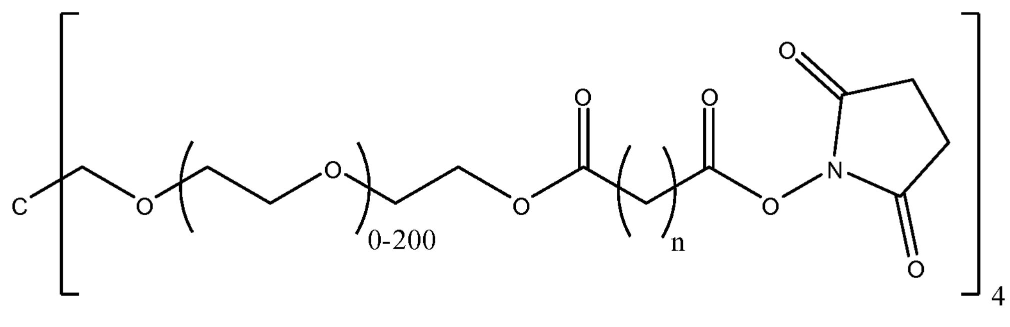

(n = 1 to 6)

E:

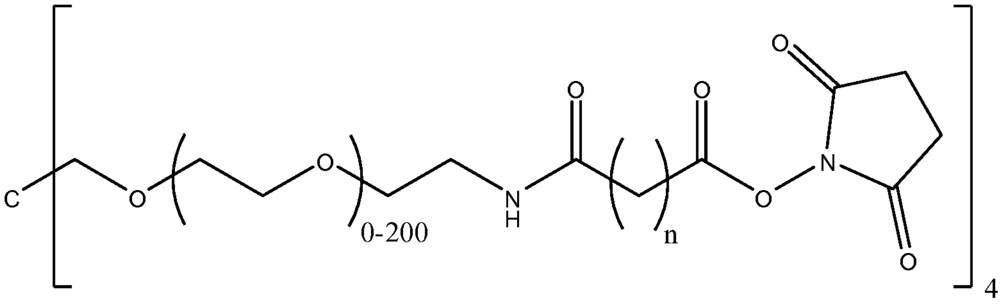

(n = 1 to 6)

Suitable second compounds comprising an electrophilic group include, but are not limited to, pentaerythritol polyethylene glycol maleimide (4ARM-PEG-MAL) (molecular weight selected from about 5000 to about 40000, e.g., 10000 or 20000), pentaerythritol polyethylene glycol succinimidyl succinate (4ARM-PEG-SS) (molecular weight selected from about 5000 to about 40000, e.g., 10000 or 20000), pentaerythritol polyethylene glycol succinimidyl glutarate (4ARM-PEG-SG) (molecular weight selected from about 5000 to about 40000, e.g., 10000 or 20000), pentaerythritol polyethylene glycol succinimidyl glutaramide (4ARM-PEG-SGA) (molecular weight selected from about 5000 to about 40000, e.g., 10000 or 20000), hexaglycerin polyethylene glycol succinimidyl succinate (8ARM-PEG-SS) (molecular weight selected from about 5000 to about 40000, e.g., 10000 or 20000), hexaglycerin polyethylene glycol succinimidyl glutarate (8ARM-PEG-SG) (molecular weight selected from about 5000 to about 40000, e.g., 10000, 15000, 20000, or 40000), hexaglycerin polyethylene glycol succinimidyl glutaramide (8ARM-PEG-SGA) (molecular weight selected from about 5000 to about 40000, e.g., 10000, 15000, 20000, or 40000), tripentaerythritol polyethylene glycol succinimidyl succinate (8ARM(TP)-PEG-SS) (molecular weight selected from about 5000 to about 40000, e.g., 10000 or 20000), tripentaerythritol polyethylene glycol succinimidyl glutarate (8ARM(TP)-PEG-SG) (molecular weight selected from about 5000 to about 40000, e.g., 10000, 15000, 20000, or 40000), or tripentaerythritol polyethylene glycol succinimidyl glutaramide (8ARM(TP)-PEG-SGA) (molecular weight selected from about 5000 to about 40000, e.g., 10000, 15000, 20000, or 40000). The 4(or 8)ARM-PEG-SG monomers comprise ester groups, while the 4(or 8)ARM-PEG-SGA monomers do not comprise ester groups.

Other suitable second compounds comprising an electrophilic group are sorbitol polyglycidyl ethers, including, but not limited to, sorbitol polyglycidyl ether (DENACOL® EX-611), sorbitol polyglycidyl ether (DENACOL® EX-612), sorbitol polyglycidyl ether (DENACOL® EX-614), sorbitol polyglycidyl ether (DENACOL® EX-614 B), polyglycerol polyglycidyl ether (DENACOL® EX-512), polyglycerol polyglycidyl ether (DENACOL® EX-521), diglycerol polyglycidyl ether (DENACOL® EX-421), glycerol polyglycidyl ether (DENACOL® EX-313), glycerol polyglycidyl ether (DENACOL® EX-313), trimethylolpropane polyglycidyl ether (DENACOL® EX-321), sorbitol polyglycidyl ether (DENACOL® EJ-190).

***Formation of Biocompatible Hydrogel Polymer Matrices***

Provided herein are biocompatible pre-formulations, comprising at least one first compound comprising more than one nucleophilic group, at least one second compound comprising more than one electrophilic group, at least one cell, and optionally additional components. An exemplary additional component is a culture medium. In certain instances, the culture medium is a buffer. In certain instances, the culture medium is a nutrient rich medium. In certain embodiments the cell is a stem cell. The biocompatible pre-formulation undergoes polymerization and/or gelling to form a biocompatible hydrogel polymer matrix. In certain instances, the biocompatible hydrogel polymer matrix is biodegradable. In certain instances, the biocompatible hydrogel polymer matrix comprises a biocompatible hydrogel scaffold.

Provided herein are biocompatible pre-formulations, comprising at least one first compound comprising more than one nucleophilic group, at least one second compound comprising more than one electrophilic group, a culture medium, and optionally additional components. An exemplary additional component is at least one cell. In certain embodiments the cell is a stem cell. In certain instances, the culture medium is a buffer. In certain instances, the culture medium is a nutrient rich medium. The biocompatible pre-formulation undergoes polymerization and/or gelling to form a biocompatible hydrogel polymer matrix. In certain instances, the biocompatible hydrogel polymer matrix is biodegradable. In certain instances, the biocompatible hydrogel polymer matrix comprises a biocompatible hydrogel scaffold.

In certain instances, the pre-formulation safely undergoes polymerization at a target site inside or on a mammalian body, for instance at the site of a wound, surgical site, or in a joint. In certain instances, the biocompatible hydrogel polymer matrix forms a wound patch, suture, or joint spacer. In some instances, the first compound and the second compound are monomers forming a polymer matrix through the reaction of a nucleophilic group in the first compound with the electrophilic group in the second compound. In certain instances, the monomers are polymerized at a predetermined time. In some instances, the monomers are polymerized under mild and nearly neutral pH conditions. In certain instances, the biocompatible hydrogel polymer matrix does not change volume after gelling.

In some instances, the first and second compounds react to form amide, thioester, or thioether bonds. When a thiol nucleophile reacts with a succinimidyl electrophile, a thioester is formed. When an amino nucleophile reacts with a succinimidyl electrophile, an amide is formed.

In some instances, one or more first compounds comprising an amino group react with one or more second compounds comprising a succinimidyl ester group to form amide linked first and second monomer units. In certain instances, one or more first compounds comprising a thiol group react with one or more second compounds comprising a succinimidyl ester group to form thioester linked first and second monomer units. In some instances, one or more first compounds comprising an amino group react with one or more second compounds comprising an epoxide group to from amine linked first and second monomer units. In certain instances, one or more first compounds comprising a thiol group react with one or more second compounds comprising an epoxide group to form thioether linked first and second monomer units.

In some instances, a first compound is mixed with a different first compound before addition to one or more second compounds. In other instances, a second compound is mixed with a different second compound before addition to one or more first compounds. In certain instances, the properties of the biocompatible pre-formulation and the biocompatible hydrogel polymer matrix are controlled by the properties of the at least one first and at least one second monomer mixture.

In some instances, one first compound is used in the biocompatible hydrogel polymer matrix. In certain instances, two different first compounds are mixed and used in the biocompatible hydrogel polymer matrix. In some instances, three different first compounds are mixed and used in the biocompatible hydrogel polymer matrix. In certain instances, four or more different first compounds are mixed and used in the biocompatible hydrogel polymer matrix.

In some instances, one second compound is used in the biocompatible hydrogel polymer matrix. In certain instances, two different second compounds are mixed and used in the biocompatible hydrogel polymer matrix. In some instances, three different second compounds are mixed and used in the biocompatible hydrogel polymer matrix. In certain instances, four or more different second compounds are mixed and used in the biocompatible hydrogel polymer matrix.

In some instances, a first compound comprising ether linkages to the nucleophilic group are mixed with a different first compound comprising ester linkages to the nucleophilic group. This allows the control of the concentration of ester groups in the resulting biocompatible hydrogel polymer matrix. In certain instances, a second compound comprising ester linkages to the electrophilic group are mixed with a different second compound comprising ether linkages to the electrophilic group. In some instances, a second compound comprising ester linkages to the electrophilic group are mixed with a different second compound comprising amide linkages to the electrophilic group. In certain instances, a second compound comprising amide linkages to the electrophilic group are mixed with a different second compound comprising ether linkages to the electrophilic group.

In some instances, a first compound comprising an aminoacetate (e.g., glycine derived) nucleophile is mixed with a different first compound comprising an amine nucleophile (e.g., an ethylamine ether) at a specified molar ratio (x/y). In certain instances, the molar ratio (x/y) is 5/95, 10/90, 15/85, 20/80, 25/75, 30/70, 35/65, 40/60, 45/55, 50/50, 55/45, 60/40, 65/35, 70/30, 75/25, 80/20, 85/15, 90/10, or 95/5. In certain instances, a first compound comprising an aminoacetate (e.g., glycine derived) nucleophile is mixed with a different first compound comprising an amine nucleophile (e.g., an ethylamine ether) at a specified weight ratio (x/y). In certain instances, the weight ratio (x/y) is 5/95, 10/90, 15/85, 20/80, 25/75, 30/70, 35/65, 40/60, 45/55, 50/50, 55/45, 60/40, 65/35, 70/30, 75/25, 80/20, 85/15, 90/10, or 95/5. In certain instances, the mixture of two first compounds is mixed with one or more second compounds at a molar amount equivalent to the sum of x and y.

In some instances, the first compound comprising more than one nucleophilic group and the at least one cell are pre-mixed in the presence of water. In some instances, the first compound comprising more than one nucleophilic group and the cell are pre-mixed without the presence of water. Once pre-mixing is complete, the second compound comprising more than one electrophilic group is added to the pre-mixture in the presence of water to form a biocompatible hydrogel polymer matrix. Shortly after final mixing, the biocompatible hydrogel polymer matrix mixture is delivered to the target site. In certain instances, an optional additional component is added to the pre-mix, the second compound, or to the mixture just before delivery of the biocompatible hydrogel polymer matrix mixture to the target site. In certain instances, an optional additional component is added to the pre-mix, the second compound, or to the mixture after delivery of the biocompatible hydrogel polymer matrix mixture to the target site. In some instances, the additional component is a buffer. In some instances, the biocompatible hydrogel polymer matrix polymerizes and/or gels prior to delivery to the target site. In some instances, the biocompatible hydrogel polymer matrix polymerizes and/or gels at the target site.

In some instances, the first compound comprising more than one nucleophilic group and the buffer are pre-mixed in the presence of water. In some instances, the first compound comprising more than one nucleophilic group and the buffer are pre-mixed without the presence of water. Once pre-mixing is complete, the second compound comprising more than one electrophilic group is added to the pre-mixture in the presence of water, forming a biocompatible hydrogel polymer matrix. Shortly after final mixing, the biocompatible hydrogel polymer matrix mixture is delivered to the target site. In certain instances, an optional additional component is added to the pre-mix, the second compound, or to the mixture just before delivery of the biocompatible hydrogel polymer matrix mixture to the target site. In certain instances, an optional additional component is added to the pre-mix, the second compound, or to the mixture after delivery of the biocompatible hydrogel polymer matrix mixture to the target site. In some instances, the additional component is at least one cell. In some instances, the biocompatible hydrogel polymer matrix polymerizes and/or gels prior to delivery to the target site. In some instances, the biocompatible hydrogel polymer matrix polymerizes and/or gels at the target site.

In other instances, the second compound comprising more than one electrophilic group and the at least one cell are pre-mixed in the presence of water. In other instances, the second compound comprising more than one electrophilic group and the cell are pre-mixed without the presence of water. Once pre-mixing is complete, the first compound comprising more than one nucleophilic group is added to the pre-mixture, forming a biocompatible hydrogel polymer matrix. Shortly after final mixing, the biocompatible hydrogel polymer matrix mixture is delivered to the target site. In certain instances, an optional component is added to the pre-mix, the first compound, or to the mixture just before delivery of the biocompatible hydrogel polymer matrix mixture to the target site. In certain instances, an optional additional component is added to the pre-mix, the first compound, or to the mixture after delivery of the biocompatible hydrogel polymer matrix mixture to the target site. In some instances, the additional component is a buffer. In some instances, the biocompatible hydrogel polymer matrix polymerizes and/or gels prior to delivery to the target site. In some instances, the biocompatible hydrogel polymer matrix polymerizes and/or gels at the target site.

In other instances, the second compound comprising more than one electrophilic group and the buffer are pre-mixed in the presence of water. In other instances, the second compound comprising more than one electrophilic group and the buffer are pre-mixed without the presence of water. Once pre-mixing is complete, the first compound comprising more than one nucleophilic group is added to the pre-mixture, forming a biocompatible hydrogel polymer matrix. Shortly after final mixing, the biocompatible hydrogel polymer matrix mixture is delivered to the target site. In certain instances, an optional component is added to the pre-mix, the first compound, or to the mixture just before delivery of the biocompatible hydrogel polymer matrix mixture to the target site. In certain instances, an optional additional component is added to the pre-mix, the first compound, or to the mixture after delivery of the biocompatible hydrogel polymer matrix mixture to the target site. In some instances, the additional component is at least one cell. In some instances, the biocompatible hydrogel polymer matrix polymerizes and/or gels prior to delivery to the target site. In some instances, the biocompatible hydrogel polymer matrix polymerizes and/or gels at the target site.

In some instances, a first compound comprising more than one nucleophilic group, a second compound comprising more than one electrophilic group, and at least one cell are mixed together in the presence of water, whereby a biocompatible hydrogel polymer matrix is formed. In some instances, a first compound comprising more than one nucleophilic group, a second compound comprising more than one electrophilic group, and a buffer are mixed together in the presence of water, whereby a biocompatible hydrogel polymer matrix is formed. In some instances, a first compound comprising more than one nucleophilic group, a second compound comprising more than one electrophilic group, at least one cell, and a buffer are mixed together in the presence of water, whereby a biocompatible hydrogel polymer matrix is formed. In certain instances, the first compound comprising more than one nucleophilic group, the second compound comprising more than one electrophilic group, and/or the cell are individually diluted in an aqueous buffer in the pH range of about 5.0 to about 9.5, wherein the individual dilutions or neat monomers are mixed and a biocompatible hydrogel polymer matrix is formed. In some instances, the aqueous buffer is in the pH range of about 6.0 to about 8.5. In certain instances, the aqueous buffer is in the pH range of about 8. In certain instances, the aqueous buffer is a culture medium. In certain instances, the culture medium is a nutrient rich medium.

In certain instances, the concentration of the monomers in the aqueous is from about 1% to about 100%. In some instances, the dilution is used to adjust the viscosity of the monomer dilution. In certain instances, the concentration of a monomer in the aqueous buffer is about 1%, about 2%, about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100%.

In some instances, the electrophilic and nucleophilic monomers are mixed in such ratio that there is a slight excess of electrophilic groups present in the mixture. In certain instances, this excess is about 10%, about 5%, about 2%, about 1%, about 0.9%, about 0.8%, about 0.7%, about 0.6%, about 0.5%, about 0.4%, about 0.3%, about 0.2%, about 0.1%, or less than 0.1%.

In certain instances, the gelling time or curing time of the biocompatible hydrogel polymer matrix is controlled by the selection of the first and second compounds. In some instances, the concentration of nucleophilic or electrophilic groups in the first or second compound influences the gelling time of the biocompatible pre-formulation. In certain instances, temperature influences the gelling time of the biocompatible pre-formulation. In some instances, the type of aqueous buffer influences the gelling time of the biocompatible pre-formulation. In some instances, the aqueous buffer

is a culture medium. In certain instances, the concentration of the aqueous buffer influences the gelling time of the biocompatible pre-formulation. In some instances, the nucleophilicity and/or electrophilicity of the nucleophilic and electrophilic groups of the monomers influences the gelling time of the biocompatible pre-formulation. In some instances, the cell type influences the gelling time of the biocompatible pre-formulation. In some instances, the cell concentration influences the gelling time of the biocompatible pre-formulation.

In some instances, the gelling time or curing time of the biocompatible hydrogel polymer matrix is controlled by the pH of the aqueous buffer. In certain embodiments, the gelling time is between about 20 seconds and 10 minutes. In some instances, the gelling time is less than 30 minutes, less than 20 minutes, less than 10 minutes, less than 5 minutes, less than 4.8 minutes, less than 4.6 minutes, less than 4.4 minutes, less than 4.2 minutes, less than 4.0 minutes, less than 3.8 minutes, less than 3.6 minutes, less than 3.4 minutes, less than 3.2 minutes, less than 3.0 minutes, less than 2.8 minutes, less than 2.6 minutes, less than 2.4 minutes, less than 2.2 minutes, less than 2.0 minutes, less than 1.8 minutes, less than 1.6 minutes, less than 1.4 minutes, less than 1.2 minutes, less than 1.0 minutes, less than 0.8 minutes, less than 0.6 minutes, or less than 0.4 minutes. In certain instances, the pH of the aqueous buffer is from about 5 to about 9.5. In some instances, the pH of the aqueous buffer is from about 7.0 to about 9.5. In specific instances, the pH of the aqueous buffer is about 8. In some instances, the pH of the aqueous buffer is about 5, about 5.5, about 6.0, about 6.5, about 6.6, about 6.7, about 6.8, about 6.9, about 7.0, about 7.1, about 7.2, about 7.3, about 7.4, about 7.5, about 7.6, about 7.8, about 7.9, about 8.0, about 8.1 about 8.2 about 8.3, about 8.4, about 8.5, about 9.0, or about 9.5.

In certain instances, the gelling time or curing time of the biocompatible pre-formulation is controlled by the type of aqueous buffer. In some instances, the aqueous buffer is a physiologically acceptable buffer. In certain instances, aqueous buffers include, but are not limited to, aqueous saline solutions, phosphate buffered saline, borate buffered saline, a combination of borate and phosphate buffers wherein each component is dissolved in separate buffers, N-2-Hydroxyethylpiperazine-N'-2-hydroxypropanesulfonic acid (HEPES), 3-(N-Morpholino) propanesulfonic acid (MOPS), 2-([2-Hydroxy-1,1-bis(hydroxymethyl)ethyl]amino)ethanesulfonic acid (TES), 3-[N-tris(Hydroxy-methyl) ethylamino]-2-hydroxyethyl]-1-piperazinepropanesulfonic acid (EPPS), Tris[hydroxymethyl]-aminomethane (THAM), and Tris[hydroxymethyl]methyl aminomethane (TRIS). In some instances, the thiol-ester chemistry (e.g., ETTMP nucleophile with SGA or SG electrophile) is performed in borate buffer. In certain instances, the amine-ester chemistry (NH2 or AA nucleophile with SGA or SG electrophile) is performed in phosphate buffer. In some instances the aqueous buffer is a culture medium. In certain instances, culture media include, but are not limited to, DMEM, IMDM, OptiMEM®, AlgiMatrix™, Fetal Bovine Serum, GS1-R®, GS2-M®, iSTEM®, NDiff® N2,NDiff® N2-AF, RHB-A®, RHB-Basal®, RPMI, SensiCell™, GlutaMAX™, FluoroBrite™, Gibco® TAP, Gibco® BG-11, LB, M9 Minimal, Terrific Broth, 2YXT, MagicMedia™, ImMedia™, SOC, YPD, CSM, YNB, Grace's Insect Media, 199/109 and HamF10/HamF12. In certain instances, the cell culture medium may be serum free. In certain instances, the culture media may include additives. In some instances, culture media additives include, but are not limited to, antibiotics, vitamins, proteins, inhibitors, small molecules, minerals, inorganic salts, nitrogen, growth factors, amino acids, serum, carbohydrates, lipids, hormones and glucose. In some instances, growth factors include, but are not limited to, EGF, bFGF, FGF, ECGF, IGF-1, PDGF, NGF, TGF-$\alpha$ and TGF-$\beta$. In certain instances, the culture medium may not be aqueous. In certain instances, the non-aqueous culture media include, but are not limited to, frozen cell stocks, lyophilized medium, and agar.

In certain instances, the biocompatible hydrogel polymer matrix comprises a biocompatible hydrogel scaffold. In certain instances, the biocompatible hydrogel scaffold comprises the pre-formulation at least one first compound and the pre-formulation at least one second compound. In certain instances, the biocompatible hydrogel scaffold comprises a buffer. In certain instances, the biocompatible hydrogel scaffold is fully synthetic. In certain instances, the biocompatible hydrogel scaffold provides an environment suitable for sustained cell viability and/or growth.

In certain instances, the first compound and the second compound do not react with the cell during formation of the biocompatible hydrogel polymer matrix. In some instances, the cell remains unchanged after polymerization of the first and second compounds (i.e., monomers). In certain instances, the cell does not change the properties of the biocompatible hydrogel polymer matrix. In some instances, the physiochemical properties of the cell and the biocompatible hydrogel polymer matrix formulation are not affected by the polymerization of the monomers. In certain instances, delivery of the cell using a biocompatible hydrogel polymer matrix minimizes the degradation or denaturing of the cell. In some instances, the physiochemical properties of the cell are not affected by the delivery or release of the cell to the target site.

In some instances, the biocompatible hydrogel polymer matrix formulations further comprise a contrast agent for visualizing the biocompatible hydrogel polymer matrix formulation and locating a tumor using e.g., X-ray, fluoroscopy, or computed tomography (CT) imaging. In certain instances, the contrast agent enables the visualization of the bioabsorption of the biocompatible hydrogel polymer matrix. In some instances, the contrast agent is a radiopaque material. In certain instances, the radiopaque material is selected from, but not limited to, sodium iodide, potassium iodide, and barium sulfate, VISIPAQUE®, OMNIPAQUE®, or HYPAQUE®, tantalum, and similar commercially available compounds, or combinations thereof. In other instances, the biocompatible hydrogel polymer matrix further comprises a pharmaceutically acceptable dye.

The biocompatible hydrogel polymer matrix formulations further comprise a viscosity enhancer. Examples of viscosity enhancer include, but are not limited to, hydroxyethylcellulose, hydroxypropylcellulose, methylcellulose, polyvinylcellulose, polyvinylpyrrolidone.

***Area of for Treatment - Target Sites***

In certain instances, the target site is inside a mammal. In some instances, the target site is inside a human being. In certain instances, the target site is on the human body. In some instances, the target site is accessible through surgery. In certain instances, the target site is accessible through minimally invasive surgery. In some instances, the target site is accessible through an endoscopic device. In certain instances, the target site is a wound on the skin of a mammal. In other instances, the target site is in a joint or on a bone of a mammal. In some instances, the target site is a surgical site in a mammal

In some instances, a biocompatible pre-formulation or a biocompatible hydrogel polymer matrix is used as a sealant or adhesive. In certain instances, the biocompatible pre-formulation or biocompatible hydrogel polymer matrix is used to seal a wound on a mammal. In other instances, the biocompatible pre-formulation or biocompatible hydrogel polymer matrix is used to fill cavities, e.g., in a joint space to form a gel cushion. In other instances, the biocompatible pre-formulation or biocompatible hydrogel polymer matrix is used as a carrier for delivery of cells to target sites.

In some instances, the biocompatible hydrogel polymer matrix formulation is polymerized ex vivo. In certain instances, the ex vivo polymerized biocompatible hydrogel polymer matrix formulation is delivered through traditional routes of administration (e.g., oral, implantation, or rectal). In other instances, the ex vivo polymerized biocompatible hydrogel polymer matrix formulation is delivered during surgery to a target site.

***Delivery of the Biocompatible Hydrogel Formulation to a Target Site***

In some instances, the biocompatible pre-formulation is delivered as a biocompatible pre-formulation to a target site through a catheter or a needle to form a biocompatible hydrogel polymer matrix at the target site. In other instances, the biocompatible pre-formulation is delivered to the target site in or on the mammal using syringe and needle. In some instances, a delivery device is used to deliver the biocompatible pre-formulation to the target site. In some instances, the biocompatible pre-formulation is delivered to the target site so that the biocompatible pre-formulation mostly covers the target site. In certain instances, the biocompatible pre-formulation substantially covers an exposed portion of diseased tissue. In some instances, the biocompatible pre-formulation does not spread to any other location intentionally. In some instances, the biocompatible pre-formulation substantially covers diseased tissue and does not significantly cover healthy tissue. In certain instances, the biocompatible hydrogel polymer matrix does not significantly cover healthy tissue. In some instances, the biocompatible pre-formulation gels over the target site and thoroughly covers diseased tissue. In some instances, the biocompatible hydrogel polymer matrix adheres to tissue. In some instances, the biocompatible hydrogel polymer matrix mixture gels after delivery at the target site, covering the target site. In some instances, the biocompatible hydrogel polymer matrix mixture gels prior to delivery at the target site.

In some instances, the gelling time of the biocompatible pre-formulation is set according to the preference of the doctor delivering the biocompatible pre-formulation mixture to a target site. In most instances, a physician delivers the biocompatible pre-formulation mixture to the target within 15 to 30 seconds. In certain instances, the gelling time is between about 20 seconds and 10 minutes. In some instances, the gelling time or curing time of the biocompatible pre-formulation is controlled by the pH of the aqueous buffer. In certain instances, the gelling time or curing time of the biocompatible pre-formulation is controlled by the selection of the first and second compounds. In some instances, the concentration of nucleophilic or electrophilic groups in the first or second compound influences the gelling time of the biocompatible pre-formulation. In some instances, cell concentration influences the gelling time of the biocompatible pre-formulation. In some instances, cell type influences the gelling time of the biocompatible pre-formulation. In some instances, optional addition components influence the gelling time of the biocompatible pre-formulation.

In some instances, curing of the biocompatible hydrogel polymer matrix is verified post-administration. In certain instances, the verification is performed in vivo at the delivery site. In other instances, the verification is performed ex vivo. In some instances, curing of the biocompatible hydrogel polymer matrix is verified visually through the fiber-optics of an endoscopic device. In certain instances, curing of biocompatible hydrogel polymer matrices comprising radiopaque materials is verified using X-ray, fluoroscopy, or computed tomography (CT) imaging. A lack of flow of the biocompatible hydrogel polymer matrix indicates that the biocompatible hydrogel polymer matrix has gelled and the biocompatible hydrogel is sufficiently cured. In further instances, curing of the biocompatible hydrogel polymer matrix is verified by evaluation of the residue in the delivery device, for instance the residue in the catheter of the bronchoscope or other endoscopic device, or the residue in the syringe used to deliver the biocompatible hydrogel polymer matrix. In other instances, curing of the biocompatible hydrogel polymer matrix is verified by depositing a small sample (e.g., ~1 mL) on a piece of paper or in a small vessel and subsequent evaluation of the flow characteristics after the gelling time has passed.

In some instances, the biocompatible pre-formulation delivers at least one cell to a target site. In some instances, the biocompatible pre-formulation delivers nutrients to at least one cell located at a target site. In some instances, the biocompatible pre-formulation delivers structural support to at least one cell located at a target site. In some instances, the biocompatible pre-formulation delivers at least one cell and at least one buffer to a target site. In some instances, the biocompatible hydrogel polymer matrix delivers at least one cell to a target site. In some instances, the biocompatible hydrogel polymer matrix delivers nutrients to at least one cell located at a target site. In some instances, the biocompatible hydrogel polymer matrix delivers structural support to at least one cell located at a target site. In some instances, the biocompatible hydrogel polymer matrix delivers at least one cell to a target site.

***Bioabsorbance of the Biocompatible Hydrogel Polymer matrix***

In some instances, the biocompatible hydrogel polymer matrix is a bioabsorbable polymer. In certain instances, the biocompatible hydrogel polymer matrix is bioabsorbed within about 5 to 30 days. In some instances, the biocompatible hydrogel polymer matrix is bioabsorbed within about 30 to 180 days. In some instances, the biocompatible hydrogel polymer matrix is bioabsorbed within about 1 to 70 days. In preferred embodiments, the biocompatible hydrogel polymer matrix is bioabsorbed within about 14 to 180 days. In some instances the biocompatible hydrogel polymer matrix is bioabsorbed within about 365 days, 180 days, about 150 days, about 120 days, about 90 days, about 80 days, about 70 days, about 60 days, about 50 days, about 40 days, about 35 days, about 30 days, about 28 days, about 21 days, about 14 days, about 10 days, about 7 days, about 6 days, about 5 days, about 4 days, about 3 days, about 2 days, or about 1 day. In certain instances the biocompatible hydrogel polymer matrix is bioabsorbed within less than 365 days, 180 days, less than 150 days, less than 120 days, less than 90 days, less than 80 days, less than 70 days, less than 60 days, less than 50 days, less than 40 days, less than 35 days, less than 30 days, less than 28 days, less than 21 days, less than 14 days, less than 10 days, less than 7 days, less than 6 days, less than 5 days, less than 4 days, less than 3 days, less than 2 days, or less than 1 day. In some instances the biocompatible hydrogel polymer matrix is bioabsorbed within more than 365 days, 180 days, more than 150 days, more than 120 days, more than 90 days, more than 80 days, more than 70 days, more than 60 days, more than 50 days, more than 40 days, more than 35 days, more than 30 days, more than 28 days, more than 21 days, more than 14 days, more than 10 days, more than 7 days, more than 6 days, more than 5 days, more than 4 days, more than 3 days, more than 2 days, or more than 1 day. In some instances, the biocompatible hydrogel polymer matrix is substantially non-bioabsorbable.

The biocompatible hydrogel polymer matrix is slowly bioabsorbed, dissolved, and or excreted. In some instances, the rate of bioabsorption is controlled by the number of ester groups in the biocompatible and/or biodegradable hydrogel polymer matrix. In other instances, the higher the concentration of ester units is in the biocompatible hydrogel polymer matrix, the longer is its lifetime in the body. In further instances, the electron density at the carbonyl of the ester unit controls the lifetime of the biocompatible hydrogel polymer matrix in the body. In certain instances, biocompatible hydrogel polymer matrices without ester groups are essentially not biodegradable. In additional instances, the molecular weight of the first and second compounds controls the lifetime of the biocompatible hydrogel polymer matrix in the body. In further instances, the number of ester groups per gram of polymer matrix controls the lifetime of the biocompatible hydrogel polymer matrix in the body.

In some instances, the lifetime of the biocompatible hydrogel polymer matrix can be estimated using a model, which controls the temperature and pH at physiological levels while exposing the biocompatible hydrogel polymer matrix to a buffer solution. In certain instances, the biodegradation of the biocompatible hydrogel polymer matrix is substantially non-enzymatic degradation.

In some instances, the selection of reaction conditions determines the degradation time of the biocompatible hydrogel polymer matrix. In certain instances, the concentration of the first compound and second compound monomers determines the degradation time of the resulting biocompatible hydrogel polymer matrix. In some instances, a higher monomer concentration leads to a higher degree of cross-linking in the resulting biocompatible hydrogel polymer matrix. In certain instances, more cross-linking leads to a later degradation of the biocompatible hydrogel polymer matrix. In certain instances, temperature determines the degradation time of the resulting biocompatible hydrogel polymer matrix. In some instances, a higher monomer concentration leads to a higher degree of cross-linking in the resulting biocompatible hydrogel polymer matrix.

In certain instances, the composition of the linker in the first and/or second compound influences the speed of degradation of the resulting biocompatible hydrogel polymer matrix. In some instances, the more ester groups are present in the biocompatible hydrogel polymer matrix, the faster the degradation of the biocompatible hydrogel polymer matrix. In certain instances, the higher the concentration of mercaptopropionate (ETTMP), acetate amine (AA), glutarate or succinate (SG or SS) monomers, the faster the rate of degradation.

In certain instances, the composition of the cell influences the speed of degradation of the resulting biocompatible hydrogel polymer matrix. In certain instances, the concentration of the cell influences the speed of degradation of the resulting biocompatible hydrogel polymer matrix. In certain instances, the composition of a buffer influences the speed

of degradation of the resulting biocompatible hydrogel polymer matrix. In certain instances, the concentration of a buffer influences the speed of degradation of the resulting biocompatible hydrogel polymer matrix. In certain instances, the pH of a buffer influences the speed of degradation of the resulting biocompatible hydrogel polymer matrix. In certain instances, the composition of the optional additional components influences the speed of degradation of the resulting biocompatible hydrogel polymer matrix.

***Pre-formulations and Hydrogel Matrices for Cell Delivery in the Treatment of Disease***

The treatment of tendon injuries by stem cells necessitates controlled delivery and release of cells at the target area. For example, bone marrow mesenchymal stem cells (MSCs) have a beneficial effect on the healing of tendon injuries in a horse. Current methodologies inject MSCs in autologous bone marrow aspirate in large numbers (10 - 20 million cells), however less than 25% of the MSCs remain in the injury area after 24 hours due to systemic clearance. Retention of cells at the delivery site may encourage the cells to engraft into the tissue resulting in increased amounts of MSCs available to contribute to tissue healing. The biocompatible pre-formulation and hydrogel polymer matrix described herein are configured to deliver cells such as MSCs within a pliable, injectable and absorbable gel that is tolerated clinically and is compatible with cell survival and growth. In some instances, the biocompatible pre-formulation and hydrogel polymer matrix described herein provide improved cell viability over cells injected without the use of a biocompatible pre-formulation. In certain instances, the biocompatible hydrogel polymer matrix functions as a scaffold supporting the growth of cells loaded on or within the hydrogel polymer matrix.

In some instances, the biocompatible pre-formulation or hydrogel polymer matrix described herein is delivered to a target site on or in a mammal. In certain instances, the biocompatible pre-formulation or hydrogel polymer matrix is delivered to a target site in a joint. In some instances, the biocompatible pre-formulation forms a biocompatible hydrogel polymer matrix inside a joint. In certain instances, the biocompatible pre-formulation forms a sticky biocompatible polymer matrix to seal a wound on or in an animal. In some instances, the biocompatible pre-formulation forms a suture. In certain instances, the wound patch, joint spacer, or suture gels at least in part at the target site in or on the mammal. In some instances, the wound patch, joint spacer, or suture polymerizes at least in part at a target site. In some instances, the wound patch, joint spacer, or suture adheres at least partially to the target site.

In certain instances, the biocompatible pre-formulation is used as a "liquid suture" or as a drug delivery platform to transport medications directly to the targeted site in or on the mammal. In some instances the target site is a joint, a wound or a surgical site. In some instances, the spreadability, viscosity, optical clarity, and adhesive properties of the biocompatible pre-formulation or hydrogel polymer matrix are optimized to create materials ideal as liquid sutures for the treatment of diseases. In certain instances, the gel time is controlled from 50 seconds to 15 minutes.

In some instances, a biocompatible pre-formulation or hydrogel polymer matrix comprising at least one cell is delivered to a target site in a mammal. In some instances, the biocompatible pre-formulation or hydrogel polymer matrix is configured to deliver cells into damaged tissue in order to treat disease or injury. In some instances, the diseases include, but are not limited to, cancer, diabetes, Alzheimer’s disease, Parkinson’s disease, Huntington’s disease, and Celiac disease. In some instances, the injury is caused by cardiac failure, muscle damage, brain damage, or neurological disorders. In some instances, the injury is a spinal cord injury. In some instances, the delivered cells are configured to treat orthopedic diseases or injuries. In some instances, the delivered cells are configured to repair tendons, joints, bone defects, muscle, or nerves.

***Control of Release Rate of a Cell***

In some instances, the biocompatible hydrogel polymer matrix slowly delivers at least one cell to a target site by diffusion and/or osmosis over time ranging from hours to days. In certain instances, the cell is delivered directly to the target site. In some instances, the procedure of delivering a biocompatible hydrogel polymer matrix comprising a cell to a target site is repeated several times, if needed. In other instances, the cell is released from the biocompatible hydrogel polymer matrix through biodegradation of the biocompatible hydrogel polymer matrix. In some instances, the cell is released through a combination of diffusion, osmosis, and/or biocompatible hydrogel degradation mechanisms. In certain instances, the release profile of the cell from the biocompatible hydrogel polymer matrix is unimodal. In some instances, the release profile of the cell from the biocompatible hydrogel polymer matrix is bimodal. In certain instances, the release profile of the cell from the biocompatible hydrogel polymer matrix is multimodal.

In some instances, the cell is released from the biocompatible hydrogel polymer matrix though diffusion or osmosis. In certain instances, the cell is substantially released from the biocompatible hydrogel polymer matrix within 180 days. In some instances, the cell is substantially released from the biocompatible hydrogel polymer matrix within 14 days. In certain instances, the cell is substantially released from the biocompatible hydrogel polymer matrix within 24 hours. In some instances, the cell is substantially released from the biocompatible hydrogel polymer matrix within one hour. In certain instances, the cell is substantially released from the biocompatible hydrogel polymer matrix within

about 180 days, about 150 days, about 120 days, about 90 days, about 80 days, about 70 days, about 60 days, about 50 days, about 40 days, about 35 days, about 30 days, about 28 days, about 21 days, about 14 days, about 10 days, about 7 days, about 6 days, about 5 days, about 4 days, about 3 days, about 2 days, about 1 day, about 0.5 day, about 6 hours, about 4 hours, about 2 hours, about or 1 hour. In some instances, the cell is substantially released from the biocompatible hydrogel polymer matrix within more than 180 days, more than 150 days, more than 120 days, more than 90 days, more than 80 days, more than 70 days, more than 60 days, more than 50 days, more than 40 days, more than 35 days, more than 30 days, more than 28 days, more than 21 days, more than 14 days, more than 10 days, more than 7 days, more than 6 days, more than 5 days, more than 4 days, more than 3 days, more than 2 days, more than 1 day, more than 0.5 day, more than 6 hours, more than 4 hours, more than 2 hours, more than or 1 hour. In certain instances, the cell is substantially released from the biocompatible hydrogel polymer matrix within less than 180 days, less than 150 days, less than 120 days, less than 90 days, less than 80 days, less than 70 days, less than 60 days, less than 50 days, less than 40 days, less than 35 days, less than 30 days, less than 28 days, less than 21 days, less than 14 days, less than 10 days, less than 7 days, less than 6 days, less than 5 days, less than 4 days, less than 3 days, less than 2 days, less than 1 day, less than 0.5 day, less than 6 hours, less than 4 hours, less than 2 hours, less than or 1 hour. In some instances, the cell is substantially released from the biocompatible hydrogel polymer matrix within about one day to about fourteen days. In certain instances, the cell is substantially released from the biocompatible hydrogel polymer matrix within about one day to about 70 days.

In some instances, release of the cell from the biocompatible hydrogel polymer matrix is controlled by the composition of the biocompatible hydrogel polymer matrix. In certain instances, the cell is released when the biocompatible hydrogel polymer matrix starts to degrade. In some instances, the pore size of the biocompatible hydrogel polymer matrix is small enough to prevent the early phase release of the cell (i.e., release before the degradation of the biocompatible hydrogel polymer matrix). In certain instances, the pore size of the biocompatible hydrogel polymer matrix is large enough to allow the early phase release of the cell.

In some instances, large PEG groups in the monomers leads to large pore sizes in the resulting biocompatible hydrogel polymer matrix allowing the elution of large cells. In certain instances, large molecular weights of the monomers lead to biocompatible hydrogel polymer matrices with large pore sizes. In some instances, large monomer molecular weights of about 10kDa lead to biocompatible hydrogel polymer matrices with large pore sizes. In certain instances, large monomer molecular weights of about 20kDa lead to biocompatible hydrogel polymer matrices with large pore sizes.

In some instances, small PEG groups in the monomers leads to small pore sizes in the resulting biocompatible hydrogel polymer matrix restricting the elution of small (and large) cells. In certain instances, small molecular weights of the monomers lead to biocompatible hydrogel polymer matrices with small pore sizes. In some instances, small monomer molecular weights of about 5kDa lead to biocompatible hydrogel polymer matrices with small pore sizes. In certain instances, small monomer molecular weights of about 10kDa in an 8-ARM monomer lead to biocompatible hydrogel polymer matrices with small pore sizes. In some instances, the small pore sizes restrict the elution of cells.

## ***Exemplary Cells***

The biocompatible hydrogel polymer matrix comprises at least one cell. In some instances, the biocompatible hydrogel polymer matrix is delivered with a cell. Examples of cells include, but are not limited to mammalian, insect, protozoal, bacterial, viral, and fungal. In some instances, the cells may be genetically engineered. In some instances, the cells may be a vaccine.

In certain instances, the cell is a mammalian cell. Examples of mammalian cells include, but are not limited to human, murine, hamster, rat, canine and primate. Examples of human cells include, but are not limited to embryonic, adult, bone marrow stromal, embryonic germline, fetal, oligopotent progenitor, somatic and induced pluripotent. Mammalian cells include, but are not limited to, established or developed cell lines. Examples of cell lines include, but are not limited to, HEK-293, CHO, 293-T, A2780, BHK-21, BCP-1, DU145, H1299, HeLa, High-Five, HUVEC, MCF-7 and RBL. Mammalian cells include, but are not limited to, stem cells. Examples of stem cells include, but are not limited to adult, embryonic, hematopoietic, embryonic, mesenchymal, multipotent, neural, pluripotent, totipotent, umbilical cord and unipotent.

In certain instances, the cell is an insect cell. In certain instances, the insect cell is genetically engineered. In certain instances, the insect cell is non-infectious. Examples of insect cells include, but are not limited to, *Spodoptera frugiperda, Drosophila* and *Trichoplusia ni.*

In certain instances, the cell is a protozoa cell. In certain instances, the protozoa cell is genetically engineered. In certain instances, the protozoa cell is a vaccine. In certain instances, the protozoa cell is non-infectious. Examples of protozoa cells include, but are not limited to, *Giardia lamblia, Entamoeba histolytica, Plasmodium knowlesi* and *Balantidium coli.*

In certain instances, the cell is a bacterial cell. In certain instances, the bacterial cell is genetically engineered. In certain instances, the bacterial cell is a vaccine. In certain instances, the bacterial cell is non-infectious. Examples of

bacterial cells include, but are not limited to, *Acetobacter aurantius, Agrobacterium radiobacter, Anaplasma phagocytophilum, Azorhizobium caulinodans, Bacillus anthracis, Bacillus brevis, Bacillus cereus, Bacillus subtilis, Bacteroides fragilis, Bacteroides gingivalis, Bacteroides melaninogenicus, Bartonella quintana, Bordetella bronchiseptica, Bordetella pertussis, Borrelia burgdorferi, Brucella abortus, Brucella melitensis, Brucella suis, Burkholderia mallei, Burkholderia pseudomallei, Burkholderia cepacia, Calymmatobacterium granulomatis, Campylobacter coli, Campylobacter fetus, Campylobacter jejuni, Campylobacter pylori, Chlamydia trachomatis, Chlamydophila pneumoniae, Chlamydophila psittaci, Clostridium botulinum, Clostridium difficile, Corynebacterium diphtheriae, Corynebacterium fusiforme, Coxiella burnetii, Enterobacter cloacae, Enterococcus faecalis, Enterococcus faecium, Enterococcus galllinarum, Enterococcus maloratus, Escherichia coli, Francisella tularensis, Fusobacterium nucleatum, Gardnerella vaginalis, Haemophilus influenzae, Haemophilus parainfluenzae, Haemophilus pertussis, Haemophilus vaginalis, Helicobacter pylori, Klebsiella pneumoniae, Lactobacillus acidophilus, Lactococcus lactis, Legionella pneumophila, Listeria monocytogenes, Methanobacterium extroquens, Microbacterium multiforme, Micrococcus luteus, Moraxella catarrhalis, Mycobacterium phlei, Mycobacterium smegmatis, Mycobacterium tuberculosis, Mycoplasma genitalium, Mycoplasma hominis, Mycoplasma pneumonie, Neisseria gonorrhoeae, Neisseria meningitidis, Pasteurella multocida, Pasteurella tularensis, Peptostreptococcus, Porphyromonas gingivalis, Prevotella melaninogenica, Pseudomonas aeruginosa, Rhizobium radiobacter, Rickettsia rickettsii, Rothia dentocariosa, Salmonella enteritidis, Salmonella typhi, Salmonella typhimurium, Shigella dysenteriae, Staphylococcus aureus, Staphylococcus epidermidis, Stenotrophomonas maltophilia, Streptococcus pneumoniae, Streptococcus pyogenes, Treponema pallidum, Treponema denticola, Vibrio cholerae, Vibrio comma, Vibrio parahaemolyticus, Vibrio vulnificus, Yersinia enterocolitica* and *Yersinia pseudotuberculosis.*

In certain instances, the cell is a viral cell. In certain instances, the viral cell is genetically engineered. In certain instances, the cell is a vaccine. In certain instances, the cell is non-infectious. In certain instances, the viral cell is a bacteriophage. Examples of viral cells include, but are not limited to, Adenoviruses, Herpesviruses, Poxviruses, Parvoviruses, Reoviruses, Picornaviruses, Togaviruses, Orthomyxoviruses, Rhabdoviruses, Retroviruses and Hepadnaviruses.

In certain instances, the cell is a fungal cell. In certain instances, the fungal cell is genetically engineered. In certain instances, the fungal cell is non-infectious. Examples of fungal cells include, but are not limited to, *Cryptococcus neoformans, Cryptococcus gattii, Candida albicans, Candida tropicalis, Candida stellatoidea, Candida glabrata, Candida krusei, Candida parapsilosis, Candida guilliermondii, Candida viswanathii, Candida lusitaniae, Rhodotorula mucilaginosa, Schizosaccharomyces pombe, Saccharomyces cerevisiae, Brettanomyces bruxellensis, Candida stellata, Schizosaccharomyces pombe, Torulaspora delbrueckii, Zygosaccharomyces bailii, Yarrowia lipolytica, Saccharomyces exiguus* and *Pichia pastoris.*

## *Exemplary Culture Media*

In some instances, the biocompatible hydrogel polymer matrix comprises a buffer or culture medium. In some instances, the biocompatible hydrogel polymer matrix comprises a buffer and at least one cell. In some instances, the culture medium is a buffer. In some instances, the culture medium comprises a growth medium. In some instances, the culture medium is nutrient rich. In certain instances, the culture medium provides nutrients sufficient for cell viability, growth, and/or proliferation. In certain instances, culture media include, but are not limited to, DMEM, IMDM, OptiMEM®, AlgiMatrix™, Fetal Bovine Serum, GS1-R®, GS2-M®, iSTEM®, NDiff® N2,NDiff® N2-AF, RHB-A®, RHB-Basal®, RPMI, SensiCell™, GlutaMAX™, FluoroBrite™, Gibco® TAP, Gibco® BG-11, LB, M9 Minimal, Terrific Broth, 2YXT, MagicMedia™, ImMedia™, SOC, YPD, CSM, YNB, Grace's Insect Media, 199/109 and HamF10/HamF12. In certain instances, the cell culture medium may be serum free. In certain instances, the culture medium includes additives. In some instances, culture medium additives include, but are not limited to, antibiotics, vitamins, proteins, inhibitors, small molecules, minerals, inorganic salts, nitrogen, growth factors, amino acids, serum, carbohydrates, lipids, hormones and glucose. In some instances, growth factors include, but are not limited to, EGF, bFGF, FGF, ECGF, IGF-1, PDGF, NGF, TGF-$\alpha$ and TGF-$\beta$. In certain instances, the culture medium may not be aqueous. In certain instances, the non-aqueous culture medium include, but are not limited to, frozen cell stocks, lyophilized medium and agar.

## *Exemplary Combinations*

In some instances, one or more optional additional components can be incorporated into the biocompatible hydrogel polymer matrix formulation. Provided herein are biocompatible pre-formulations, comprising at least one first compound comprising more than one nucleophilic group, at least one second compound comprising more than one electrophilic group, at least one cell, and optionally additional components. An exemplary additional component is a buffer. In certain embodiments, the cell is a stem cell. In certain instances, the additional component is a culture medium. In certain instances, the culture medium is nutrient rich. A biocompatible hydrogel polymer matrix is formed following mixing the first compound, the second compound, and the at least one cell in the presence of water; wherein the

biocompatible hydrogel polymer matrix gels at a target site. In some instances a buffer or other additional components may be added to the pre-formulation mix prior to or after biocompatible hydrogel polymer matrix formation. In some instances, the first compound and the second compound do not react with the at least one cell during formation of the biocompatible hydrogel polymer matrix. In certain instances, the biocompatible hydrogel polymer matrix comprises a biocompatible hydrogel scaffold. In certain instances, the biocompatible hydrogel scaffold comprises the at least one first compound and the at least one second compound. In certain instances, the biocompatible hydrogel scaffold comprises a buffer. In certain instances, the biocompatible hydrogel scaffold is fully synthetic.

Provided herein are biocompatible pre-formulations, comprising at least one first compound comprising more than one nucleophilic group, at least one second compound comprising more than one electrophilic group, a buffer, and optionally additional components. An exemplary additional component is at least one cell. In certain embodiments the cell is a stem cell. In certain instances, the buffer is a culture medium. In certain instances, the culture medium is nutrient rich. A biocompatible hydrogel polymer matrix is formed following mixing the first compound, the second compound, and the buffer in the presence of water; wherein the biocompatible hydrogel polymer matrix gels at a target site. In some instances at least one cell or other additional components may be added to the mix prior to or after biocompatible hydrogel polymer matrix formation. In some instances, the first compound and the second compound do not react with the at least one cell during formation of the biocompatible hydrogel polymer matrix. In certain instances, the biocompatible hydrogel polymer matrix comprises a biocompatible hydrogel scaffold. In certain instances, the biocompatible hydrogel scaffold comprises the at least one first compound, the at least one second compound and a buffer. In certain instances, the biocompatible hydrogel scaffold is fully synthetic.

In certain instances, the biocompatible pre-formulation or biocompatible hydrogel polymer matrix comprises at least one additional component. Additional components include, but are not limited to, proteins, biomolecules, growth factors, anesthetics, antibacterials, antivirals, immunosuppressants, anti-inflammatory agents, anti-proliferative agents, anti-angiogenesis agents and hormones.

In some instances, the biocompatible hydrogel polymer matrix or biocompatible pre-formulation further comprise a visualization agent for visualizing the placement of the biocompatible hydrogel polymer matrix at a target site The visualization agent assists in visualizing the placement using minimally invasive delivery, e.g., using an endoscopic device. In certain instances, the visualization agent is a dye. In specific instances, the visualization agent is a colorant.

In some instances, the biocompatible hydrogel polymer matrix formulations further comprise a contrast agent for visualizing the biocompatible hydrogel formulation and locating a tumor using e.g., X-ray, fluoroscopy, or computed tomography (CT) imaging. In certain instances, the contrast agent is radiopaque. In some instances, the radiopaque material is selected from sodium iodide, potassium iodide, barium sulfate, VISIPAQUE®, OMNIPAQUE®, or HYPAQUE®, tantalum, and similar commercially available compounds, or combinations thereof.

## EXAMPLES

The following specific examples are to be construed as merely illustrative, and not limitative of the remainder of the disclosure in any way whatsoever.

The following are general characteristics of the biocompatible pre-formulations and biocompatible hydrogel polymer matrices consistent with biocompatibility.

| | Pre-formulations Property | Characteristics |
|---|---|---|
| 1 | In vivo polymerizable | Could be polymerized inside mammalian cavity or over the skin |
| 2 | Reaction mixture pH | Physiological to 8.0 pH range |
| 3 | Reaction temperature | Ambient to body temperature |
| 4 | Formulation physical form | Two or three component system; Mixed immediately prior to use, may contain radiopaque agent such as barium sulphate or iodine containing organic compounds or other known radiopaque agents |
| 5 | Mixing time for the reaction to start | Few seconds (~10 sec) |
| 6 | Gel formation time | Gel formation time ranges from 10 seconds to 120 seconds, or could be as long as 30 minutes depending on the application |
| 7 | Solution viscosity | Solution viscosity ranges from 1 to 800 cps |
| 8 | Sterilization capability | ETO to E-beam sterilizable |

(continued)

| | Pre-formulations Property | Characteristics |
|---|---|---|
| 9 | Localized delivery | Ideal for localized delivery for small molecules, large molecules and cells |
| 10 | Stability of drugs in formulation mixture | All small molecule drugs and proteins studied so far have been found to be stable |

The following are some characteristics of adhesive biocompatible hydrogel polymer matrices.

| | Hydrogel Property | Characteristics |
|---|---|---|
| 1 | Tissue adhesion | Sticky formulations, physicochemical characteristics ideal for bonding to skin, bones, or other mammalian tissues |
| 2 | Polymer hardness | Can be controlled from soft tissues to harder cartilage like materials |
| 3 | Bioabsorption Time | About 2 weeks up to 10 years, or totally non-bioabsorbable |
| 4 | Biocompatibility | Highly biocompatible; passed all the subjected ISO 10993 tests |
| 5 | Polymer cytotoxicity | Non-cytotoxic formulations |
| 6 | Small molecule elution | Small drug molecules elution can be controlled and thus pharmaceutical drugs could also be delivered using the formulations, if needed |
| 7 | Compatibility with proteins and Cells | Highly compatible due to physiological pH of the polymers |

Biocompatible pre-formulation chemical components used to form biocompatible hydrogel polymer matrices are listed in *Table 1.* These biocompatible pre-formulation components will be referred to by their abbreviations. Several USP grade viscosity enhancing agents were purchased from Sigma-Aldrich and were stored at 25°C. They include methylcellulose (Methocel® MC, 10-25MPA.S) abbreviated as MC; hypromellose (hydroxypropylmethylcellulose 2910) abbreviated as HPMC; and povidone K-30 (polyvinylpyrrolidone) abbreviated as PVP.

The biocompatible pre-formulation components were stored at 5°C and allowed to warm to room temperature before use, which typically took 30 minutes. After use the contents were purged with $N_2$ for approximately 30 seconds before sealing with parafilm and returning to 5°C. Alternately, the biocompatible pre-formulation components were stored at -20°C and allowed to warm to room temperature before use under the flow of inert gas, which typically took 30 minutes. The biocompatible pre-formulation components were purged with inert gas for at least 30 seconds before returning to -20°C.

A 0.15 M phosphate buffer was made by dissolving 9.00 g (0.075 mol) $NaH_2PO_4$ in 500 mL of distilled water at 25°C with magnetic stirring. The pH was then adjusted to 7.99 with the dropwise addition of 50% aqueous NaOH. Several other phosphate buffers were prepared in a similar fashion: 0.10 M phosphate at pH 9, 0.10 M phosphate at pH 7.80, 0.10 M phosphate at 7.72, 0.10 M phosphate at pH 7.46, 0.15 M phosphate at pH 7.94, 0.15 M phosphate at pH 7.90, 0.4 M phosphate at pH 9, and 0.05 M phosphate at pH 7.40.

A sterile 0.10 M phosphate buffer at pH 7.58 with 0.30% HPMC was prepared for use in kits. First, 1.417 g HPMC was dissolved in 471 mL of 0.10 M phosphate buffer at pH 7.58 by vigorous shaking. The viscous solution was allowed to clarify overnight. The solution was filtered through a 0.22 μm filter (Corning #431097) with application of light vacuum. The viscosity of the resulting solution was measured to be 8.48 cSt +/- 0.06 at 20°C.

A sterile 0.10 M phosphate buffer at pH 7.58 with 0.3% HPMC was prepared. First, a 0.10 M phosphate buffer was made by dissolving 5.999 g (0.05 mol) of $NaH_2PO_4$ in 500 mL of distilled water at 20°C with magnetic stirring. The pH was then adjusted to 7.58 with the dropwise addition of 50% aqueous NaOH. Then, 1.5 g of HPMC was dissolved in 500 mL of the above buffer solution by vigorous shaking. The viscous solution was allowed to clarify overnight. The solution was filtered through a 0.22 μm filter (Corning #431097) with application of light vacuum. The viscosity of the resulting solution was measured via the procedure as described in the *Viscosity Measurements* section and was found to be 8.48 cSt +/- 0.06 at 20°C.

Phosphate buffered saline (PBS) was prepared by dissolving two PBS tablets (Sigma Chemical, P4417) in 400 mL of distilled water at 25°C with vigorous shaking. The solution has the following composition and pH: 0.01 M phosphate, 0.0027 M potassium chloride, 0.137 M sodium chloride, pH 7.46.

A 0.058 M phosphate buffer was made by dissolving 3.45 g (0.029 mol) of $NaH_2PO_4$ in 500 mL of distilled water at 25°C with magnetic stirring. The pH was then adjusted to 7.97 with the dropwise addition of 50% aqueous NaOH.

A 0.05 M borate buffer was made by dissolving 9.53 g (0.025 mol) of $Na_2B_4O_7 \cdot 10\ H_2O$ in 500 mL of distilled

water at 25°C with magnetic stirring. The pH was then adjusted to 7.93 or 8.35 with the dropwise addition of 6.0 N HCl.

An antiseptic liquid component was prepared in a similar fashion with a commercial 2% chlorhexidine solution. To 100 mL of 2% chlorhexidine solution was dissolved 0.3 g of HPMC. The viscous solution was allowed to clarify overnight at 5°C. The resulting clear blue solution has the following composition: 2% chlorhexidine, 0.3% HPMC and an unknown quantity of nontoxic blue dye and detergent.

Other liquid components were prepared in a similar fashion by simply dissolving the appropriate amount of the desired additive to the solution. For example, an antiseptic liquid component with 1% denatonium benzoate, a bittering agent, was prepared by dissolving 2 g of denatonium benzoate in 200 mL of 2% chlorhexidine solution.

Alternatively, commercially available drug solutions were used as the liquid component. For example, saline solution, Kenalog-10 (10 mg/mL solution of triamcinolone acetonide) and Depo-Medrol (40 mg/mL of methylprednisolone acetate) were used.

The amine or thiol component (typically in the range of 0.1 mmol arms equivalents) was added to a 50 mL centrifuge tube. A volume of reaction buffer was added to the tube via a pipette such that the final concentration of solids in solution was about 5 percent. The mixture was gently swirled to dissolve the solids before adding the appropriate amount of ester or epoxide. Immediately after adding the ester or epoxide, the entire solution was shaken for 10 seconds before letting it rest.

*Table 1. Components used in biocompatible_pre-formulations.*

| Pre-formulation Components | Technical Name |
|---|---|
| ETTMP-1300 | Ethoxylated trimethylolpropane tri(3-mercaptopropionate) |
| 4ARM-5k-SH | 4ARM PEG Thiol (pentaerythritol) |
| 4ARM-2k-NH2 | 4ARM PEG Amine (pentaerythritol), HCl Salt, MW 2000 |
| 4ARM-5k-NH2 | 4ARM PEG Amine (pentaerythritol), HCl Salt, MW 5000 |
| 8ARM-20k-NH2 | 8ARM PEG Amine (hexaglycerol), HCl Salt, MW 20000 |
| 4ARM-20k-AA | 4ARM PEG Acetate Amine HCl Salt, MW 20000 |
| 8ARM-20k-AA | 8ARM PEG Acetate Amine (hexaglycerol) HCl Salt, MW 20000 |
| 8ARM-20k-AA | 8ARM PEG Acetate Amine (hexaglycerol) TFA Salt, MW 20000 |
| 4ARM-10k-SG | 4ARM PEG Succinimidyl Glutarate (pentaerythritol), MW 10000 |
| 8ARM-15k-SG | 8ARM PEG Succinimidyl Glutarate (hexaglycerol), MW 15000 |
| 4ARM-20k-SGA | 4ARM PEG Succinimidyl Glutaramide (pentaerythritol), MW 20000 |
| 4ARM-10k-SS | 4ARM PEG Succinimidyl Succinate (pentaerythritol), MW 10000 |
| EJ-190 | Sorbitol polyglycidyl ether |
| MC | Methyl Cellulose (Methocel® MC) |
| HPMC | Hypromellose (Hydroxypropylmethylcellulose) |
| PVP | Povidone (polyvinylpyrrolidone) |

The gel time for all cases was measured starting from the addition of the ester or epoxide until the gelation of the solution. The gel point was noted by pipetting 1 mL of the reaction mixture and observing the dropwise increase in viscosity. Degradation of the polymers was performed by the addition of 5 to 10 mL of phosphate buffered saline to ca. 5 g of the material in a 50 mL centrifuge tube and incubating the mixture at 37°C. The degradation time was measured starting from the day of addition of the phosphate buffer to complete dissolution of the polymer into solution.

## Example 1: Manufacture of a Biocompatible Hydrogel Polymer matrix (Amine-Ester Chemistry)

A solution of 8ARM-20K-NH2 was prepared in a Falcon tube by dissolving about 0.13 g solid monomer in about 2.5 mL of sodium phosphate buffer (buffer pH 7.36). The mixture was shaken for about 10 seconds at ambient temperature until complete dissolution was obtained. The Falcon tube was allowed to stand at ambient temperature. In another Falcon tube, 0.10 g of 8ARM-15K-SG was dissolved in the same phosphate buffer as above. The mixture was shaken for about 10 seconds and at this point all the powder dissolved. The 8ARM-15K-SG solution was poured immediately into the 8ARM-20K-NH2 solution and a timer was started. The mixture was shaken and mixed for about 10 seconds and a 1 mL

solution of the mixture was pipetted out using a mechanical high precision pipette. The gel time of 1 mL liquid was collected and then verified with the lack of flow for the remaining liquids. The gel time data of the formulation was recorded and was about 90 seconds.

**Example 2: Manufacture of a Biocompatible Hydrogel Polymer matrix (Amine-Ester Chemistry)**

A solution of amines was prepared in a Falcon tube by dissolving about 0.4 g solid 4ARM-20k-AA and about 0.2 g solid 8ARM-20k-NH2 in about 18 mL of sodium phosphate buffer (buffer pH 7.36). The mixture was shaken for about 10 seconds at ambient temperature until complete dissolution was obtained. The Falcon tube was allowed to stand at ambient temperature. To this solution, 0.3 g of 8ARM-15K-SG was added. The mixture was shaken to mix for about 10 seconds until all the powder dissolved. 1 mL of the mixture was pipetted out using a mechanical high precision pipette. The gel time of the formulation was collected using the process described above. The gel time was about 90 seconds.

**Example 3: Manufacture of a Biocompatible Hydrogel Polymer matrix (Thiol-Ester Chemistry)**

A solution of ETTMP-1300 was prepared in a Falcon tube by dissolving about 0.04 g monomer in about 5 mL of sodium borate buffer (buffer pH 8.35). The mixture was shaken for about 10 seconds at ambient temperature until complete dissolution was obtained. The Falcon tube was allowed to stand at ambient temperature. To this solution, 0.20 g of 8ARM-15K-SG was added. The mixture was shaken for about 10 seconds until the powder dissolved. 1 mL of the mixture was pipetted out using a mechanical high precision pipette. The gel time was found to be about 70 seconds.

**Example 4: Manufacture of a Biocompatible Hydrogel Polymer matrix (Thiol-Epoxide Chemistry)**

A solution of ETTMP-1300 was prepared in a Falcon tube by dissolving about 0.04 g monomer in about 5 mL of sodium borate buffer (buffer pH 8.35). The mixture was shaken for about 10 seconds at ambient temperature until complete dissolution was obtained. The Falcon tube was allowed to stand at ambient temperature. To this solution, 0.10 g of EJ-190 was added. The mixture was shaken for about 10 seconds until complete dissolution is obtained. 1 mL of the mixture was pipetted out using a mechanical high precision pipette. The gel time was found to be about 6 minutes.

**Example 5: In vitro Bioabsorbance Testing**

A 0.10 molar buffer solution of pH 7.40 was prepared with deionized water. A 50 mL portion of this solution was transferred to a Falcon tube. A sample polymer was prepared in a 20 cc syringe. After curing, a 2-4 mm thick slice was cut from the polymer slug and was placed in the Falcon tube. A circulating water bath was prepared and maintained at 37°C. The Falcon tube with polymer was placed inside the water bath and time was started. The dissolution of the polymer was monitored and recorded. The dissolution time ranged from 1-90 days depending on the type of sample polymer.

**Example 6: Gelling and Degradation Times of Amine-Ester Polymers**

Amines studied were 8ARM-20k-NH2 and 4ARM-5k-NH2. The formulation details and material properties are given in *Table 2.* With 8ARM-20k-NH2, it was found that a phosphate buffer with 0.058 M phosphate and pH of 7.97 was necessary to obtain acceptable gel times of around 100 seconds. Using a 0.05 M phosphate buffer with a pH of 7.41 resulted in a more than two-fold increase in gel time (270 seconds).

With the 8ARM-20k-NH2, the ratio of 4ARM-10k-SS to 4ARM-20k-SGA was varied from 50:50 to 90:10. The gel time remained consistent, but there was a marked shift in degradation time around a ratio of 80:20. For formulations with ratios of 75:25 and 50:50, degradation times spiked to one month and beyond. Using lower amounts of 4ARM-20k-SGA (80:20, 85:15, 90:10) resulted in degradation times of less than 7 days.

As a comparison, the 4ARM-5k-NH2 was used in a formulation with a ratio of 4ARM-10k-SS to 4ARM-20k-SGA of 80:20. As was expected, the degradation time remained consistent, which suggests that the mechanism of degradation was unaffected by the change in amine. However, the gel time increased by 60 seconds, which may reflect the relative accessibility of reactive groups in a high molecular weight 8ARM amine and a low molecular weight 4ARM amine.

*Table 2. Gel and degradation times for varying 4ARM-10k-SS/4ARM-20k-SGA ratios with 8ARM-15k-SG ester.*

| Pre-formulation Components | Ratio of 4ARM-10k-SS / 4ARM-20k-SGA | Phosphate Reaction Buffer Concentration and pH | Gel Time (s) | Degradation Time (days) |
|---|---|---|---|---|
| 8ARM-20k-NH2 4ARM-10k-SS, 4ARM-20k-SGA | 50/50 | 0.05 M pH 7.41 | 270 | N/A |
| 8ARM-20k-NH2 4ARM-10k-SS, 4ARM-20k-SGA | 50/50 | 0.058 M pH 7.97 | 100 | >41 |
| 8ARM-20k-NH2 4ARM-10k-SS, 4ARM-20k-SGA | 75/25 | 0.058 M pH 7.97 | 90 | 29 |
| 8ARM-20k-NH2 4ARM-10k-SS, 4ARM-20k-SGA | 80/20 | 0.058 M pH 7.97 | 100 | 7 |
| 4ARM-5k-NH2 4ARM-10k-SS, 4ARM-20k-SGA | 80/20 | 0.058 M pH 7.97 | 160 | 6 |
| 8ARM-20k-NH2 4ARM-10k-SS, 4ARM-20k-SGA | 85/15 | 0.058 M pH 7.97 | 100 | 5 |
| 8ARM-20k-NH2 4ARM-10k-SS, 4ARM-20k-SGA | 90/10 | 0.058 M pH 7.97 | 90 | 6 |

**Example** 7: **Gelling and Degradation Times of Thiol-Ester Polymers**

Thiols studied were 4ARM-5k-SH and ETTMP-1300. The formulation details and material properties are given in *Table 3*. It was found that a 0.05 M borate buffer with a pH of 7.93 produced gel times of around 120 seconds. Increasing the amount of 4ARM-20k-SGA in the formulation increased the gel time to 190 seconds (25:75 ratio of 4ARM-10k-SS to 4ARM-20k-SGA) up to 390 seconds (0:100 ratio of 4ARM-10k-SS to 4ARM-20k-SGA). Using a 0.05 M borate buffer with a pH of 8.35 resulted in a gel time of 65 seconds, about a two-fold decrease in gel time. Thus, the gel time may be tailored by simply adjusting the pH of the reaction buffer.

The ratio of 4ARM-10k-SS to 4ARM-20k-SGA was varied from 0:100 to 100:0. In all cases, the degradation time did not vary significantly and was typically between 3 and 5 days. It is likely that degradation is occurring via alternate pathways.

*Table 3. Gel and degradation times for varying 4ARM-10k-SS/4ARM-20k-SGA ratios with 4ARM-5k-SH and ETTMP-1300 thiols.*

| Pre-formulation Components | Ratio of 4ARM-10k-SS / 4ARM-20k-SGA | Phosphate Reaction Buffer Concentration and pH | Gel Time (s) | Degradation Time (days) |
|---|---|---|---|---|
| 4ARM-5k-SH 4ARM-10k-SS, 4ARM-20k-SGA | 50/50 | 0.05 M pH 8.35 | 65 | N/A |
| 4ARM-5k-SH 4ARM-10k-SS, 4ARM-20k-SGA | 50/50 | 0.05 M pH 7.93 | 120 | 4 |
| 4ARM-5k-SH 4ARM-10k-SS, 4ARM-20k-SGA | 75/25 | 0.05 M pH 7.93 | 125 | 4 |

(continued)

| Pre-formulation Components | Ratio of 4ARM-10k-SS / 4ARM-20k-SGA | Phosphate Reaction Buffer Concentration and pH | Gel Time (s) | Degradation Time (days) |
|---|---|---|---|---|
| 4ARM-5k-SH 4ARM-10k-SS, 4ARM-20k-SGA | 90/10 | 0.05 M pH 7.93 | 115 | 4 |
| 4ARM-5k-SH 4ARM-10k-SS, 4ARM-20k-SGA | 25/75 | 0.05 M pH 7.93 | 190 | 4 |
| 4ARM-5k-SH 4ARM-10k-SS, 4ARM-20k-SGA | 10/90 | 0.05 M pH 7.93 | 200 | 4 |
| ETTMP-1300 4ARM-20k-SGA | 0/100 | 0.05 M | 390 | 3 |
| 4ARM-5k-SH 4ARM-10k-SS | 100/0 | 0.05 M pH 7.93 | 120 | 4 |

## Example 8: Gelling and Degradation Times of Amine-Ester and Thiol-Ester Polymers

An amine (4ARM-5k-NH2) and a thiol (4ARM-5k-SH) were studied with the ester 4ARM-10k-SG. The formulation details and material properties are given in *Table 4.* A 0.058 M phosphate buffer with a pH of 7.97 yielded a gel time of 150 seconds with the amine. A 0.05 M borate buffer with a pH of 8.35 produced a gel time of 75 seconds with the thiol.

The amine-based polymer appeared to show no signs of degradation, as was expected from the lack of degradable groups. However, the thiol-based polymer degraded in 5 days. This suggests that degradation is occurring through alternate pathways, as was observed in the thiol formulations with 4ARM-10k-SS and 4ARM-20k-SGA (vida supra).

*Table 4. Gel and degradation times for amines and thiols with 4ARM-10k-SG biocompatible pre-formulations.*

| Pre-formulation Components | Reaction Buffer Type, Concentration, and pH | Gel Time (s) | Degradation Time (days) |
|---|---|---|---|
| 4ARM-5k-NH2 & 4ARM-10k-SG | Phosphate (0.058 M, pH 7.97) | 150 | Indefinite |
| 4ARM-5k-SH & 4ARM-10k-SG | Borate (0.05 M, pH 8.35) | 75 | 5 |

## Example 9: Gelling and Degradation Times of Thiol-Sorbitol Polyglycidyl Ether Polymers

With ETTMP-1300 conditions such as high pH (10), high solution concentration (50%), or high borate concentration (0.16 M) were necessary for the mixture to gel. Gel times ranged from around 30 minutes to many hours. The conditions that were explored include: pH from 7 to 12; solution concentration from 5% to 50%; borate concentration from 0.05 M to 0.16 M; and thiol to epoxide ratios from 1:2 to 2:1.

The high pH necessary for the reaction to occur could result in degradation of the thiol. Thus, a polymer with EJ-190 and 4ARM-5k-SH was prepared. A 13% solution formulation exhibited a gel time of 230 seconds at a pH of between 9 and 10. The degradation time was 32 days. At a lower pH of around 8, the mixture exhibited gel times in the range of 1 to 2 hours.

## Example 10: General Procedure for the Preparation of Polymerizable Biocompatible Pre-Formulations

Several representative sticky formulations are listed in *Table 5* along with specific reaction details for the preparation of polymerizable biocompatible pre-formulations. The biocompatible hydrogel polymers were prepared by first dissolving the amine component in phosphate buffer or the thiol component in borate buffer. The appropriate amount of the ester component was then added and the entire solution was mixed vigorously for 10 to 20 seconds. The gel time

was measured starting from the addition of the ester until the gelation of the solution.

*Table 5. (A) Summary of the reaction details for several representative sticky formulations without viscosity enhancer; (B) more detailed tabulation of a selection of the reaction details including moles (degradation times were measured in phosphate buffered saline (PBS) at 37°C).*

| **(A)** | | | | | |
|---|---|---|---|---|---|
| **Pre-formulation Components** | **Amine or Thiol/ Ester Molar Ratio** | **Buffer** | **% Solution** | **Gel Time (s)** | **Degradation Time (days)** |
| 8ARM-20k-NH2 4ARM-20K-SGA | 3 | 0.15 M phosphate, pH 7.99 | 3 | 130 | N/A |
| 8ARM-20k-NH2 4ARM-20K-SGA | 1/3 | 0.15 M phosphate, pH 7.99 | 3 | 300 | N/A |
| 8ARM-20k-4ARM-10K-SS | 3 | 0.15 M phosphate, pH 7.99 | 8 | 50 | N/A |
| 8ARM-20k-NH2 4ARM-10K-SS | 1/3 | 0.15 M phosphate, pH 7.99 | 8 | 80 | N/A |
| 4ARM-20K-AA/8ARM-20k-NH2 (75/25) 4ARM-20K-SGA | 3 | 0.15 M phosphate, pH 7.99 | 5 | 210 | 1 to 3 |
| 4ARM-20K-AA/8ARM-20k-NH2 (75/25) 4ARM-20K-SGA | 5 | 0.15 M phosphate, pH 7.99 | 10 | 180 | 1 to 3 |
| 4ARM-5K-NH2 4ARM-10K-SG | 5 | 0.10 M phosphate, pH 7.80 | 10 | 160 | 7 |
| 4ARM-5K-NH2 4ARM-10K-SS | 5 | 0.10 M phosphate, pH 7.80 | 20 | 160 | 1 to 3 |
| 4ARM-5K-NH2 4ARM-10K-SG | 3 | 0.10 M phosphate, pH 7.80 | 5 | 160 | 13 |
| 4ARM-5K-NH2 4ARM-10K-SG | 5 | 0.15 M phosphate, pH 7.99 | 20 | 80 | 7 |

(continued)

| Pre-formulation Components | Amine or Thiol/ Ester Molar Ratio | Buffer | % Solution | Gel Time (s) | Degradation Time (days) |
|---|---|---|---|---|---|
| 4ARM-5K-NH2 4ARM-10K-SG | 5 | 0.15 M phosphate, pH 7.99 | 30 | 70 | 10 |
| 4ARM-5K-NH2 4ARM-20K-SGA | 5 | 0.15 M phosphate, pH 7.99 | 19 | 60 | 53 |
| 4ARM-5K-NH2 4ARM-20K-SGA | 5 | 0.15 M phosphate, pH 7.99 | 12 | 70 | 53 |
| 4ARM-5K-NH2 4ARM-10K-SG | 1/5 | 0.15 M phosphate, pH 7.99 | 19 | 160 | 15 |
| 4ARM-SH-5K 4ARM-10K-SG | 5 | 0.05 M borate, pH 7.93 | 20 | 120 | 2 to 4 |
| 4ARM-NH2-2K 8ARM-15K-SG | 5 | 0.10 M phosphate, pH 7.46 | 10 | 120 | 15 |
| 4ARM-NH2-2K 4ARM-20K-SGA | 7 | 0.10 M phosphate, pH 7.80 | 30 | 150 | N/A |

**(B)**

| Pre-formulation Components | MW | Mmoles | Wt (g) | Arm | mmoles | Arms Eq | Polymer % Solution (w/v) |
|---|---|---|---|---|---|---|---|
| 8ARM-20k-NH2 | 20000 | 1000 | 0.075 | 8 | 0.00375 | 0.03 | |
| 4ARM-20k-SGA | 20000 | 1000 | 0.05 | 4 | 0.0025 | 0.01 | |
| Buffer Volume (phosphate) | | | 4.1 | | | | 3.0 |
| | | | | | | | |
| 8ARM-20k-NH2 | 20000 | 1000 | 0.025 | 8 | 0.00125 | 0.01 | |
| 4ARM-20k-SGA | 20000 | 1000 | 0.15 | 4 | 0.0075 | 0.03 | |
| Buffer Volume (phosphate) | | | 5.8 | | | | 3.0 |
| | | | | | | | |

(continued)

| (B) | | | | | | | |
|---|---|---|---|---|---|---|---|
| **Pre-formulation Components** | **MW** | **Mmoles** | **Wt (g)** | **Arm** | **mmoles** | **Arms Eq** | **Polymer % Solution (w/v)** |
| 8ARM-20k-NH2 | 20000 | 1000 | 0.3 | 8 | 0.015 | 0.12 | |
| 4ARM-10k-SS | 10000 | 1000 | 0.1 | 4 | 0.01 | 0.04 | |
| Buffer Volume (phosphate) | | | 5 | | | | 8.0 |
| **Pre-formulation Components** | **MW** | **Mmoles** | **Wt (g)** | **Arm** | **mmoles** | **Arms Eq** | **Polymer % Solution (w/v)** |
| 8ARM-20k-NH2 | 20000 | 1000 | 0.1 | 8 | 0.005 | 0.04 | |
| 4ARM-10k-SS | 10000 | 1000 | 0.3 | 4 | 0.03 | 0.12 | |
| Buffer Volume (phosphate) | | | 5 | | | | 8.0 |

*Table 6. Gel times for the 8ARM-20k-NH2/4ARM-20k-SGA(1/1) sticky polymers including HPMC as viscosity enhancer with varying buffers and concentrations.*

| Pre-formulation Components | Amine/Ester Molar Ratio | Buffer | % Solution | Gel Time (min) |
|---|---|---|---|---|
| 8ARM-20k-NH2 4ARM-20K-SGA 0.3% HPMC | 1 | 0.10 M phosphate, pH 7.80 | 4.8 | 1.5 |
| 8ARM-20k-NH2 4ARM-20K-SGA 0.3% HPMC | 1 | 0.10 M phosphate, pH 7.46 | 4.8 | 3.5 |
| 8ARM-20k-NH2 4ARM-20K-SGA 0.3% HPMC | 1 | 0.05 M phosphate, pH 7.42 | 4.8 | 4.5 |
| 8ARM-20k-NH2 4ARM-20K-SGA 0.3% HPMC | 1 | 0.05 M phosphate, pH 7.42 | 4 | 5.5 |
| 8ARM-20k-NH2 4ARM-20K-SGA 0.3% HPMC | 1 | 0.05 M phosphate, pH 7.42 | 3 | 8.5 |
| 8ARM-20k-NH2 4ARM-20K-SGA 0.3% HPMC | 1 | 0.05 M phosphate, pH 7.24 | 4.8 | 6.75 |
| 8ARM-20k-NH2 4ARM-20K-SGA 0.3% HPMC | 1 | 0.05 M phosphate, pH 7.24 | 3 | 12 |
| 8ARM-20k-NH2 4ARM-20K-SGA 0.3% HPMC | 1 | 0.05 M phosphate, pH 7.24 | 2.5 | 15.5 |

Gel times ranged from 60 to 300 seconds and were found to be easily tuned by adjusting the reaction buffer pH, buffer concentration, or polymer concentration. An example of gel time control for a single formulation is shown in *Table 6*, where the gel time for the 8ARM-20k-NH2/4ARM-20k-SGA (1/1) polymer was varied from 1.5 to 15.5 minutes.

In some instances, the stickiness of the polymers originates from a mismatching in the molar equivalents of the components. A variety of sticky materials using combinations of 4 or 8 armed amines of molecular weights between 2 and 20 thousand and 4 or 8 armed esters of molecular weights between 10 and 20 thousand were created. It was found that in comparison with the 8 armed esters, the 4 armed esters resulted in stickier materials. For the amine component, it was found that smaller molecular weights led to stickier materials and higher amine to ester molar ratios.

A mismatch (amine to ester molar ratio) of at least 3 was required to qualitatively sense stickiness. More preferably, a ratio of around 5 produced a desirable level of stickiness combined with polymer strength. Polymers with amine to ester molar ratios higher than 5 may be formed as well, but some reaction conditions, such as the polymer concentration, may need to be adjusted to obtain a reasonable gel time. Furthermore, it was found that the use of a viscosity enhanced solution improves the polymers by increasing their strength and elasticity, allowing for higher amine to ester molar ratios (Example 11; *Table 9*).

The materials formed were typically transparent and elastic. Stickiness was tested for qualitatively by touch. Thus, a sticky material adhered to a human finger or other surface and remained in place until removed. Degradation times varied from 1 to 53 days. In certain instances, the polymer properties, such as gel and degradation times, pore sizes, swelling, etc. may be optimized for different applications without losing the stickiness.

**Example 11: General Procedure for the Preparation of Solutions with Enhanced Viscosity**

Polymer solutions with enhanced viscosities were prepared by the addition of a viscosity enhancing agent to the reaction buffer. *Table 9B* lists the viscosity enhancing agents studied, including observations on the properties of the formed polymers. Stock solutions of reaction buffers were prepared with varying concentrations of methylcellulose (MC), hypromellose (HPMC) or polyvinylpyrrolidone (PVP). As an example, a 2% (w/w) HPMC solution in buffer was made by adding 0.2 g of HPMC to 9.8 mL of 0.10 M phosphate buffer at pH 7.80, followed by vigorous shaking. The solution was allowed to stand overnight. Buffer solutions with HPMC concentrations ranging from 0.01% to 2.0% were prepared in a similar fashion. Buffer solutions with PVP concentrations ranging from 5% to 20% and buffer solutions with MC concentrations ranging from 1.0 to 2.0% were also prepared by a similar method.

The polymers were formed in the same method as described above in the general procedures for the preparation of the sticky materials (Example 10). A typical procedure involved first dissolving the amine component in the phosphate buffer containing the desired concentration of viscosity enhancing agent. The appropriate amount of the ester component was then added and the entire solution was mixed vigorously for 10 to 20 seconds. The gel time was measured starting from the addition of the ester until the gelation of the solution.

Several representative formulations are listed in *Table 7* and *Table 8* along with specific reaction details. The percent of degradable acetate amine component by mole equivalents is represented by a ratio designated in parenthesis. For example, a formulation with 75% degradable amine will be written as 8ARM-20k-AA/8ARM-20k-NH2 (75/25). The polymer was prepared by first dissolving the formulation amine component in phosphate buffer. The appropriate amount of the formulation ester component was then added and the entire solution was mixed vigorously for 10 to 20 seconds. The gel time was measured starting from the addition of the ester until the gelation of the solution.

The gel time is dependent on several factors: pH, buffer concentration, polymer concentration, temperature and the biocompatible pre-formulation monomers used. Previous experiments have shown that the extent of mixing has little effect on the gel time once the components are in solution, which typically takes up to 10 seconds. The effect of biocompatible pre-formulation monomer addition on buffer pH was measured. For the 8ARM-20k-NH2 & 4ARM-20k-SGA formulation, the buffer pH drops slightly from 7.42 to 7.36 upon addition of the biocompatible pre-formulation monomers. For the 8ARM-20k-AA/8ARM-20k-NH2 (70/30) & 4ARM-20k-SGA formulation, the buffer pH drops from 7.4 to 7.29 upon addition of the biocompatible pre-formulation monomers. The additional decrease in the pH was found to originate from acidic residues in the degradable acetate amine. The same pH drop phenomenon was observed for the 4ARM-20k-AA amine. In certain instances, a quality control specification on the acetate amine solution pH may be required to improve the consistency of degradable formulations.

The effect of reaction buffer pH on gel times was measured. The gel times increase with an increase in the concentration of hydronium ions in an approximately linear fashion. More generally, the gel times decrease with an increase in the buffer pH. In addition, the effect of reaction buffer phosphate concentration on gel times was determined. The gel times decrease with an increase in the phosphate concentration. Furthermore, the effect of polymer concentration on gel times was investigated. The gel times decrease significantly with an increase in the polymer concentration. At low polymer concentrations where the gel time is greater than 5 minutes, hydrolysis reactions of the ester begin to compete with the formation of the polymer. The effect of temperature on gel times appears to follow the Arrhenius equation. The gel time is directly related to the extent of reaction of the polymer solution and so this behavior is not unusual.

The rheology of the polymers during the gelation process as a function of the percent time to the gel point was determined. When 100% represents the gel point and 50% represents half the time before the gel point, the viscosity of the reacting solution remains relatively constant until about 80% of the gel point. After that point, the viscosity increases dramatically, representing the formation of the solid gel.

The gel time stability of a single formulation using the same lot of biocompatible pre-formulation monomers over the course of about a year was measured. The biocompatible pre-formulation monomers were handled according to the standard protocol outlined above. The gel times remained relatively stable; some variations in the reaction buffer may account for differences in the gel times.

*Table 7. (A) Summary of the reaction details for several representative sticky formulations; (B) more detailed tabulation of a selection of the reaction details including moles (degradation times were measured in phosphate buffered saline (PBS) at 37°C).*

| **(A)** | | | | |
|---|---|---|---|---|
| **Pre-formulation Components** | **Buffer** | **% Solution** | **Gel Time (s)** | **Degradation Time (days)** |
| 4ARM-20k-AA/8ARM-20k-NH2 (60/40)<br>4ARM-20k-SGA | 0.10 M phosphate, pH 7.80 | 5 | 150 | 21 |
| 4ARM-20k-AA/8ARM-20k-NH2 (60/40)<br>4ARM-20k-SGA<br>0.3% HPMC | 0.10 M phosphate, pH 7.80 | 5 | 150 | 21 |
| 8ARM-20k-NH2<br>4ARM-20k-SGA<br>0.3% HPMC | 0.10 M phosphate, pH 7.80 | 4.8 | 100 | N/A |
| 8ARM-20k-NH2<br>8ARM-15k-SG<br>0.3% HPMC | 0.10 M phosphate, pH 7.80 | 4.8 | 70 | 48 |
| 4ARM-20k-AA/8ARM-20k-NH2 (60/40)<br>8ARM-15k-SG<br>0.3% HPMC | 0.10 M phosphate, pH 7.80 | 4.8 | 110 | 12 |
| 4ARM-20k-AA/8ARM-20k-NH2 (60/40)<br>4ARM-20k-SGA<br>0.3% HPMC | 0.10 M phosphate, pH 7.80 | 20 | 160 | 21 |
| 8ARM-20k-NH2<br>4ARM-20k-SGA | 0.10 M phosphate, pH 7.80 | 4.8 | 90 | N/A |
| 8ARM-20k-NH2<br>4ARM-20k-SGA<br>1.0% HPMC | 0.10 M phosphate, pH 7.80 | 4.8 | 80 | N/A |
| 8ARM-20k-NH2<br>4ARM-20k-SGA<br>0.3% HPMC | 0.10 M phosphate, pH 7.46 | 4.8 | 210 | N/A |
| 8ARM-20k-NH2<br>4ARM-20k-SGA<br>0.3% HPMC | 0.05 M phosphate, pH 7.42 | 4.8 | 270 | N/A |

(continued)

| Pre-formulation Components | Buffer | % Solution | Gel Time (s) | Degradation Time (days) |
|---|---|---|---|---|
| 8ARM-20k-NH2<br>4ARM-20k-SGA<br>0.3% HPMC | 0.05 M phosphate, pH 7.42 | 4 | 330 | N/A |
| 8ARM-20k-NH2<br>4ARM-20k-SGA<br>0.3% HPMC | 0.05 M phosphate, pH 7.42 | 3 | 510 | N/A |
| 8ARM-20k-NH2<br>4ARM-20k-SGA<br>0.3% HPMC | 0.05 M phosphate, pH 7.24 | 4.8 | 405 | N/A |
| 8ARM-20k-NH2<br>4ARM-20k-SGA<br>0.3% HPMC | 0.05 M phosphate, pH 7.24 | 3 | 720 | N/A |
| 8ARM-20k-NH2<br>4ARM-20k-SGA<br>0.3% HPMC | 0.05 M phosphate, pH 7.24 | 2.5 | 930 | N/A |
| 8ARM-20k-AA<br>4ARM-20k-SGA<br>HPMC (0.3%) | 0.10 M phosphate, pH 7.46 | 4.8 | 90 | 6 |
| 8ARM-20k-AA/8ARM-20k-NH2 (75/25)<br>4ARM-20k-SGA<br>HPMC (0.3%) | 0.10 M phosphate, pH 7.46 | 4.8 | 100 | 16 |
| 8ARM-20k-AA/8ARM-20k-NH2 (60/40)<br>4ARM-20k-SGA<br>HPMC (0.3%) | 0.10 M phosphate, pH 7.46 | 4.8 | 95 | 256 (estimated) |
| 8ARM-20k-AA/8ARM-20k-NH2 (50/50)<br>4ARM-20k-SGA<br>HPMC (0.3%) | 0.10 M phosphate, pH 7.46 | 4.8 | 120 | N/A |
| 8ARM-20k-AA/8ARM-20k-NH2 (70/30)<br>4ARM-20k-SGA<br>HPMC (0.3%) | 0.10 M phosphate, pH 7.46 | 4.8 | 100 | 21 |
| 8ARM-20k-AA/8ARM-20k-NH2 (65/35)<br>4ARM-20k-SGA<br>HPMC (0.3%) | 0.10 M phosphate, pH 7.46 | 4.8 | 100 | 28 |
| 8ARM-20k-NH2<br>4ARM-20k-SGA<br>1.5% HPMC | 0.10 M phosphate, pH 7.80 | 4.8 | 90 | N/A |
| 8ARM-20k-AA/8ARM-20k-NH2 (75/25)<br>4ARM-20k-SGA<br>HPMC (0.3%) | 0.10 M phosphate, pH 7.46 | 4.8 | 90 | 16 |

(continued)

| Pre-formulation Components | Buffer | % Solution | Gel Time (s) | Degradation Time (days) |
|---|---|---|---|---|
| 8ARM-20k-AA/8ARM-20k-NH2 (70/30)<br>4ARM-20k-SGA<br>HPMC (0.3%) | 0.10 M phosphate, pH 7.46 | 4.8 | 105 | 21 |
| 8ARM-20k-AA/8ARM-20k-NH2 (50/50)<br>4ARM-20k-SGA<br>HPMC (0.3%) | 0.10 M phosphate, pH 7.46 | 4.8 | 120 | N/A |
| 8ARM-20k-AA/8ARM-20k-NH2 (70/30)<br>8ARM-15k-SG<br>HPMC (0.3%) | 0.10 M phosphate, pH 7.46 | 4.8 | 70 | 7 |
| 4ARM-20k-AA/8ARM-20k-NH2 (70/30)<br>4ARM-20k-SGA<br>HPMC (0.3%) | 0.10 M phosphate, pH 7.46 | 4.8 | 260 | 10 |
| 8ARM-20k-AA/8ARM-20k-NH2 (60/40)<br>8ARM-15k-SG<br>HPMC (0.3%) | 0.10 M phosphate, pH 7.46 | 4.8 | 70 | 17 |
| 8ARM-20k-AA<br>4ARM-20k-SGA<br>HPMC (0.3%) | 0.10 M phosphate, pH 7.46 | 4.8 | 85 | 7 |
| 8ARM-20k-AA/8ARM-20k-NH2 (70/30)<br>4ARM-20k-SGA<br>HPMC (0.3%) | 0.10 M phosphate, pH 7.46 | 4.8 | 95 | 13 |
| 8ARM-20k-AA/8ARM-20k-NH2 (75/25)<br>4ARM-20k-SGA<br>HPMC (0.3%) | 0.10 M phosphate, pH 7.46 | 4.8 | 95 | 10 |
| 8ARM-20k-AA/8ARM-20k-NH2 (75/25)<br>4ARM-20k-SGA<br>HPMC (0.3%) | 0.10 M phosphate, pH 7.58 | 4 | 110 | In Progress |
| 8ARM-20k-AA/8ARM-20k-NH2 (75/25)<br>4ARM-20k-SGA<br>HPMC (0.3%) | 0.10 M phosphate, pH 7.58 | 3.5 | 150 | In Progress |
| 8ARM-20k-AA/8ARM-20k-NH2 (75/25)<br>4ARM-20k-SGA<br>HPMC (0.3%) | 0.10 M phosphate, pH 7.58 | 3 | 190 | In Progress |

(continued)

| (B) | | | | | | | |
|---|---|---|---|---|---|---|---|
| **Pre-formulation Components** | **MW** | **Mmoles** | **Wt (g)** | **Arm** | **mmoles** | **Arms Eq** | **Polymer % Solution (w/v)** |
| 8ARM-20k-NH2 | 20000 | 1000 | 0.04 | 8 | 0.002 | 0.016 | |
| 4ARM-20k-SGA | 20000 | 1000 | 0.08 | 4 | 0.004 | 0.016 | |
| Buffer Volume (phosphate) | | | 2.5 | | | | 4.8 |
| Viscosity Enhancer | 0.3% HPMC | | | | | | |
| | | | | | | | |
| 8ARM-20k-NH2 | 20000 | 1000 | 0.08 | 8 | 0.004 | 0.032 | |
| 8ARM-15k-SG | 15000 | 1000 | 0.06 | 8 | 0.004 | 0.032 | |
| Buffer Volume (phosphate) | | | 2.9 | | | | 4.8 |
| Viscosity Enhancer | 0.3% HPMC | | | | | | |
| | | | | | | | |
| 8ARM-20k-AA | 20000 | 1000 | 0.04 | 8 | 0.002 | 0.016 ' | |
| 4ARM-20k-SGA | 20000 | 1000 | 0.08 | 4 | 0.004 | 0.016 | |
| Buffer Volume (phosphate) | | | 2.5 | | | | 4.8 |
| Viscosity Enhancer | 0.3% HPMC | | | | | | |
| | | | | | | | |
| 4ARM-20k-AA | 20000 | 1000 | 0.06 | 4 | 0.003 | 0.012 | |
| 8ARM-20k-NH2 | 20000 | 1000 | 0.02 | 8 | 0.001 | 0.008 | |
| 4ARM-20k-SGA | 20000 | 1000 | 0.1 | 4 | 0.005 | 0.02 | |
| Buffer Volume (phosphate) | | | 3.6 | | | | 5.0 |
| Viscosity Enhancer | 0.3% HPMC | | | | | | |
| | | | | | | | |
| 4ARM-20k-AA | 20000 | 1000 | 0.12 | 4 | 0.006 | 0.024 | |
| 8ARM-20k-NH2 | 20000 | 1000 | 0.04 | 8 | 0.002 | 0.016 | |
| 8ARM-15k-SG | 15000 | 1000 | 0.075 | 4 | 0.005 | 0.02 | |
| Buffer Volume (phosphate) | | | 4.9 | | | | 4.8 |
| Viscosity Enhancer | 0.3% HPMC | | | | | | |
| | | | | | | | |
| 8ARM-20k-AA | 20000 | 1000 | 0.06 | 8 | 0.003 | 0.024 | |

(continued)

| (B) | | | | | | | |
|---|---|---|---|---|---|---|---|
| **Pre-formulation Components** | **MW** | **Mmoles** | **Wt (g)** | **Arm** | **mmoles** | **Arms Eq** | **Polymer % Solution (w/v)** |
| 8ARM-20k-NH2 | 20000 | 1000 | 0.02 | 8 | 0.001 | 0.008 | |
| 4ARM-20k-SGA | 20000 | 1000 | 0.16 | 4 | 0.008 | 0.032 | |
| Buffer Volume (phosphate) | | | 5 | | | | 4.8 |
| Viscosity Enhancer | 0.3% HPMC | | | | | | |
| | | | | | | | |
| 8ARM-20k-AA | 20000 | 1000 | 0.03 | 8 | 0.0015 | 0.012 | |
| 8ARM-20k-NH2 | 20000 | 1000 | 0.02 | 8 | 0.001 | 0.008 | |
| 4ARM-20k-SGA | 20000 | 1000 | 0.1 | 4 | 0.005 | 0.02 | |
| Buffer Volume (phosphate) | | | 3.1 | | | | 4.8 |
| Viscosity Enhancer | 0.3% HPMC | | | | | | |
| **Pre-formulation Components** | **MW** | **Mmoles** | **Wt (g)** | **Arm** | **mmoles** | **Arms Eq** | **Polymer % Solution (w/v)** |
| 8ARM-20k-AA | 20000 | 1000 | 0.02 | 8 | 0.001 | 0.008 | |
| 8ARM-20k-NH2 | 20000 | 1000 | 0.02 | 8 | 0.001 | 0.008 | |
| 4ARM-20k-SGA | 20000 | 1000 | 0.08 | 4 | 0.004 | 0.016 | |
| Buffer Volume (phosphate) | | | 2.5 | | | | 4.8 |
| Viscosity Enhancer | 0.3% HPMC | | | | | | |
| | | | | | | | |
| 8ARM-20k-AA | 20000 | 1000 | 0.035 | 8 | 0.00175 | 0.014 | |
| 8ARM-20k-NH2 | 20000 | 1000 | 0.015 | 8 | 0.00075 | 0.006 | |
| 4ARM-20k-SGA | 20000 | 1000 | 0.1 | 4 | 0.005 | 0.02 | |
| Buffer Volume (phosphate) | | | 3.1 | | | | 4.8 |
| Viscosity Enhancer | 0.3% HPMC | | | | | | |
| | | | | | | | |
| 8ARM-20k-AA | 20000 | 1000 | 0.039 | 8 | 0.00195 | 0.0156 | |
| 8ARM-20k-NH2 | 20000 | 1000 | 0.021 | 8 | 0.00105 | 0.0084 | |
| 4ARM-20k-SGA | 20000 | 1000 | 0.12 | 4 | 0.006 | 0.024 | |
| Buffer Volume (phosphate) | | | 3.75 | | | | 4.8 |

(continued)

| Pre-formulation Components | MW | Mmoles | Wt (g) | Arm | mmoles | Arms Eq | Polymer % Solution (w/v) |
|---|---|---|---|---|---|---|---|
| Viscosity Enhancer | 0.3% HPMC | | | | | | |
| | | | | | | | |
| 8ARM-20k-AA | 20000 | 1000 | 0.09 | 8 | 0.0045 | 0.036 | |
| 8ARM-20k-NH2 | 20000 | 1000 | 0.03 | 8 | 0.0015 | 0.012 | |
| 4ARM-20k-SGA | 20000 | 1000 | 0.24 | 4 | 0.012 | 0.048 | |
| Buffer Volume (phosphate) | | | 9 | | | | 4.0 |
| Viscosity Enhancer | 0.3% HPMC | | | | | | |
| | | | | | | | |
| 8ARM-20k-AA | 20000 | 1000 | 0.075 | 8 | 0.00375 | 0.03 | |
| 8ARM-20k-NH2 | 20000 | 1000 | 0.025 | 8 | 0.00125 | 0.01 | |
| 4ARM-20k-SGA | 20000 | 1000 | 0.2 | 4 | 0.01 | 0.04 | |
| Buffer Volume (phosphate) | | | 8.55 | | | | 3.5 |
| Viscosity Enhancer | 0.3% HPMC | | | | | | |
| | | | | | | | |
| 8ARM-20k-AA | 20000 | 1000 | 0.06 | 8 | 0.003 | 0.024 | |
| 8ARM-20k-NH2 | 20000 | 1000 | 0.02 | 8 | 0.001 | 0.008 | |
| 4ARM-20k-SGA | 20000 | 1000 | 0.16 | 4 | 0.008 | 0.032 | |
| Buffer Volume (phosphate) | | | 8 | | | | 3.0 |
| Viscosity Enhancer | 0.3% HPMC | | | | | | |

*Table 8. (A) Summary of the reaction details for several representative sticky formulations; (B) more detailed tabulation of a selection of the reaction details including moles (degradation times were measured in phosphate buffered saline (PBS) at 37°C).*

| (A) | | | | | | |
|---|---|---|---|---|---|---|
| **Components (Arm Equiv. Mol%)** | | **Poly. Conc.** | **Buffer Type & Comp onents** | | **Estim. Deg. Time** | **Appr. Gel Time** |
| 4ARM-20k-SGA | 100% | 5% | Liquid | 0.10M Phosphate, pH 7.58 | 2 to 4 weeks | 125 s |
| 8ARM-20k-AA | 65% | | 2.5 mL | | | |
| 8ARM-20k-NH2 | 35% | | | | | |
| HPMC | 0.3% | | | | | |

(continued)

| (A) | | | | | | |
|---|---|---|---|---|---|---|
| **Components (Arm Equiv. Mol%)** | | **Poly. Conc.** | **Buffer Type & Comp onents** | | **Estim. Deg. Time** | **Appr. Gel Time** |
| 4ARM-20k-SGA | 100% | 5% | Liquid | 0.10M Phosphate, pH 7.58 | 2 weeks | 115 s |
| 8-ARM-20k-AA | 75% | | 2.5 mL | | | |
| 8ARM-20k-NH2 | 25% | | | | | |
| HPMC | 0.3% | | | | | |
| 4ARM-20k-SGA | 100% | 5% | Liquid | 0.10M Phosphate, pH 7.58 | 2 weeks | 155s |
| 8ARM-20k-AA | 70% | | 2.5 mL | | | |
| 8ARM-20k-NH2 | 30% | | | | | |
| HPMC | 0.3% | | | | | |
| 4ARM-20k-SGA | 100% | 5% | Liquid | 0.10M Phosphate, pH 7.58 | 2 weeks | 110 s to 125 s |
| 8ARM-20k-AA | 75% | | 2.5 mL | | | |
| 8ARM-20k-NH2 | 25% | | | | | |
| HPMC | 0.3% | | | | | |
| 4ARM-20k-SGA | 100% | 5% | Liquid | 0.10M Phosphate, pH 7.58 | 2 weeks | 122 s |
| 8ARM-20k-AA | 75% | | 2.5 mL | | | |
| 8ARM-20k-NH2 | 25% | | | | | |
| HPMC | 0.3% | | | | | |
| 4ARM-20k-SGA | 100% | 5% | Liquid | 0.10M Phosphate, pH 7.58 | 2 weeks | 90s to 120 s |
| 8ARM-20k-AA | 75% | | 2.5 mL | | | |
| 8ARM-20k-NH2 | 25% | | | | | |
| HPMC | 0.3% | | 1000 ppm Denatonium benzoate | | | |
| 4ARM-20k-SGA | 100% | 5% | Liquid | 0.10M Phosphate, pH 7.58 | 2 weeks | 90s to 120 s |
| 8ARM-20k-AA | 75% | | 2.5 mL | | | |
| 8ARM-20k-NH2 | 25% | | | | | |
| HPMC | 0.3% | | 500 ppm Denatonium benzoate | | | |
| 4ARM-20k-SGA | 100% | 5% | Liquid | 0.10M Phosphate, pH 7.58 | 2 weeks | 90s to 120s |
| 8ARM-20k-AA | 75% | | 2.5 mL | | | |
| 8ARM-20k-NH2 | 25% | | | | | |
| HPMC | 0.3% | | 100 ppm Denatonium benzoate | | | |
| 4ARM-20k-SGA | 100% | 5% | Liquid | 0.10M Phosphate, pH 7.58 | 2 weeks | 130 s |
| 8ARM-20k-AA | 70% | | 2.5 mL | | | |
| 8ARM-20k-NH2 | 30% | | | | | |
| HPMC | 0.3% | | | | | |

(continued)

| (A) | | | | | | | |
|---|---|---|---|---|---|---|---|
| **Components (Arm Equiv. Mol%)** | | **Poly. Conc.** | **Buffer Type & Comp onents** | | **Estim. Deg. Time** | **Appr. Gel Time** |
| 4ARM-20k-SGA | 100% | 4% | Liquid | 0.10M Phosphate, pH 7.46 | 2 weeks | 205 s to 230 s |
| 8ARM-20k-AA | 60% | | 2.25 mL | | | |
| 8-ARM-20k-NH2 | 40% | | | | | |
| HPMC | 0.3% | | | | | |
| 4ARM-20k-SGA | 100% | 6% | Solid | 0.10M Phosphate, pH 7.4 | 30-60 days | 90s |
| 8ARM-20k-AA | 65% | | Freeze-dried (Aldrich) | | | |
| 8ARM-20k-NH2 | 35% | | Suggested use w/ 2 mL drug solution | | | |
| 4ARM-20k-SGA | 100% | 5% | Liquid | 0.10M Phosphate, pH 7.58 | 2 weeks | 90s to 120 s |
| 8ARM-20k-AA | 75% | | 2.5 mL | | | |
| 8ARM-20k-NH2 | 25% | | | | | |
| HPMC | 0.3% | | 10000 ppm Denatonium benzoate | | | |
| 4ARM-20k-SGA | 100% | 5% | Liquid | 0.10M Phosphate, pH 7.58 | 2 weeks | 115 s |
| 8ARM-20k-AA | 75% | | 2.5 mL | | | |
| 8ARM-20k-NH2 | 25% | | | | | |
| HPMC | 0.3% | | | | | |
| 4ARM-20k-SGA | 100% | 5% | Liquid | 0.10 M Phosphate, pH 7.4 | 2 weeks | 150 s |
| 8ARM-20k-AA | 75% | | 2.5 mL | | | |
| 8ARM-20k-NH2 | 25% | | Using freeze-dried phosphate | | | |
| | | | 1% Denatonium benzoate, 2% Chlorhexidine | | | |
| 4ARM-20k-SGA | 100% | 6% | Solid | 0.10M Phosphate, pH 7.4 | 2 weeks | 110 s |
| 8ARM-20k-AA | 75% | | Freeze-dried (Aldrich) | | | |
| 8ARM-20k-NH2 | 25% | | Suggested use w/ 2 mL drug solution | | | |
| 4ARM-20k-SGA | 100% | 6% | Liquid | 0.01 M Phosphate, 0.137 M NaCl, 0.0027 M KC1, pH 7.2 | 2 weeks | 27 min to 31 min |
| 8ARM-20k-AA | 70% | | 2.0 mL | | | |
| 8ARM-20k-NH2 | 30% | | Phosphate Buffered Saline (PBS) | | | |
| HPMC | 0.3% | | | | | |
| 4ARM-20k-SGA | 100% | 5% | Liquid | 0.10M Phosphate, pH 7.4 | 2 weeks | 158 s |
| 8ARM-20k-AA | 70% | | 2.5 mL | | | |
| 8ARM-20k-NH2 | 30% | | Nolvasan (2% Chlorhexidine) | | | |

(continued)

| (B) | | | | | | | |
|---|---|---|---|---|---|---|---|
| **Components** | **MW** | **Mmoles** | **Wt (g)** | **Arm** | **mmoles** | **Arms Eq** | **Pol.% Sol. (w/v)** |
| 8ARM-20k-AA | 20000 | 1000 | 0.03 | 8 | 0.0015 | 0.012 | |
| 8ARM-20k-NH2 | 20000 | 1000 | 0.01 | 8 | 0.0005 | 0.004 | |
| 4ARM-20k-SGA | 20000 | 1000 | 0.08 | 4 | 0.004 | 0.016 | |
| Buffer Volume (phosphate) | | | 2.5 | | | | 4.8 |
| Viscosity Enhancer | 0.3% HPMC | | | | | | |
| | | | | | | | |
| 8ARM-20k-AA | 20000 | 1000 | 0.03 | 8 | 0.0015 | 0.012 | |
| 8ARM-20k-NH2 | 20000 | 1000 | 0.01 | 8 | 0.0005 | 0.004 | |
| 4ARM-20k-SGA | 20000 | 1000 | 0.08 | 4 | 0.004 | 0.016 | |
| Buffer Volume (phosphate) | | | 2.5 | | | | 4.8 |
| Denatonium benzoate | | 1000 ppm | | | | | |
| Viscosity Enhancer | 0.3% HPMC | | | | | | |
| | | | | | | | |
| 8ARM-20k-AA | 20000 | 1000 | 0.03 | 8 | 0.0015 | 0.012 | |
| 8ARM-20k-NH2 | 20000 | 1000 | 0.01 | 8 | 0.0005 | 0.004 | |
| 4ARM-20k-SGA | 20000 | 1000 | 0.08 | 4 | 0.004 | 0.016 | |
| Buffer Volume (phosphate) | | | 2.5 | | | | 4.8 |
| Denatonium benzoate | | 500 ppm | | | | | |
| Viscosity Enhancer | 0.3% HPMC | | | | | | |
| | | | | | | | |
| 8ARM-20k-AA 20000 | | 1000 | 0.03 | 8 | 0.0015 | 0.012 | |
| 8ARM-20k-NH2 | 20000 | 1000 | 0.01 | 8 | 0.0005 | 0.004 | |
| 4ARM-20k-SGA | 20000 | 1000 | 0.08 | 4 | 0.004 | 0.016 | |
| Buffer Volume (phosphate) | | | 2.5 | | | | 4.8 |
| Denatonium benzoate | | 100 ppm | | | | | |
| Viscosity Enhancer | 0.3% HPMC | | | | | | |
| 8ARM-20k-AA | 20000 | 1000 | 0.03 | 8 | 0.0015 | 0.012 | |
| 8ARM-20k-NH2 | 20000 | 1000 | 0.01 | 8 | 0.0005 | 0.004 | |

(continued)

| (B) | | | | | | | |
|---|---|---|---|---|---|---|---|
| **Components** | **MW** | **Mmoles** | **Wt (g)** | **Arm** | **mmoles** | **Arms Eq** | **Pol.% Sol. (w/v)** |
| 4ARM-20k-SGA | 20000 | 1000 | 0.08 | 4 | 0.004 | 0.016 | |
| Buffer Volume (phosphate) | | | 2.5 | | | | 4.8 |
| Denatonium benzoate | | 10000 ppm | | | | | |
| Viscosity Enhancer | 0.3% HPMC | | | | | | |
| | | | | | | | |
| 8ARM-20k-AA | 20000 | 1000 | 0.03 | 8 | 0.0015 | 0.012 | |
| 8ARM-20k-NH2 | 20000 | 1000 | 0.01 | 8 | 0.0005 | 0.004 | |
| 4ARM-20k-SGA | 20000 | 1000 | 0.08 | 4 | 0.004 | 0.016 | |
| Solid Phosphate | | | 0.043 | | | | |
| Nolvasan Volume (2% chlorhexidine) | | | 2.5 | | | | 4.8 |
| Denatonium benzoate | | 10000 ppm | | | | | |
| | | | | | | | |
| 8ARM-20k-AA | 20000 | 1000 | 0.026 | 8 | 0.0013 | 0.0104 | |
| 8ARM-20k-NH2 | 20000 | 1000 | 0.014 | 8 | 0.0007 | 0.0056 | |
| 4ARM-20k-SGA | 20000 | 1000 | 0.08 | 4 | 0.004 | 0.016 | |
| Buffer Volume (phosphate) | | | 2.5 | | | | 4.8 |
| Viscosity Enhancer | 0.3% HPMC | | | | | | |
| | | | | | | | |
| 8ARM-20k-AA | 20000 | 1000 | 0.028 | 8 | 0.0014 | 0.0112 | |
| 8ARM-20k-NH2 | 20000 | 1000 | 0.012 | 8 | 0.0006 | 0.0048 | |
| 4ARM-20k-SGA | 20000 | 1000 | 0.08 | 4 | 0.004 | 0.016 | |
| Buffer Volume (phosphate) | | | 2.5 | | | | 4.8 |
| Viscosity Enhancer | 0.3% HPMC | | | | | | |
| | | | | | | | |
| 8ARM-20k-AA | 20000 | 1000 | 0.018 | 8 | 0.0009 | 0.0072 | |
| 8ARM-20k-NH2 | 20000 | 1000 | 0.012 | 8 | 0.0006 | 0.0048 | |
| 4ARM-20k-SGA | 20000 | 1000 | 0.06 | 4 | 0.003 | 0.012 | |
| Buffer Volume (phosphate) | | | 2.25 | | | | 4 |

(continued)

| (B) | | | | | | | |
|---|---|---|---|---|---|---|---|
| **Components** | **MW** | **Mmoles** | **Wt (g)** | **Arm** | **mmoles** | **Arms Eq** | **Pol.% Sol. (w/v)** |
| Viscosity Enhancer | 0.3% HPMC | | | | | | |
| **Components** | **MW** | **Mmoles** | **Wt (g)** | **Arm** | **mmoles** | **Arms Eq** | **Pol.% Sol. (w/v)** |
| | | | | | | | |
| 8ARM-20k-AA | 20000 | 1000 | 0.026 | 8 | 0.0013 | 0.0104 | |
| 8ARM-20k-NH2 | 20000 | 1000 | 0.014 | 8 | 0.0007 | 0.0056 | |
| 4ARM-20k-SGA | 20000 | 1000 | 0.08 | 4 | 0.004 | 0.016 | |
| Solid Phosphate | | | 0.035 | | | | 6 |
| Drug Solution | 2.0 mL | | | | | | |
| | | | | | | | |
| 8ARM-20k-AA | 20000 | 1000 | 0.027 | 8 | 0.00135 | 0.0108 | |
| 8ARM-20k-NH2 | 20000 | 1000 | 0.009 | 8 | 0.00045 | 0.0036 | |
| 4ARM-20k-SGA | 20000 | 1000 | 0.072 | 4 | 0.0036 | 0.0144 | |
| Solid Phosphate | | | 0.035 | | | | 5.4 |
| Drug Solution | 2.0 mL | | | | | | |
| | | | | | | | |
| 8ARM-20k-AA | 20000 | 1000 | 0.028 | 8 | 0.0014 | 0.0112 | |
| 8ARM-20k-NH2 | 20000 | 1000 | 0.012 | 8 | 0.0006 | 0.0048 | |
| 4ARM-20k-SGA | 20000 | 1000 | 0.08 | 4 | 0.004 | 0.016 | |
| Buffer Volume (phosphate) | | | 2 | | | | 6 |
| Viscosity Enhancer | 0.3% HPMC | | | | | | |
| | | | | | | | |
| 8ARM-20k-AA | 20000 | 1000 | 0.028 | 8 | 0.0014 | 0.0112 | |
| 8ARM-20k-NH2 | 20000 | 1000 | 0.012 | 8 | 0.0006 | 0.0048 | |
| 4ARM-20k-SGA | 20000 | 1000 | 0.08 | 4 | 0.004 | 0.016 | |
| Solid Phosphate | | | 0.043 | | | | |
| Nolvasan Volume (2% chlorhexidine) | | | 2.5 | | | | 4.8 |
| Denatonium benzoate | | 1% | | | | | |

Cytotoxicity & Hemolysis Evaluation

Several polymer samples were sent out to NAMSA for cytotoxicity and hemolysis evaluation. Cytotoxic effects

were evaluated according to ISO 10993-5 guidelines. Hemolysis was evaluated according to procedures based on ASTM F756 and ISO 10993-4.

The polymer 8ARM-20k-NH2 & 4ARM-20k-SGA at 4.8% solution with 0.3% HPMC was found to be non-cytotoxic and non-hemolytic. The polymer 8ARM-20k-AA/8ARM-20k-NH2 (70/30) & 4ARM-20k-SGA at 4.8% solution with 0.3% HPMC was found to be non-cytotoxic and non-hemolytic. In addition, formulations involving 4ARM-20kAA and 8ARM-15k-SG were also non-cytotoxic and non-hemolytic.

Gel and Degradation Time Measurements

The gel time for all cases was measured starting from the addition of the ester until the gelation of the solution. The gel point was noted by pipetting 1 mL of the reaction mixture and observing the dropwise increase in viscosity until the mixture ceased to flow. Degradation of the polymers was performed by the addition of 1 to 10 mL of phosphate buffered saline per 1 g of the material in a 50 mL centrifuge tube and incubating the mixture at 37°C. A digital water bath was used to maintain the temperature. The degradation time was measured starting from the day of addition of the phosphate buffer to complete dissolution of the polymer into solution.

The effect of reaction buffer pH, phosphate concentration, polymer concentration and reaction temperature on the gel times were characterized. The buffer pH was varied from 7.2 to 8.0 by the dropwise addition of either 50% aqueous NaOH or 6.0 N HC1. Phosphate concentrations of 0.01, 0.02 and 0.05 M were prepared and adjusted to pH 7.4. Polymer concentrations from 2 to 20% solution were studied. Reaction temperatures of 5,20, and 37°C were tested by keeping the monomers, buffers, and reaction mixture at the appropriate temperature. The 5°C environment was provided by a refrigerator and the 37°C temperature was maintained via the water bath. Room temperature was found to be 20°C.

The effect of degradation buffer pH and the proportion of degradable amine in the polymer formulation on the degradation times were explored. The degradation buffer pH was varied from 7.2 to 9.0 by the dropwise addition of either 50% aqueous NaOH or 6.0 N HCl. The degradable amine components studied were either the 4ARM-20k-AA or the 8ARM-20k-AA, and the percent of degradable amine relative to the non-degradable amine was varied from 50 to 100%.

The degradation time is largely dependent on the buffer pH, temperature, and the biocompatible pre-formulation monomers used. Degradation occurs primarily through ester bond hydrolysis; in biological systems, enzymatic pathways may also play a role. Figure 1 compares the degradation times of formulations with 4ARM-20k-AA and 8ARM-20k-AA in varying amounts. In general, increasing the amount of degradable acetate amine in relation to the non-degradable amine decreases the degradation times. Additionally, in some instances, the 8ARM-20k-AA exhibits a longer degradation time than the 4ARM-20k-AA per mole equivalent, which becomes especially apparent when the percent of acetate amine drops below 70%.

The effect of the buffer pH on the degradation time was investigated. The pH range between 7.2 and 9.0 was studied. In general, a high pH environment results in a greatly accelerated degradation. For example, an increase in pH from approximately 7.4 to 7.7 decreases the degradation time by about half.

The degradation time of different Acetate Amine formulations was evaluated. The formulation with 70% Acetate Amine has a degradation time of approximately 14 days whereas the formulation with 62.5% Acetate Amine has a degradation time of approximately 180 days.

**Figure** 2 shows the effect of polymer concentration on degradation time for different Acetate Amine formulations, where increasing polymer concentration slightly increases the degradation time (75% Acetate Amine formulation). This effect is less apparent for 100% Acetate Amine formulation, where the rate of ester hydrolysis is more significant.

The monomers used in the formulations have also been found to play a role in the way the polymer degrades. For the 8ARM-20k-AA/8ARM-20k-NH2 (70/30) & 4ARM-20k-SGA polymer, degradation occurred homogeneously throughout the material, resulting in a "smooth" degradation process. The polymer absorbed water and swelled slightly over the initial few days. Then, the polymer became gradually softer yet maintained its shape. Finally, the polymer lost its shape and became a highly viscous fluid.

Fragmenting degradation processes are observed when the amount of degradable amine becomes low, non-degradable regions in the polymer may occur. For instance a 4ARM-20k-AA/8ARM-20k-NH2 (70/30) & 4ARM-20k-SGA formulation degraded into several large fragments. For applications where the polymers are subjected to great forces, fragmentation may also occur as the polymer becomes softer and weaker over time.

Polymer Concentration

More dilute polymer solutions may be employed with minimal changes in the mechanical properties. For the formulation 8ARM-20k-AA-20K/8ARM-20k-NH2 (75/25) with 4ARM-20k-SGA and 0.3% HPMC, polymer concentrations of 3.0, 3.5 and 4.0% were studied. The gel times increased steadily as the polymer concentration was lowered. The firmness decreased slightly as the polymer concentration was lowered. There was essentially no change in the polymer

adhesive properties. The elastic modulus decreased slightly as the polymer concentration was lowered.

*Table 9. (A) Reaction details for specific sticky formulation; (B) Formulation results for $\alpha$ specific sticky formulation with $\alpha$ variety of viscosity enhancing agents (the biocompatible hydrogel surface spread test is conducted on $\alpha$ hydrophilic biocompatible hydrogel surface composed of 97.5% water at an angle of approximately 30°; one drop of the polymer solution from $\alpha$ 22 gauge needle is applied to the surface before gelation); (C) the clarity of solutions containing $\alpha$ variety of viscosity enhancing agents, as measured by the % transmission at 650 nm.*

**(A)**

| Pre-formulation Components | MW | wt (g) | Arm | mmoles | Arms Eq | % Solution |
|---|---|---|---|---|---|---|
| 8ARM-20k-NH2 | 20000 | 0.04 | 8 | 0.002 | 0.016 | |
| 4ARM-20k-SGA | 20000 | 0.08 | 4 | 0.004 | 0.016 | |
| Phosphate buffer | | 2.5 mL 0.10 M, pH 7.80 | | | | 4.8 |

**(B)**

| **Viscous Agent % (w/w)** | **Approx. Viscosity (cP)** | **Gel Time (s)** | **Hydrogel Surface Spread Test Category** | **Notes** |
|---|---|---|---|---|
| 0 (Original Formulation) | 1.1 | 80 | 2 | Rigid, has "bounce". Slight elasticity. |
| 5% PVP | 1 to 5 | 90 | 2 to 3 | No change, except for a slight increase in elasticity. |
| 10% PVP | 3 to 5 | 90 | 2 to 3 | Slightly opaque, moderate increase in elasticity. Slippery. |
| 15% PVP | 5 to 10 | 100 | 2 to 3 | Opaque, definite increase in elasticity. Slippery when wet, slightly sticky when dry. |
| 20% PVP | 10 | 110 | 2 | Opaque, definite increase in elasticity. Slippery when wet, very sticky when dry. |
| 0.3% HPMC | 8.4 | 80 | 2 | No change. |
| 1.0% HPMC | 340.6 | 90 | 1 | No change. |
| 1.25% HPMC | 1,000 | 90 | 1 | No change. |
| 1.5% HPMC | 2,000 | 100 | 1 | Slightly softer, lacks "bounce". |
| 2.0% HPMC | 4,000 | 100 | 1 | Slightly softer, lacks "bounce". Slippery. |

**(C)**

| Sample | % Transmission @ 650 nm |
|---|---|
| 0.10 M phosphate buffer, pH 7.80 | 100.0% |
| 10% PVP | 99.9% |
| 1.5% HPMC | 95.7% |
| 1.0% HPMC | 96.8% |
| 0.5% HPMC | 99.1% |
| 0.1% HPMC | 99.6% |

Hydrogel Surface Spread Test Categories: 1) No spreading, tight drops that stay in place; 2) Mild spreading, drops drip slowly down; 3) Severe spreading, drops completely wet surface. Water is in category 3.

Methylcellulose (MC) was found to behave similarly to hypromellose (HPMC) and provided workable viscous solutions in the concentration range of 0 to 2% (w/w). However, the HPMC dissolved more readily than the MC, and the HPMC solutions possessed greater optical clarity; thus the use of HPMC was favored. Povidone (PVP) dissolved easily

in the buffer, but provided minimal viscosity enhancement even at 20% (w/w). Higher molecular weight grades of PVP are available, but have not yet been explored.

For the most part, the polymers remain unchanged by the addition of low concentrations of HPMC or PVP. However, there was a noticeable change in the polymer around 0.3% HPMC that was characterized by an enhanced elasticity, as evidenced by the ability of the material to elongate more than usual without breakage. Above 1.5% HPMC, the polymer became slightly softer and exhibited less bounce. The gel times also remained within 10 seconds of the gel time for the formulation with no viscous agent. In the case of PVP, significant changes in the polymer occurred above 10% PVP. The polymer became more opaque with a noticeable increase in elasticity and stickiness. At 15% to 20% PVP, the polymer became similar to the sticky materials, but with a better mechanical strength. The gel times also increased by roughly 20 seconds relative to the formulation with no viscous agent. Thus, the addition of lower concentrations of PVP or HPMC to the polymer solutions may be beneficial in improving the polymer's elasticity and lubricity.

The results of the biocompatible hydrogel surface spread test show that most formulations belong in category 2.

Based on these observations, a formulation utilizing 0.3% HPMC was chosen for further evaluation. Above 1.0% HPMC, the solutions became significantly more difficult to mix and dissolution of the monomers became an issue. At 0.5% HPMC and above, the formation of air bubbles during mixing became significant. Furthermore, the solutions were not easily filtered through a 0.5 μm syringe filter to remove the bubbles. However, the 0.3% HPMC solution was easily filtered even after moderate mixing, resulting in a bubble-free, optically clear polymer.

Viscosity Measurements

The viscosities of the resulting buffer solutions were measured with the appropriately sized Cannon-Fenske viscometer tube from Ace Glass. Viscometer sizes used ranged from 25 to 300. Measurements of select solutions were performed in triplicate at both 20°C and 37°C. The results are shown in *Table 9B.* To calculate the approximate dynamic viscosities, it was assumed that all the buffer solutions had the same density as water.

To characterize the rheology of the polymers during the gelation process, a size 300 viscometer was used with a formulation that was designed to gel after approximately 15 minutes. The formulation used involved the 8ARM-20k-NH2 with the 4ARM-20k-SGA ester at 2.5% solution and 0.3% HPMC. The reaction occurred in a 0.05 M phosphate buffer at a pH of 7.2. Thus, one viscosity measurement with the size 300 viscometer was obtained in about one minute and subsequent measurements may be obtained in quick succession up to the gel point.

Hydrogel Surface Spread Test

To model the performance of the polymer solutions on a hydrophilic surface the extent of spreading and dripping of droplets on a high water content biocompatible hydrogel polymer matrix surface at an incline of about 30° was recorded. The biocompatible hydrogel polymer matrix was made by dissolving 0.10 g (0.04 mol arm eq.) of 8ARM-20k-NH2in 7 mL 0.05 M phosphate buffer at pH 7.4 in a Petri-dish, followed by the addition of 0.075 g (0.04 mol arm eq.) of 8ARM-15k-SG ester. The solution was stirred with a spatula for 10 to 20 seconds and allowed to gel, which typically took 5 to 10 minutes. The water content of the resulting polymer was 97.5%.

The test was performed by first preparing the polymer solution in the usual fashion. After thorough mixing, the polymer solution was dispensed dropwise through a 22 gauge needle onto the biocompatible biocompatible hydrogel polymer matrix surface. The results are shown in *Table 9B* and were divided into three general categories: 1) no spreading, tight drops that stay in place; 2) mild spreading, drops drip slowly down; 3) severe spreading, drops completely wet surface. Water is in category 3.

Swelling & Drying Measurements

The extent of swelling in the polymers during the degradation process was quantified as the liquid uptake of the polymers. A known mass of the polymer was placed in PBS at 37°C. At specified time intervals, the polymer was isolated from the buffer solution, patted dry with paper towels and weighed. The percent increase in the mass was calculated from the initial mass.

The fate of the polymers in air under ambient conditions was quantified as the weight loss over time. A polymer film of about 1 cm thickness was placed on a surface at 20°C. Mass measurements were performed at set intervals. The percent weight loss was calculated from the initial mass value.

The percent of water uptake by the 8ARM-20k-NH2/4ARM-20k-SGA polymers with 0, 0.3 and 1.0% HPMC was investigated. The 1.0% HPMC polymer absorbed up to 30% of its weight in water until day 20. After day 20, the polymer returned to about 10% of its weight in water. In comparison, the 0% HPMC polymer initially absorbed up to 10% of its weight in water, but began to lose water gradually, hovering about 5% of its weight in water. The 0.3% HPMC polymer behaved in an intermediate fashion. It initially absorbed up to 20% of its weight in water, but returned to about

10% of its weight in water after a week and continued to slowly lose water.

The percent of weight loss under ambient conditions over 24 hours by the 8ARM-20k-AA/8ARM-20k-NH2 (75/25) & 4ARM-20k-SGA polymer with 0.3% HPMC and 1.0% HPMC is shown in **Figure** 3. Ambient conditions were roughly 20°C and 30 to 50% relative humidity. The rate of water loss was fairly constant over 6 hours at about 10% per hour. After 6 hours, the rate slowed significantly as the polymer weight approached a constant value. The rate of water loss is expected to vary based on the polymer shape and thickness, as well as the temperature and humidity.

Specific Gravity Measurements

The specific gravity of the polymers was obtained by preparing the polymer solution in the usual fashion and pipetting 1.00 mL of the thoroughly mixed solution onto an analytical balance. The measurements were performed in triplicate at 20°C. The specific gravity was calculated by using the density of water at 4°C as the reference.

The specific gravity of the polymers did not differ significantly from that of the buffer solution only, both of which were essentially the same as the specific gravity of water. Exceptions may occur when the polymer solution is not filtered and air bubbles become embedded in the polymer matrix.

Barium Sulfate Suspensions

For imaging purposes, barium sulfate was added to several polymer formulations as a radiocontrast agent. Barium sulfate concentrations of 1.0, 2.0, 5.0 and 10.0% (w/v) were explored. The viscosity of the resulting polymer solutions was measured and the effect of barium sulfate addition on the polymer gel times and syringability characteristics were also studied.

Barium sulfate concentrations of 1.0, 2.0, 5.0 and 10.0% (w/v) were explored. The opaque, milky white suspensions formed similarly opaque and white polymers. No changes in the gel times were observed. Qualitatively, the polymers appeared to have similar properties to that of polymers without barium sulfate. All formulations were able to be readily dispensed through a 22 gauge needle.

The viscosity measurements for barium sulfate concentrations of 1.0, 2.0, 5.0 and 10.0% was measured. The viscosity remained relatively stable up to 2.0%; at 5.0%, the viscosity increased slightly to about 2.5 cP. There was a sharp increase in the viscosity to nearly 10 cP as the concentration approached 10.0%. Thus, a barium sulfate concentration of 5.0% was chosen as a balance between high contrast strength and similarity to unmodified polymer formulations.

Biocompatible Hydrogel Firmness, Elastic Modulus, and Adhesion

The firmness of the polymers was characterized by a Texture Analyzer model TA.XT.plus with Exponent software version 6.0.6.0. The method followed the industry standard "Bloom Test" for measuring the firmness of gelatins. In this test, the TA-8 ¼" ball probe was used to penetrate the polymer sample to a defined depth and then return out of the sample to the original position. The peak force measured is defined as the "firmness" of the sample. For the polymers studied, a test speed of 0.50 mm/sec, a penetration depth of 4 mm, and a trigger force of 5.0 g were used. The polymers were prepared on a 2.5 mL scale directly in a 5 mL size vial to ensure consistent sample dimensions. The vials used were ThermoScientific/Nalgene LDPE sample vials, product# 6250-0005 (LOT# 7163281060). Measurements were conducted at 20°C. The polymers were allowed to rest at room temperature for approximately 1 hour before measuring. Measurements were performed in triplicate for at least three samples. A sample plot generated by the Exponent software running the firmness test is given in **Figure** 4. The peak of the plot represents the point at which the target penetration depth of 4 mm was reached.

The elastic modulus of the polymers was characterized by a Texture Analyzer model TA.XT.plus with Exponent software version 6.0.6.0. In this test, the TA-19 Kobe probe was used to compress a polymer cylinder of known dimensions until fracture of the polymer occurs. The probe has a defined surface area of 1 $cm^2$. The modulus was calculated as the initial slope up to 10% of the maximum compression stress. For the polymers studied, a test speed of 5.0 mm/min and a trigger force of 5.0 g were used. The sample height was auto-detected by the probe. The polymers were prepared on a 2.5 mL scale directly in a 5 mL size vial cap to ensure consistent sample dimensions. The vials used were Thermo-Scientific/Nalgene LDPE sample vials, product# 6250-0005 (LOT# 7163281060). Measurements were conducted at 20°C. The polymers were allowed to rest at room temperature for approximately 1 hour before measuring. Measurements were performed for at least three samples. A sample plot generated by the Exponent software running the modulus test is given in **Figure** 5. The polymers typically behaved elastically for the initial compression, as evidenced by the nearly linear plot.

The adhesive properties of the polymers were characterized by a Texture Analyzer model TA.XT.plus with Exponent software version 6.0.6.0. In the adhesive test, the TA-57R 7 mm diameter punch probe was used to contact the polymer sample with a defined force for a certain amount of time, and then return out of the sample to the original

position. An exemplary plot generated by the Exponent software running the adhesive test is given in **Figure** 6. The plot begins when the probe hits the surface of the polymer. The target force is applied on the sample for a defined unit of time, represented by the constant force region in the plot. Then, the probe returns out of the sample to the original position and the adhesive force between the probe and the sample is measured as the "tack", which is the peak force required to remove the probe from the sample. Other properties that were measured include the adhesion energy or the work of adhesion, and the material's "stringiness." The adhesion energy is simply the area under the curve representing the tack force. Thus, a sample with a high tack and low adhesion energy will qualitatively feel very sticky, but may be cleanly removed with a quick pull; a sample with a high tack and high adhesion energy will also feel very sticky, but the removal of the material will be more difficult and may be accompanied by stretching of the polymer, fibril formation and adhesive residues. The elasticity of the polymer is proportional to the measured "stringiness", which is the distance the polymer stretches while adhered to the probe before failure of the adhesive bond. For the polymers studied, a test speed of 0.50 mm/sec, a trigger force of 2.0 g, and a contact force of 100.0 g and contact time of 10.0 sec were used. The polymers were prepared on a 1.0 to 2.5 mL scale directly in a 5 mL size vial to ensure consistent sample surfaces. The vials used were Thermo Scientific/Nalgene LDPE sample vials. Measurements were conducted at 20°C. The polymers were allowed to rest at room temperature for approximately 1 hour before measuring. As reference materials, the adhesive properties of a standard Post-It Note® and Scotch Tape® were measured. All measurements were performed in triplicate. The averages and standard deviations were calculated.

The effect of HPMC addition to the mechanical properties of the polymers was explored, along with the effect of adding degradable 8ARM-20k-AA amine. Under the stated conditions of the firmness test, it was found that the addition of 0.3% HPMC decreased the firmness of the polymer by about half. This corresponds to a slight decrease in the elastic modulus. The 1.0% HPMC polymer had approximately the same firmness as the 0.3% HPMC polymer, but a slight decrease in the elastic modulus. The disparity between the firmness and modulus tests is likely due to experimental error. The polymer solutions were not filtered, so the presence of air bubbles likely increased the errors. The water content of the polymers may also change as the polymers were sitting in the air, essentially changing the physical properties of the materials.

It was found that the addition of the degradable 8ARM-20k-AA amine did not substantially change the measured values of the firmness or the elastic modulus. The measured values for a standard commercial Post-It™ Note are also included as a reference. The polymer tack was found to be around 40 mN, which is about three times less than that of a Post-It™ Note. The adhesive properties of the polymer were not found to vary with the addition of the degradable amine.

**Figure** 7 shows the firmness vs. degradation time for the 8ARM-20k-AA/8ARM-20k-NH2 (70/30) & 4ARM-20k-SGA at 4.8% solution with 0.3% HPMC. The error bars represent the standard deviations of 3 samples. The degradation time for the polymer was 18 days. The firmness of the polymer strongly correlated with the extent of degradation. Swelling may also play a role during the early stages.

The effect of various additives to the formulation on the polymer properties was explored. Gel gel time, degradation time, firmness, adhesion and elastic modulus was measured for polymers prepared with varying combinations of 1% HPMC, 2% chlorhexidine and 1% denatonium benzoate. Essentially no change in the polymer properties were found except for formulations containing 2% chlorhexidine, which exhibited decreased firmness and elastic modulus. It was apparent from visual inspection of the polymer that the change was due to the detergent present in the Nolvasan solution used and not the chlorhexidine; the detergent caused heavy foaming during mixing that gelled into an aerated polymer.

Optical Clarity

A Thermo Scientific GENESYS 10S UV-Vis spectrophotometer was used to measure the optical clarity of the viscous solutions. To a quartz cuvette, 1.5 mL of the sample solution was pipetted. The buffer solution with no additives was used as the reference. The stable % transmission of the sample was recorded at 650 nm.

To measure the light transmission of the polymers, 1 mL of polymer solution was filtered with a 5 μm filter into a cuvette before gelation. The cuvette was then placed horizontally so that the polymer gelled on the side of the cuvette as a film. The film thickness was found to be 3 mm. The polymer was allowed to cure for 15 minutes at room temperature before measuring the % light transmission at 400, 525 and 650 nm with air as the reference.

All of the viscous solutions under consideration were found to have acceptable to excellent optical clarity under the concentration ranges used (greater than 97% transmission). For the highly viscous solutions, air bubble formation during mixing was observed, which may be resolved by the addition of an anti-foaming agent, or through the use of a syringe filter (See *Table 9C).*

The polymers exhibited excellent optical clarities over the visible spectrum. The lowest % transmission relative to buffer only was 97.2% and the highest was 99.7%. The drop in the % transmission at lower wavelengths is likely due to some energy absorption as the ultraviolet region is approached.

Drug Elution: General Procedures

A Thermo Scientific GENESYS 10S UV-Vis spectrophotometer was used to quantify the release of various drugs from several polymers. First, the reference drug or drug solution was dissolved in an appropriate solvent. Typically, phosphate buffered saline (PBS), ethanol or dimethylsulfoxide (DMSO) were used as the solvent. Next, the optimal absorption peak for identifying and quantifying the drug was determined by performing a scan of the drug solution between 200 and 1000 nm. With the absorption peak selected, a reference curve was established by measuring the peak absorbance for various concentrations of the drug. The different drug concentration solutions were prepared by standard dilution techniques using analytical pipettes. A linear fit of the absorbance vs. drug concentration resulted in a general equation that was used to convert the measured absorbance of the elution samples to the drug concentration.

The polymer was prepared with a known drug dosage in the same fashion as a doctor administering the polymer in a clinical setting. However, in this case the polymer was molded into a cylinder with a diameter of approximately 18 mm. The polymer cylinder was then placed in a 50 mL Falcon tube with a set amount of PBS and placed at 37°C. The temperature was maintained by a digitally controlled water bath.

Elution samples were collected daily by decanting the PBS solution from the polymer. The volume of sample collected was recorded. The polymer was placed in a volume of fresh PBS equivalent to the volume of sample that was collected and returned to 37°C. The elution sample was analyzed by first diluting the sample in the appropriate solvent using analytical pipettes such that the measured absorbance was in the range determined by the reference curve. The dilution factor was recorded. The drug concentration was calculated from the measured absorbance via the reference curve and the dilution factor. The drug amount was calculated by multiplying the drug concentration with the sample volume. The percent elution for that day was calculated by dividing the drug amount by the total amount of drug administered.

Drug Elution: Chlorhexidine

The peak found between 255 and 260 nm was chosen and a reference curve was established by measuring the peak absorbance for 0,0.5, 1,2.5,5, 10, 20, 40, and 50 ppm of chlorhexidine. Concentrations above 50 ppm did not exhibit linear behavior in peak absorbance.

The polymer was prepared with a commercial Nolvasan solution, which corresponds to a 2% chlorhexidine dose (50 mg). The elution volume was 2 mL of PBS per 1 g of polymer. The elution samples were stored at 20°C. The elution samples were analyzed by diluting the sample 1,000-fold with dimethyl sulfoxide (DMSO) in a quartz cuvette.

The chlorhexidine elution behavior proceeded similarly to previous experiments with other small molecules. Almost half of the chlorhexidine was released within the first three days. Then, the elution rate slowed dramatically for the next three to four days followed by another large release of chlorhexidine as the polymer degrades **(Figure 8**).

The elution of the steroidal drugs, triamcinolone and methylprednisolone, behaved similarly. The first few days typically exhibit an elevated elution rate, presumably as weakly bound surface drug is released. Then, the elution is relatively constant at a rate that is related to the drug solubility. Finally, the remaining drug in the polymer is released as degradation begins. Several examples are given in **Figure 9, Figure 10,** and **Figure 11** of the control over the elution behavior that was developed. Drugs may be released over a short time (weeks) or long period (years, projected).

**Example 12: General Procedure for the Preparation of Polymerizable Biocompatible Pre-Formulations**

Several representative formulations for both sticky and non-sticky films are listed in *Table 10* along with specific reaction details. The films had thicknesses ranging from 100 to 500 μm, and may be layered with different formulations in a composite film.

*Table 10. (A) Summary of the reaction details for several representative thin film formulations; (B) more detailed tabulation of a selection of the reaction details including moles (films ranged in thickness from 100 to 500 μm).*

| **(A)** | | | |
|---|---|---|---|
| **Pre-formulation Components** | **Amine/Ester Molar Ratio** | **Buffer** | **% Solution** |
| 4ARM-20k-AA & 8ARM-15k-SG | 1 | 0.15 M phosphate, pH 7.99 | 19.6 |
| 4ARM-5k-NH2 & 4ARM-10k-SG | 4.5/1 | 0.05 M phosphate, pH 7.40 | 39 |

(continued)

| (A) | | | |
|---|---|---|---|
| **Pre-formulation Components** | **Amine/Ester Molar Ratio** | **Buffer** | **% Solution** |
| 4ARM-5k-NH2 & 4ARM-10k-SG | 1 | 0.05 M phosphate, pH 7.40 | 36.4 |
| **Pre-formulation Components** | **Amine/Ester Molar Ratio** | **Buffer** | **% Solution** |
| 4ARM-5k-NH2 & 4ARM-10k-SG & HPMC (1.25%) | 4.5/1 | 0.10 M phosphate, pH 7.80 | 39 |
| 4ARM-2k-NH2 & 4ARM-10k-SG & HPMC (1.5 %) | 8/1 | 0.10 M phosphate, pH 7.80 | 30.6 |
| 4ARM-2k-NH2 & 4ARM-20k-SGA & MC (2%) | 8/1 | 0.15 M phosphate, pH 7.94 | 30 |
| 4ARM-2k-NH2 & 4ARM-20k-SGA & MC (2%) | 10/1 | 0.15 M phosphate, pH 7.94 | 30 |

| (B) | | | | | | | |
|---|---|---|---|---|---|---|---|
| Pre-formulation Components | MW | Mmoles | Wt (g) | Arm | mmoles | Arms Eq | Polymer % Solution (w/v) |
| 4ARM-20k-AA | 20000 | 1000 | 0.2 | 4 | 0.01 | 0.04 | |
| 8ARM-15k-SG | 15000 | 1000 | 0.075 | 8 | 0.01 | 0.04 | |
| Buffer Volume (phosphate) | | | 1.4 | | | | 19.6 |
| | | | | | | | |
| 4ARM-5k-NH2 | 5000 | 1000 | 0.27 | 4 | 0.05 | 0.22 | |
| 4ARM-10k-SG | 10000 | 1000 | 0.12 | 4 | 0.01 | 0.05 | |
| Buffer Volume (phosphate) | | | 1 | | | | 39.0 |
| | | | | | | | |
| 4ARM-5k-NH2 | 5000 | 1000 | 0.17 | 4 | 0.03 | 0.14 | |
| 4ARM-10k-SG | 10000 | 1000 | 0.34 | 4 | 0.03 | 0.14 | |
| Buffer Volume (phosphate) | | | 1.4 | | | | 36.4 |
| | | | | | | | |
| 4ARM-5k-NH2 | 5000 | 1000 | 0.27 | 4 | 0.05 | 0.22 | |
| 4ARM-10k-SG | 10000 | 1000 | 0.12 | 4 | 0.01 | 0.05 | |
| Buffer Volume (phosphate) | | | 1 | | | | 39.0 |
| Viscosity Enhancer | | 1.25% HPMC | | | | | |

**Example** 13: **Preparation of Kits and Their Use**

Several kits were prepared with the polymer formulation tested earlier. The materials used to assemble the kits are listed in *Table 11* and the formulations used are listed in *Table 12.* The kits are typically composed of two syringes, one syringe containing the solid components and the other syringe containing the liquid buffer. The syringes are connected

via a mixing tube and a one-way valve. The contents of the syringes are mixed via opening the valve and transferring the contents of one syringe into the other, repeatedly, for 10 to 20 seconds. The spent syringe and mixing tube are then removed and discarded, and the active syringe is fitted with a dispensing unit, such as a needle or cannula, and the polymer solution is expelled until the onset of gelation. In other instances, the viscous solution impedes the dissolution of the solid components and thus a third syringe is employed. The third syringe contains a concentrated viscous buffer that enhances the viscosity of the solution once all the components have dissolved. In some instances, the optical clarity of the resulting polymer is improved through the addition of a syringe filter.

All of the formulations tested were easily dispensed through a 22 gauge needle. The mixing action between the two syringes was turbulent and the introduction of a significant amount of air bubbles was apparent. Gentle mixing results in a clear material free of bubbles. Alternatively, the use of a syringe filter was found to remove bubbles without any change in the polymer properties.

*Table 11. Materials used to fabricate kits including vendor, part number and lot number.*

| Description | Vendor |
|---|---|
| Vincon Tubing, 1/8" I.D. 1/4" O.D. 1/16" wall, 100 Ft. | Ryan Herco Flow Solutions |
| 12 mL Luer-Lock Syringe | Tyco Healthcare, Kendall Monoject™ |
| 3 mL Luer-Lock Syringe | Tyco Healthcare, Kendall Monoject™ |
| One Way Stopcock, Female Luer Lock to Male Luer | QOSINA |
| Female Luer Lock Barb for 1/8" I.D. tubing, RSPC | QOSINA |
| Non-vented Luer Dispensor Tip Cap, White | QOSINA |
| 32 mm Hydrophilic Syringe Filter, 5 micron | PALL® Life Sciences |

*Table 12. The detailed contents for four different kits; the solid components are in one syringe, while the liquid components are in another syringe; a mixing tube connects the two syringes.*

| Pre-formulation Components | MW | wt (g) | Arm | mmoles | Arms Eq | % Solution |
|---|---|---|---|---|---|---|
| 8ARM-20k-NH2 | 20000 | 0.04 | 8 | 0.002 | 0.016 | |
| 4ARM-20k-SGA | 20000 | 0.08 | 4 | 0.004 | 0.016 | |
| Phosphate buffer | | 2.5 mL 0.10 M, pH 7.80 | | | | 4.8 |
| Viscosity Enhancer | | No viscosity enhancer | | | | |
| | | | | | | |
| 8ARM-20k-NH2 | 20000 | 0.04 | 8 | 0.002 | 0.016 | |
| 4ARM-20k-SGA | 20000 | 0.08 | 4 | 0.004 | 0.016 | |
| Phosphate buffer | | 2.5 mL 0.10 M, pH 7.80 | | | | 4.8 |
| Viscosity Enhancer | | 0.3% HPMC | | | | |
| 8ARM-20k-NH2 | 20000 | 0.04 | 8 | 0.002 | 0.016 | |
| 4ARM-20k-SGA | 20000 | 0.08 | 4 | 0.004 | 0.016 | |
| Phosphate buffer | | 2.5 mL 0.10 M, pH 7.80 | | | | 4.8 |
| Viscosity Enhancer | | 7.5% Povidone | | | | |
| | | | | | | |
| 8ARM-20k-NH2 | 20000 | 0.04 | 8 | 0.002 | 0.016 | |
| 4ARM-20k-SGA | 20000 | 0.08 | 4 | 0.004 | 0.016 | |
| Phosphate buffer | | 2.5 mL 0.10 M, pH 7.80 | | | | 4.8 |
| Viscosity Enhancer | | 1.0% HPMC | | | | |

Several additional kits were prepared with the polymer formulation that performed the best in initial trials. The materials used to assemble the kits are listed in *Table 13.* The kits are typically composed of two syringes, one syringe containing the solid components and the other syringe containing the liquid buffer. The syringes were loaded by removing the plungers, adding the components, purging the syringe with a gentle flow of nitrogen gas for 20 seconds, and then replacing the plunger. Finally, the plungers were depressed as much as possible to reduce the internal volume of the syringes. The specifications for the amounts of chemical components in the kits are listed in *Table 14A.* A summary describing the lots of kits prepared is listed in *Table 14B.*

The syringes were connected directly after uncapping, the male part locking into the female part. The contents of the syringes were mixed via transferring the contents of one syringe into the other, repeatedly, for 10 to 20 seconds. The spent syringe was then removed and discarded, and the active syringe was fitted with a dispensing unit, such as a needle or cannula, and the polymer solution was expelled until the onset of gelation. In other instances, the viscous solution impeded the dissolution of the solid components and thus a third syringe was employed. The third syringe contained a concentrated viscous buffer that enhanced the viscosity of the solution once all the components had dissolved.

All the formulations tested were easily dispensed through a 22 gauge needle. The mixing action between the two syringes was turbulent and the introduction of a significant amount of air bubbles was apparent. The use of a syringe filter was found to remove bubbles without any change in the polymer properties.

The prepared kits were placed into foil pouches along with one oxygen absorbing packet per pouch. The pouches were heat sealed with a CHTC-280 PROMAX tabletop chamber sealing unit. Two different modes of sealing were explored: under nitrogen and under vacuum. The settings for sealing under nitrogen were: 30 seconds of vacuum, 20 seconds of nitrogen, 1.5 seconds of heat sealing, and 3.0 seconds of cooling. The settings for sealing under vacuum were: 60 seconds of vacuum, 0 seconds of nitrogen, 1.5 seconds of heat sealing, and 3.0 seconds of cooling.

*Table 13. Materials used to fabricate kits including vendor, part number and lot number.*

| Description | Vendor |
|---|---|
| 12 mL Male Luer-Lock Syringe | Tyco Healthcare, Kendall Monoject™ |
| 5 mL Female Luer Lock Syringe, Purple | QOSINA |
| Male Luer Lock Cap, Non-vented | QOSINA |
| Female Non-vented Luer Dispensor Tip Cap, White | QOSINA |
| 100cc oxygen absorbing packet | IMPAK |
| 6.25" x 9" OD PAKVF4 Mylar foil pouch | IMPAK |

*Table 14. Specifications for kit components for the 8ARM-20k-AA/8ARM-20-NH2* & *4ARM-20k-SGA formulation with 60, 65, 70 and 75% degradable amine (A). LOT formulation summary (B).*

| **(A)** | | | | |
|---|---|---|---|---|
| | **Specifications** | | | |
| **Pre-formulation Components** | **60/40** | **65/35** | **70/30** | **75/25** |
| 8ARM-20k-AA | 0.024 - 0.026 g | 0.026 - 0.027 g | 0.028 - 0.029 g | 0.030 - 0.031 g |
| 8ARM-20k-NH2 | 0.014 - 0.016 g | 0.013 - 0.014 g | 0.011 - 0.012 g | 0.009 - 0.010 g |
| 4ARM-20k-SGA | 0.080 - 0.082 g | 0.080 - 0.082 g | 0.080 - 0.082 g | 0.080 - 0.082 g |
| Phosphate Buffer | 2.50 mL of 0.10 M phosphate, pH 7.58, 0.30% HPMC (8.48 cSt +/-0.06 @ 20°C) | | | |
| **(B)** | | | | |
| **Formulation** | **Buffer pH** | **Sealing Method** | **Notes** | |
| 60/40 | 7.46 | nitrogen | | |
| 60/40 | 7.58 | nitrogen | | |
| 60/40 | 7.72 | nitrogen | | |
| 70/30 | 7.58 | vacuum | | |

(continued)

| (B) | | | |
|---|---|---|---|
| Formulation | Buffer pH | Sealing Method | Notes |
| 70/30 | 7.58 | vacuum | no nitrogen purging of syringe |
| 65/35 | 7.58 | vacuum | |
| 75/25 | 7.58 | vacuum | |
| 75/25 | 7.58 | vacuum | |
| 75/25 | 7.58 | nitrogen | |
| 65/35 | 7.58 | vacuum | |
| Formulation | Buffer pH | Sealing Method | Notes |
| 65/35 | 7.58 | nitrogen | |

Several kits were prepared for use in beta testing. The materials used to assemble the kits are listed in *Table 15.* The kits are typically composed of two syringes, one syringe containing the solid components and the other syringe containing the liquid buffer. The syringes were loaded by removing the plungers, adding the components, purging the syringe with a gentle flow of inert gas for 10 seconds, and then replacing the plunger. Finally, the plungers were depressed as much as possible to reduce the internal volume of the syringes.

Alternatively, a single syringe kit may be prepared by loading the solid components into one female syringe along with a solid form of the phosphate buffer. The kit is then utilized in a similar fashion as the dual syringe kit, except the user may use a specified amount of a variety of liquids in a male syringe. Typically, any substance provided in a liquid solution for injection may be used. Some examples of suitable liquids are water, saline, Kenalog-10, Depo-Medrol and Nolvasan.

The kits are utilized in the following fashion. The syringes are connected directly after uncapping, the male part locking into the female part. The contents of the syringes are mixed via transferring the contents of one syringe into the other, repeatedly, for 10 to 20 seconds. The spent syringe is then removed and discarded, and the active syringe is fitted with a dispensing unit, such as a needle, a spray nozzle or a brush tip, and the polymer solution is expelled until the onset of gelation.

The prepared kits were placed into foil pouches along with one oxygen absorbing packet and one indicating silica gel packet per pouch. Labels were affixed to the pouches that displayed the product and company name, contact information, LOT and batch numbers, expiration date, and recommended storage conditions. A radiation sterilization indicator that changes color from yellow to red upon exposure to sterilizing radiation was also affixed to the upper left corner of the pouch. The pouches were heat sealed with a CHTC-280 PROMAX tabletop chamber sealing unit. The settings for sealing under vacuum were: 50 seconds of vacuum, 1.5 seconds of heat sealing, and 5.0 seconds of cooling.

An example detailing the lots of sterile kits prepared is listed in *Table 15.* A previous study found that if the loaded syringe was not purged with nitrogen before replacing the plunger during kit preparation, the sterile kits exhibited an increase in gel time of about 30 seconds relative to kits that had syringes flushed with nitrogen. No significant difference was found between kits that had been sealed under vacuum and kits that had been sealed under nitrogen. It was easily observable when the vacuum-sealed kits lost their seal, so it was decided to vacuumseal all kits as standard procedure. The effects of including the oxygen absorbing packet and silica gel packet to the kits on the long term storage stability is currently under investigation.

*Table 15. Materials used to fabricate kits including vendor, and part number.*

| Description | Vendor | Part # |
|---|---|---|
| 10 mL Luer-Lok Syringe | BD | 309604 |
| Non-Vented Luer Dispenser Tip Cap, White | QOSINA | 65119 |
| 5 mL Female Luer-Lock Syringe, Purple PP | QOSINA | C3610 |
| Male Luer Lock Cap, Non-Vented, PP | QOSINA | 11166 |
| Brush tip | Flumatic | BT01225R |
| 5.25"x8" PAKVF4D Mylar foil pouch | IMPAK | 0525MFDFZ08TE |

(continued)

| Description | Vendor | Part # |
|---|---|---|
| 3.5"x6.5" PAKVF4W Mylar foil pouch | IMPAK | 035MFW065Z |
| Radiation Sterilization Indicator | QOSINA | 13124 |
| 100cc oxygen absorbing packet | IMPAK | OAP100 |
| Indicating silica gel | IMPAK | 40ISG37 |

*Table 16. Example specifications for kit components for the 8-arm-AA-20K/8-arm-NH2-20K & 4-arm-SGA-20K formulation with 75% degradable amine (A). LOT formulation summary (B).*

| (A) | | | | |
|---|---|---|---|---|
| **Components** | **LOT# & Specifications** | | | |
| 8ARM-20k-AA | 0.029 - 0.031 g | | | |
| 8ARM-20k-NH2 | 0.009 - 0.011 g | | | |
| 4ARM-20k-SGA | 0.079 - 0.081 g | | | |
| Phosphate Buffer | 2.50 mL of 0.10 M phosphate, pH 7.58, 0.30% HPMC (8.48 cSt +/- 0.06 @ 20°C) | | | |
| **LOT Size** | 3 | 30 | 34 | 48 |
| **Gel Time (s)** | 110 - 125 | | | |
| **Degradation Time (days)** | 10 - 12 | | | |

| (B) | |
|---|---|
| **Components** | **LOT# & Specifications** |
| 8ARM-20k-AA | 0.029 - 0.031 g |
| 8ARM-20k-NH2 | 0.009 - 0.011 g |
| 4ARM-20k-SGA | 0.079 - 0.081 g |
| Phosphate Buffer Powder | 0.03 - 0.06 g |
| Nolvasan (2% chlorhexidine) | 2.50 mL, 1% denatonium benzoate |
| **LOT Size** | 64 |
| **Gel Time (s)** | 150 |
| **Degradation Time (days)** | 11 |

The kit preparation time was recorded. Loading one buffer syringe took an average of 1.5 minutes, while one solids syringe took an average of 4 minutes. Vacuum sealing one kit took approximately 1.5 minutes. Thus, the time estimate for the preparation of one kit was 7 minutes, or approximately 8 kits per hour. The kit preparation time may be improved by premixing all the solids in the correct ratios such that only one mass of solids needs to be measured, and by optimizing the vacuum sealing procedure by reducing the vacuum cycle time.

All the formulations tested were easily dispensed through a 23 to 34 gauge needle. Higher gauges exhibit a lower flow rate as expected. The mixing action between the two syringes was turbulent and the introduction of a significant amount of air bubbles was apparent. The use of a syringe filter was found to remove bubbles without any change in the polymer properties.

For the single syringe system, the effect of phosphate powder use was investigated. **Figure 12** shows the effect of varying amounts or concentrations of the solid phosphate on polymer gel times and solution pH. The system was found to be relatively insensitive to the amount of phosphate, tolerating up to 2-fold differences without significant variation.

Kit Sterilization & Testing

The sealed kits were packed into large sized FedEx boxes. Each box was sterilized via electron-beam radiation

at NUTEK Corporation according to a standard procedure that was developed. Included in this report is a copy of the standard sterilization procedure document.

For each lot of sterilized kits, a gel time and degradation time test was performed on a randomly selected kit to verify the viability of the materials. A previous study included a runner or control box of kits that was not sterilized, and concluded that environmental conditions during transit of the kits did not play a significant role in gel time changes.

Sterilized kits were sent to NAMSA for sterility verification according to USP<71>. The kits were verified as sterile.

No physical changes in the monomer and phosphate buffer solutions were observed post-sterilization. Prior experiments have shown that the polymer gel times consistently increase by approximately 30 seconds after sterilization. For example, a polymer with a 90 second gel time will exhibit a 120 second gel time after sterilization. The pH of the sterile buffer was unchanged, so it was suspected that some monomer degradation during sterilization occurred. This was confirmed by preparing unsterilized polymers at various concentrations and comparing the gel times, degradation times and mechanical properties with sterilized polymers **(Figure 13)**. The current data shows that the monomers experience roughly 15 to 20% degradation upon sterilization. Thus, a 5% polymer after sterilization will behave similarly to a 4% polymer. Additional experiments are planned to establish a detailed quality control calibration curve.

Storage Stability

The sterilized kits were stored at 5°C. Some kits were stored at 20°C or 37°C to explore the effect of temperature on storage stability. The stability of the kits was primarily quantified by recording changes in gel time, which is directly proportional to the extent of monomer degradation. The 37°C temperature was maintained by submerging the kits fully into the water bath and thus represents the worst case scenario regarding humidity.

The storage stability of the kits was explored by placing some kits at 5°C, 20°C or 37°C and measuring the change in gel times at defined intervals. The kits were prepared and sealed according to the procedures detailed in a previous section. The results are shown in **Figure 14.** Over 16 weeks, no significant change in gel times were observed for kits stored at 5°C and 20°C. At 37°C, the gel time begins to increase after roughly 1 week at a constant rate. The foil pouch proved to be an effective moisture barrier. The indicating silica gel packet exhibited only mild signs of moisture absorption as evidenced by the color. Longer term data is still in the process of being collected.

Example of Syringe Kit Preparation

One syringe kit was developed where the components are stored in two syringes, a male and a female syringe. The female syringe contains a mixture of white powders. The male syringe contains a buffer solution. The two syringes are connected and the contents mixed to produce a liquid polymer. The liquid polymer is then sprayed or applied over the suture wound where it covers the entire suture line. During the process, the polymer enters the voids left by sutures and protects the wound from infections. At the wound site, the liquid polymer turns into a solid gel and stays at the site for over two weeks. During this time, the wound is healed and infection free.

The components necessary to prepare the kit are disclosed in *Table* 17 and *Table 18*. To prepare the powder components of the kit to fill into the female syringe, the plunger of the 5 mL female Luer-lock syringe was removed, and the syringe was capped with the appropriate cap. 8ARM-20k-AA (0.028 g, the acceptable weight range is 0.0270 g to 0.0300 g), 8ARM-20k-NH2 (0.012 g, the acceptable weight range is 0.0100 g to 0.0130 g), 4ARM-20k-SGA (0.080 g, the acceptable weight range is 0.0790 g to 0.0820 g), and 0.043 g of freeze-dried phosphate buffer powder (0.043 g, the acceptable weight range is 0.035 g to 0.052 g) were each carefully weighed out and poured into the syringe. The syringe was then flushed nitrogen / argon gas for about 10 seconds at a rate of 5 to 10 L/min and the plunger was replaced to seal the contents. The syringe was then flipped so that the cap was facing towards the ceiling. The syringe cap was then loosened and the air space in the syringe was minimized by expelling as much air as possible from the syringe. Typical compressed powder volume is 0.2 mL. Then, the syringe cap was tighten until the cap was finger tight.

The liquid component was prepared on a 500 mL batch size, wherein 50 mL of commercial 2% chlohexidine solution, 450 mL of distilled water, and 1.5 g of HPMC were poured in to sterile container. The sterile container was then capped and shook vigorously for 10 seconds. The solution was allowed to stand under ambient conditions for 16 hours, thereby allowing for the foam to dissipate and any remaining HPMC to dissolve.

The liquid/buffer syringe was prepared by removing the plunger of the male Luer-lock syringe followed by capping the syringe with the appropriate cap. 2.50 mL of the buffer/liquid solution was transfered by pipette into the syringe. The syringe was then flushed with nitrogen/ argon gas for about 5 seconds at a rate of 5 to 10 L/min. The plunger of the syringe was then replaced to seal the contents. Then the syringe was flipped so that the cap was facing towards the ceiling and the syringe cap was loosen and air space was minimized by expelling as much air as possible from the syringe. Then the syringe cap was tightened until the cap was finger tight.

*Table 17. Components used to fabricate the solid components for the female syringe*

| Components | Technical Name |
|---|---|
| 8ARM-20k-AA | 8ARM PEG Acetate amine, HCl salt, MW 20k |
| 8ARM-20k-NH2 | 8ARM PEG amine (hexaglycerol), HCl salt, MW 20k |
| 4ARM-20k-SGA | 4-arm PEG succinimidyl glutaramide (pentaerythritol), MW 20k |
| | Commercial 2% chlorhexidine solution |
| | Freeze-dried phosphate buffer powder |

*Table 18. Materials used to fabricate kit including vendor, part number and lot number.*

| Description | Vendor | Part # | Vendor Catalog # |
|---|---|---|---|
| 10 mL Luer-Lok Syringe | BD | CM-0003 | 309604 |
| Non-Vented Luer Dispenser Tip Cap, White | QOSINA | CM-0004 | 65119 |
| 5 mL Female Luer-Lock Syringe, Purple PP | QOSINA | CM-0005 | C3610 |
| Male Luer Lock Cap, Non-Vented, PP | QOSINA | CM-0006 | 11166 |

Example of Syringe Kit Preparation

Another syringe kit was developed where the solid components, a mixture of white powders, are stored in one female syringe. A standard male syringe is used to take up the drug solution, such as one containing Kenalog. The two syringes are connected and the contents mixed to produce a liquid polymer. The liquid polymer is then delivered to the target site.

The components necessary to prepare the kit are disclosed in *Table 17* and *Table 18.* To prepare the powder components of the kit to fill into the female syringe, the plunger of the 5 mL female Luer-lock syringe was removed, and the syringe was capped with the appropriate cap. 8ARM-20k-AA (0.0125 g, the acceptable weight range is 0.012 g to 0.013 g), 8ARM-20k-NH2 (0.075 g, the acceptable weight range is 0.007 g to 0.008 g), 4ARM-20k-SGA (0.040 g, the acceptable weight range is 0.040 g to 0.042 g), and 0.018 g of freeze-dried phosphate buffer powder (0.043 g, the acceptable weight range is 0.017 g to 0.022 g) were each carefully weighed out and poured into the syringe. The syringe was then flushed nitrogen / argon gas for about 10 seconds at a rate of 5 to 10 L/min and the plunger was replaced to seal the contents. The syringe was then flipped so that the cap was facing towards the ceiling. The syringe cap was then loosened and the air space in the syringe was minimized by expelling as much air as possible from the syringe. Then, the syringe cap was tightened until the cap was finger tight.

**Example 14: General Procedure for the Preparation of a Polyglycol-based, Biocompatible Hydrogel Polymer Matrix**

A polyglycol-based, biocompatible pre-formulation is prepared by mixing 0.028 g of 8ARM-AA-20K, 0.012 g of 8ARM-NH2-20K, and 0.080 g of 4ARM-SGA-20K. 2.50 mL of culture medium is added to the formulation. The formulation is mixed for about 10 seconds and a 1 mL solution of the mixture is pipetted out using a mechanical high precision pipette. The polyglycol-based, biocompatible pre-formulation components polymerize to form a polyglycol-based, biocompatible hydrogel polymer matrix. The polymerization time of 1 mL liquid is collected and then verified with the lack of flow for the remaining liquids.

**Example 15: General Procedure for the Preparation of a Polyglycol-based, Biocompatible Hydrogel Polymer Matrix and Stem Cells**

A polyglycol-based, biocompatible pre-formulation is prepared by mixing 0.0125 g of 8ARM-AA-20K, 0.0075 g of 8ARM-NH2-20K, and 0.040 g of 4ARM-SGA-20K. 1.0 mL of culture medium is added to the formulation. The formulation is mixed for about 10 seconds and a 1 mL solution of the mixture is pipetted out using a mechanical high precision pipette. The polyglycol-based, biocompatible pre-formulation components polymerize to form a polyglycol-based, biocompatible hydrogel polymer matrix. The polymerization time of 1 mL liquid is collected and then verified with

the lack of flow for the remaining liquids. Various sized slices of the polymerized polyglycol-based, biocompatible hydrogel polymer matrix are placed in different wells of a 24 well plate. 0.5 mL of adult mesenchymal stem cells are seeded onto the polymer matrices at various densities. The stem cells diffuse and become incorporated into the polyglycol-based, biocompatible hydrogel polymer matrix. Incorporation of the stem cells into the polyglycol-based, biocompatible hydrogel polymer matrix is demonstrated by removing a slice of the polymer matrix 10 days after stem cell addition and using the slice to expand the cells in culture. The incorporated stem cells remain viable, as demonstrated by their ability to proliferate in culture.

**Example 16: General Procedure for the Preparation of a Polyglycol-based, Biocompatible Hydrogel Polymer Matrix and Stem Cells**

A polyglycol-based, biocompatible pre-formulation is prepared by mixing 0.0125 g of 8ARM-AA-20K, 0.0075 g of 8ARM-NH2-20K, and 0.040 g of 4ARM-SGA-20K. 1.0 mL of culture medium containing adult mesenchymal stem cells is added to the formulation. The formulation is mixed for about 10 seconds and a 1 mL solution of the mixture is pipetted out using a mechanical high precision pipette. The polyglycol-based, biocompatible pre-formulation components polymerize to form a polyglycol-based, biocompatible hydrogel polymer matrix. The polymerization time of 1 mL liquid is collected and then verified with the lack of flow for the remaining liquids.

At any point during the combination of the polyglycol-based, biocompatible pre-formulation compounds, additional components may be added to the formulation. The formulation may be solid, liquid, polymerized, gelled, or any combination thereof when the additional component is added. The additional component may combine with or diffuse through the formulation and become retained with the formulation for a determined period of time. In one example, the polyglycol-based, biocompatible hydrogel polymer matrix is formed, followed by the addition of growth factors. The growth factors are incorporated into the polyglycol-based, biocompatible hydrogel polymer matrix. Additional components include, but are not limited to, biomolecules, antibiotics, anti-cancers, anesthetics, anti-virals, or immunosuppressive agents.

**Example 17: Viability of Cells in a Polyglycol-based, Biocompatible Hydrogel Polymer Matrix**

A single cell suspension of mesenchymal stem cells in D15 (DMEM, high glucose, 15% fetal bovine serum) is prepared and the cells counted. 1 mL of cells at a 2 x $10^4$ /mL density are added to a 50 mL tube. The cells are maintained at room temperature and prepared just before addition to a pre-formulation. A female syringe containing a polyglycol-based, biocompatible pre-formulation is prepared by mixing 0.0125 g of 8ARM-AA-20K, 0.0075 g of 8ARM-NH2-20K, and 0.040 g of 4ARM-SGA-20K in the female syringe. An 18G need is attached to a male syringe and the male syringe is filled with 1 mL PBS. The next step is carried out within 90-120 seconds. The needle is removed from the male syringe and the male syringe is attached to the female syringe containing the pre-formulation. The PBS is pushed from the male syringe into the female syringe and the mixing process is started by repeatedly pushing the PBS from one syringe to the other, with 20 strokes being sufficient for mixing. After the final stroke, the entire contents are pushed into the male syringe. An 18G needle is attached to the male syringe and the liquid pre-formulation is ejected into the 50 mL tube containing the 1 mL of mesenchymal stem cells. The cells are carefully mixed while the liquid pre-formulation is being ejected into the tube. Care is made to ensure that the cells are not mixed by aspiration with the needle as this may induce cell stress.

Aliquots of the pre-formulation containing mesenchymal stem cells are placed in chambers of a 4-chamber tissue culture glass slide at 50, 100, 200 and 400 μL. The pre-formulation is allowed to gel for 2 minutes. 200 μL of D15 is added to each chamber. Three of these slides are prepared for three time points: 0, 2, and 24 hours. The cells are stained with membrane-permeant 3',6'-Di(O-acetyl)-2',7'-bis[N,N-bis(carboxymethyl) aminomethyl] fluorescein, tetraacetoxymethyl ester and membrane-impermeant ethidium homodimer-1, 1 μl/ml propidium iodide. The cells are imaged using brightfield and fluorescence microscopy. Live cells fluoresce green and dead cells fluoresce red. At the 2 hour time point, only one dead cell was observed in multiple field views. One live cell had a punctate cytoplasm. The remaining cells were viable and had typical spheroid morphology in the hydrogel polymer matrix. At the 24 hour time point, more than 95% of the cells were viable.

**Example 18: General Procedure to Determine the Properties of Cells in a Polyglycol-based, Biocompatible Hydrogel Polymer Matrix**

The proliferation rate, viability and structural characteristics of mesenchymal stem cells are evaluated after incorporation with a biocompatible hydrogel polymer matrix.

To measure the rate of proliferation of mesenchymal stem cells, a cell proliferation assay is performed. A biocompatible pre-formulation comprising polyglycol-based compounds and a suitable buffer, as described in Example

14, is prepared. The 100 μl of the pre-formulation is coated on a 24 well plate to give a coating of <5 mm thick. The stem cells are seeded onto the coated plate at various cell densities ($1x10^3$, $5x10^3$, $10x10^3$ and $20x10^3$ cells). Cells are incubated in a growth medium at 37 °C, 5% $CO_2$. For each sample, a CellTiter 96® AQueous Non-Radioactive (MTS) assay is performed at days 2, 7, and 10 after seeding to confirm that the cells are proliferating. The growth medium is removed from each well and replaced with 500μl of fresh medium and incubate at 37 °C for at least 1 hour in 5% $CO_2$. 100μl of MTS reagent is added to each well and incubated at 37 °C for 3 hours, in 5% $CO_2$. The absorbance at 490 nm is measured using a microplate reader and recorded. Wells with the formulation but without any cells are used as blanks. Similarly, only media in the wells without any cells serve as blanks. Each sample reading is obtained by subtracting the blank. The graph of absorbance versus time is plotted. Absorbance is directly proportional to the cell numbers, wherein a significant increase in absorbance indicates cell viability and proliferation. Fold change in proliferation is calculated.

To demonstrate the viability of adult mesenchymal stem cells, a staining assay is performed at days 2, 7, and 10 on cells which are seeded on a coated 24 well plate as described previously in this example. The medium is removed and the cells are washed twice with phosphate saline buffer. A 0.5 ml staining solution comprising a mixture of celcein-am (10 μg/ml) and propidium iodide (100 μg/ml) is added to each well and the plate is incubated for 5-10 minutes at 37 °C. Cells are washed with phosphate saline buffer and immediately imaged. Live cells fluoresce green and dead cells fluoresce red.

To demonstrate that the adult mesenchymal stem cells maintain their structure, a staining assay is performed on the cells which are seeded a coated 24 well plate as described previously in this example. The medium is removed and the cells are washed twice with phosphate buffer. The cells are fixed with 4% paraformaldehyde for 10 minutes at room temperature followed by two washes with phosphate buffer. To the washed cells, cytoplasmic WGA stain (wheat germ agglutinin; 488 green fluorescence) is added and the cells and the cells are incubated for 10 minutes at room temperature. The stain is removed and the cells are washed two times with phosphate buffer. A nuclear TO-PRO-3 iodide stain (red fluorescence) is added to the cells and the cells are incubated for 10 minutes at room temperature. The stain is removed and the cells are washed two times with HBSS buffer. The anti-fade reagent Pro-long gold is added to the cells and the cells are covered with a coverslip. 3D confocal microscopy is performed to visualize the structure and adherence of the cells. In general, the stem cells maintain their physiochemical properties.

**Example 19:** ***Cell Elution from a Polyelycol-based, Biocompatible Hydrogel Polymer Matrix***

A polyglycol-based, biocompatible hydrogel polymer matrix of Example 15 is prepared. Additional polyglycol-based, biocompatible hydrogel polymer matrices are prepared utilizing pre-formulation compounds of *Table 13* and cell. The polymer matrices are weighed and placed in different Falcon tubes. Two ml of buffer/ gm of the polymer matrix are added in the falcon tubes. The falcon tubes are placed in a water bath maintained at 37°C. After 24 hours, buffer is carefully removed and replaced with fresh buffer to maintain a constant volume. The extraction process is repeated until each polymer matrix is dissolved completely. The polymer matrix is dissolved in two weeks.

The elution behavior of the cells with different biocompatible pre-formulation components is tested. Cell elution profiles vary with different biocompatible pre-formulation components. Cells may diffuse while the polymer matrix is maintained, released upon degradation of the polymer matrix or any combination thereof. The composition of the biocompatible pre-formulation components may be selected to control the release of cells at a pre-determined time.

In some instances, the cell-containing polymer matrices described herein further comprise additional components such as buffers, growth factors, antibiotics, or anti-cancer agents. The composition of the biocompatible pre-formulation components and additional components may be varied to control the release of cells and/or the additional components.

In some instances, the cells of any of the cell-containing polymer matrices described in this example may be released from the polymer matrix in a manner dependent on the pore-size of the polymer matrix. In some instances, the cells remain viable after release from the polymer matrix.

**Example 20:** A **Polyglycol-based, Biocompatible Pre-formulation for Disease Treatment**

A polyglycol-based, biocompatible pre-formulation comprising, 0.0125 g 8ARM-AA-20K, 0.0075 g 8ARM-NH2-20k, 0.040 g 4ARM-SGA-20K, mesenchymal stem cells, and a suitable culture medium are combined in the presence of 1.0 mL water. The liquid formulation is delivered via injection directly to a site of tissue damage in the liver. The polyglycol-based, biocompatible pre-formulation mixture polymerizes *in vivo* at the site of delivery to form a polyglycol-based, biocompatible hydrogel polymer matrix at the target site in 4 minutes. The polyglycol-based, biocompatible hydrogel polymer matrix culture medium component is configured to influence the physical, chemical and biological environment surrounding the stem cells during and after administration to a target site.

The polyglycol-based, biocompatible hydrogel polymer matrix is retained at the target site, where the stem cells are released over a period of two weeks. The released stem cells require interaction and integration with the target tissue

through incorporation of appropriate physical and cellular signals. Therefore, the polyglycol-based, biocompatible hydrogel polymer matrix culture medium includes modifying factors, such as biologically active proteins critical for successful tissue generation. The mesenchymal stem cells begin to differentiate at the target site between 7 and 14 days, resulting in improved liver function.

**Example 20:** A **Polyglycol-based, Biocompatible Hydrogel Polymer Matrix for Disease Treatment**

A polyglycol-based, biocompatible hydrogel polymer matrix is prepared by adding 1 mL of water to a pre-formulation comprising, 0.0125 g 8ARM-AA-20K, 0.0075 g 8ARM-NH2-20k, 0.040 g 4ARM-SGA-20K, mesenchymal stem cells, and a suitable culture medium. After gelling is complete, the hydrogel polymer matrix is delivered directly to a site of tissue damage in the liver. The polyglycol-based, biocompatible hydrogel polymer matrix culture medium component is configured to influence the physical, chemical and biological environment surrounding the stem cells during and after administration to the target site in the liver.

The polyglycol-based, biocompatible hydrogel polymer matrix is retained at the target site, where the stem cells are released over a period of two weeks. The released stem cells require interaction and integration with the target tissue through incorporation of appropriate physical and cellular signals. Therefore, the polyglycol-based, biocompatible hydrogel polymer matrix culture medium includes modifying factors, such as biologically active proteins critical for successful tissue generation. The mesenchymal stem cells begin to differentiate at the target site between 7 and 14 days, resulting in improved liver function.

**Example 21:** A **Polyglycol-based, Biocompatible Polymer matrix for Delivery of Growth Factors**

A polyglycol-based, biocompatible pre-formulation comprising, 0.028g 8ARM-AA-20K, 0.012g 8ARM-NH2-20k, 0.08g 4ARM-SGA-20K, growth factors, and a buffer are combined in the presence of 2.5 mL water. The liquid formulation is delivered via injection directly to a site of tissue damage. The polyglycol-based, biocompatible pre-formulation mixture polymerizes *in vivo* at the site of delivery to form a polyglycol-based, biocompatible hydrogel polymer matrix at a target site. The polyglycol-based, biocompatible hydrogel polymer matrix is configured to release the growth factors at the target site. The growth factors are configured to recruit cells from the body to the polymer matrix site, wherein the recruited cells may form tissue upon and throughout the polymer matrix.

An alternative to growth factor incorporation in a polyglycol-based, biocompatible hydrogel polymer matrix is to integrate DNA plasmids encoding a gene and mammalian promoter into the polymer matrix. Delivery of the polyglycol-based, biocompatible hydrogel polymer matrix with the DNA programs local cells to produce their own growth factors.

**Example 22: Pore Size Determination**

The pore diameters are estimated from the molecular weight per arm of the combined components. The pore diameter is calculated based on the number of PEG units per arm and a carbon-carbon-carbon bond length of 0.252 nm with a 110° bond angle. This assumes a fully extended chain that accounts for bonding angles and complete reactivity of all functional end groups to form the pore network. The pore diameter is further modified by a correlation relating the pore size to the inverse of the biocompatible hydrogel swelling ratio:

$$\xi \approx L * (V_p / V_s)^{-1/3} \quad \textbf{(Equation 1)}$$

where $V_p$ is the volume of polymer, $V_s$ is the volume of the swollen gel, L is the calculated pore diameter, and $\xi$ is the swollen pore diameter. Based on equilibrium swelling experiments, the ratio of $V_p$ to $V_s$ is estimated to be around 0.5.

For the case of multi-component mixtures with a reactive ester, the weighted average of each component with the ester is used. For example, the pore sizes obtained from 4ARM-20k-AA with 4ARM-20k-SGA are averaged with the pore sizes obtained from 8ARM-20k-NH2 with 4ARM-20k-SGA for polymers comprised of 4ARM-20k-AA and 8ARM-20k-NH2 with 4ARM-20k-SGA.

## Claims

1. A polyglycol-based biocompatible hydrogel polymer matrix comprising a hydrogel polymer comprising at least one first monomeric unit bound through at least one amide linkage to at least one second monomeric unit, wherein the polymer forms the matrix that encapsulates:

(a) at least one cell; and
(b) a culture medium which supports the growth of the at least one cell;

wherein the hydrogel polymer is derived from the first monomeric unit of 8ARM PEG Acetate Amine (8ARM-20k-AA) and 8ARM PEG Amine (8ARM-20k-NH2); the second monomeric unit of 4ARM PEG Succinimidyl Glutaramide (4ARM-20k-SGA) ;
a phosphate buffer and a viscosity enhancing agent;
wherein the fully synthetic, polyglycol-based biocompatible hydrogel polymer matrix provides controlled release of the at least one cell, when implanted at a target site in an animal's body, to the target site of the animal's body.

**2.** The polyglycol-based biocompatible hydrogel polymer matrix of claim 1, wherein the 8ARM PEG Acetate Amine is 8ARM PEG Acetate Amine (hexaglycerol) HC1 Salt, MW 20000 or 8ARM PEG Acetate Amine (hexaglycerol) TFA Salt, MW 20000.

**3.** The polyglycol-based biocompatible hydrogel polymer matrix of any one of claims 1-2, wherein the cell is selected from a mammalian cell, insect cell, protozoal cell, bacterial cell, viral cell, or fungal cell; and optionally, wherein the mammalian cell is a stem cell.

**4.** The polyglycol-based biocompatible hydrogel polymer matrix of any one of claims 1-3, wherein the culture medium comprises a growth factor.

**5.** The polyglycol-based biocompatible hydrogel polymer matrix of any of the preceding claims, wherein the ratio of 8ARM-20k-AA to 8ARM-20k-NH2 is 1:1, 70:30, or 75:25.

**6.** The fully synthetic, polyglycol-based biocompatible hydrogel polymer matrix of any one of claims 1-5, wherein the animal is a human.

**7.** The fully synthetic, polyglycol-based biocompatible hydrogel polymer matrix of any one of claims 1-6, wherein the polyglycol-based biocompatible hydrogel polymer matrix is bioabsorbed within about 14 to 180 days.

**8.** The fully synthetic, polyglycol-based biocompatible hydrogel polymer matrix of any one of claims 1-7, wherein the controlled release of the at least one cell to the target site of the animal's body comprises diffusion of the at least one cell from the polyglycol-based biocompatible hydrogel polymer matrix; or wherein the controlled release of the at least one cell to the target site of the animal's body is at least partially through degradation and bioabsorption of the polyglycol-based biocompatible hydrogel polymer matrix.

**9.** A fully synthetic polyglycol-based biocompatible pre-formulation, comprising:

(a) at least one fully synthetic polyglycol-based first composition comprising 8ARM PEG Acetate Amine (8ARM-20k-AA) and 8ARM PEG Amine (8ARM-20k-NH2);
(b) at least one fully synthetic polyglycol-based second composition comprising 4ARM PEG Succinimidyl Glutaramide (4ARM-20k-SGA);
(c) a phosphate buffer;
(d) a viscosity enhancing agent;
(e) at least one cell; and
(f) a culture medium that supports growth of the at least one cell;

wherein the polyglycol-based biocompatible pre-formulation at least in part polymerizes and/or gels to form a polyglycol-based biocompatible hydrogel polymer matrix encapsulating the cell in the presence of water.

**10.** The fully synthetic, polyglycol-based biocompatible pre-formulation of claim 9, wherein the cell is a mammalian cell or stem cell; and optionally,
wherein the culture medium comprises a buffer.

**11.** The fully synthetic, polyglycol-based biocompatible pre-formulation of any one of claims 9 and 10, wherein the polyglycol-based biocompatible pre-formulation gels to form a polyglycol-based biocompatible hydrogel polymer matrix in between about 20 seconds and 10 minutes.

12. A fully synthetic, polyglycol based biocompatible hydrogel polymer matrix for use in a method of treating a disease, wherein the polyglycol-based biocompatible hydrogel polymer matrix is any one of claims 1-8.

13. The polyglycol-based biocompatible hydrogel polymer matrix of claim 1, wherein the viscosity enhancing agent is HPMC.

14. The fully synthetic polyglycol-based biocompatible pre-formulation of claim 9, wherein the viscosity enhancing agent is HPMC.

**Patentansprüche**

1. Polyglykolbasierte biologisch kompatible Hydrogelpolymermatrix, umfassend ein Hydrogelpolymer mit mindestens einer ersten Monomereinheit, die über mindestens eine Amidbindung an mindestens eine zweite Monomereinheit gebunden ist, wobei das Polymer die Matrix bildet, die Folgendes verkapselt:

   (a) mindestens eine Zelle und
   (b) ein Kulturmedium, das das Wachstum der mindestens einen Zelle fördert,

   wobei sich das Hydrogelpolymer von der ersten Monomereinheit von 8ARM PEG-Acetatamin (8ARM-20k-AA) und 8ARM PEG-Amin (8ARM-20k-NH2), der zweiten Monomereinheit von 4ARM PEG-Succinimidylglutaramid (4ARM-20k-SGA), einem Phosphatpuffer und einem viskositätserhöhenden Mittel ableitet,
   wobei die vollsynthetische polyglykolbasierte biologisch kompatible Hydrogelpolymermatrix bei einer Implantation an einer Zielstelle in einem Tierkörper für die kontrollierte Freisetzung der mindestens einen Zelle an der Zielstelle des Tierkörpers sorgt.

2. Polyglykolbasierte biologisch kompatible Hydrogelpolymermatrix nach Anspruch 1, wobei es sich bei dem 8ARM PEG-Acetatamin um ein 8ARM PEG-Acetatamin (Hexaglycerin)-HCl-Salz, MW 20000, oder 8ARM PEG-Acetatamin(Hexaglycerin)-TFA-Salz, MW 20000, handelt.

3. Polyglykolbasierte biologisch kompatible Hydrogelpolymermatrix nach einem der Ansprüche 1-2, wobei die Zelle aus einer Säugetierzelle, Insektenzelle, Protozoenzelle, Bakterienzelle, Virenzelle oder Pilzzelle ausgewählt ist und wobei es sich bei der Säugetierzelle gegebenenfalls um eine Stammzelle handelt.

4. Polyglykolbasierte biologisch kompatible Hydrogelpolymermatrix nach einem der Ansprüche 1-3, wobei das Kulturmedium einen Wachstumsfaktor umfasst.

5. Polyglykolbasierte biologisch kompatible Hydrogelpolymermatrix nach einem der vorhergehenden Ansprüche, wobei das Verhältnis von 8ARM-20k-AA zu 8ARM-20k-NH2 1:1, 70:30 oder 75:25 beträgt.

6. Vollsynthetische polyglykolbasierte biologisch kompatible Hydrogelpolymermatrix nach einem der Ansprüche 1-5, wobei es sich bei dem Tier um einen Menschen handelt.

7. Vollsynthetische polyglykolbasierte biologisch kompatible Hydrogelpolymermatrix nach einem der Ansprüche 1-6, wobei die polyglykolbasierte biologisch kompatible Hydrogelpolymermatrix innerhalb von etwa 14 bis 180 Tagen bioabsorbiert wird.

8. Vollsynthetische polyglykolbasierte biologisch kompatible Hydrogelpolymermatrix nach einem der Ansprüche 1-7, wobei die kontrollierte Freisetzung der mindestens einen Zelle an der Zielstelle des Tierkörpers die Diffusion der mindestens einen Zelle aus der polyglykolbasierten biologisch kompatiblen Hydrogelpolymermatrix umfasst oder wobei die kontrollierte Freisetzung der mindestens einen Zelle an der Zielstelle des Tierkörpers mindestens teilweise durch Abbau und Bioabsorption der polyglykolbasierten biologisch kompatiblen Hydrogelpolymermatrix erfolgt.

9. Vollsynthetische polyglykolbasierte biologisch kompatible Vorformulierung, umfassend:

   (a) mindestens eine vollsynthetische polyglykolbasierte erste Zusammensetzung, umfassend 8ARM PEG-Acetatamin (8ARM-20k-AA) und 8ARM PEG-Amin (8ARM-20k-NH2),
   (b) mindestens eine vollsynthetische polyglykolbasierte zweite Zusammensetzung, umfassend 4ARM PEG-Succinimidylglutaramid (4ARM-20k-SGA),
(c) einen Phosphatpuffer,
(d) ein viskositätserhöhendes Mittel,
(e) mindestens eine Zelle und
(f) ein Kulturmedium, das das Wachstum der mindestens einen Zelle fördert,

wobei die polyglykolbasierte biologisch kompatible Vorformulierung in Gegenwart von Wasser zumindest teilweise polymerisiert und/oder geliert, unter Bildung einer polyglykolbasierten biologisch kompatiblen Hydrogelpolymermatrix, die die Zelle verkapselt.

**10.** Vollsynthetische polyglykolbasierte biologisch kompatible Vorformulierung nach Anspruch 9, wobei es sich bei der Zelle um eine Säugetierzelle oder Stammzelle handelt und wobei das Kulturmedium gegebenenfalls einen Puffer umfasst.

**11.** Vollsynthetische polyglykolbasierte biologisch kompatible Vorformulierung nach einem der Ansprüche 9 und 10, wobei die polyglykolbasierte biologisch kompatible Vorformulierung innerhalb von zwischen etwa 20 Sekunden und 10 Minuten unter Bildung einer polyglykolbasierten biologisch kompatiblen Hydrogelpolymermatrix geliert.

**12.** Vollsynthetische polyglykolbasierte biologisch kompatible Hydrogelpolymermatrix zur Verwendung in einem Verfahren zur Behandlung einer Krankheit, wobei es sich bei der polyglykolbasierten biologisch kompatiblen Hydrogelpolymermatrix um eine der Ansprüche 1-8 handelt.

**13.** Polyglykolbasierte biologisch kompatible Hydrogelpolymermatrix nach Anspruch 1, wobei es sich bei dem viskositätserhöhenden Mittel um HPMC handelt.

**14.** Vollsynthetische polyglykolbasierte biologisch kompatible Vorformulierung nach Anspruch 9, wobei es sich bei dem viskositätserhöhenden Mittel um HPMC handelt.

## Revendications

**1.** Matrice de polymère d'hydrogel biocompatible à base de polyglycol comprenant un polymère d'hydrogel comprenant au moins un premier motif monomérique lié par l'intermédiaire d'au moins une liaison amide à au moins un deuxième motif monomérique, le polymère formant la matrice qui encapsule :

(a) au moins une cellule ; et
(b) un milieu de culture qui supporte la croissance de l'au moins une cellule ;

le polymère d'hydrogel étant issu du premier motif monomérique de 8ARM PEG Acétate Amine (8ARM-20k-AA) et 8ARM PEG Amine (8ARM-20k-$NH_2$) ; le deuxième motif monomérique de 4ARM PEG Glutaramide de Succinimidyle (4ARM-20k-SGA) ;
un tampon phosphate et un agent d'augmentation de la viscosité ;
la matrice de polymère d'hydrogel biocompatible à base de polyglycol, entièrement synthétique fournissant une libération contrôlée de l'au moins une cellule, lorsqu'elle est implantée au niveau d'un site cible dans un corps d'animal, au site cible du corps d'animal.

**2.** Matrice de polymère d'hydrogel biocompatible à base de polyglycol selon la revendication 1, l'8ARM PEG Acétate Amine étant l'8ARM PEG Acétate Amine (hexaglycérol) sel de HCl, poids moléculaire 20 000 ou l'8ARM PEG Acétate Amine (hexaglycérol) sel de TFA, poids moléculaire 20 000.

**3.** Matrice de polymère d'hydrogel biocompatible à base de polyglycol selon l'une quelconque des revendications 1 et 2, la cellule étant choisie parmi une cellule de mammifère, une cellule d'insecte, une cellule de protozoaire, une cellule bactérienne, une cellule virale, ou une cellule fongique ; et éventuellement la cellule de mammifère étant une cellule souche.

**4.** Matrice de polymère d'hydrogel biocompatible à base de polyglycol selon l'une quelconque des revendications 1 à 3, le milieu de culture comprenant un facteur de croissance.

**5.** Matrice de polymère d'hydrogel biocompatible à base de polyglycol selon l'une quelconque des revendications précédentes, le rapport d'8ARM-20k-AA à 8ARM-20k-$NH_2$ étant de 1:1, 70:30, ou 75:25.

**6.** Matrice de polymère d'hydrogel biocompatible à base de polyglycol, entièrement synthétique selon l'une quelconque des revendications 1 à 5, l'animal étant un humain.

**7.** Matrice de polymère d'hydrogel biocompatible à base de polyglycol, entièrement synthétique selon l'une quelconque des revendications 1 à 6, la matrice de polymère d'hydrogel biocompatible à base de polyglycol étant bioabsorbée en l'espace d'environ 14 à 180 jours.

**8.** Matrice de polymère d'hydrogel biocompatible à base de polyglycol, entièrement synthétique selon l'une quelconque des revendications 1 à 7, la libération contrôlée d'au moins une cellule au site cible du corps d'animal comprenant la diffusion de l'au moins une cellule de la matrice de polymère d'hydrogel biocompatible à base de polyglycol ; ou la libération contrôlée de l'au moins une cellule au site cible du corps d'animal étant réalisée au moins partiellement par dégradation et bioabsorption de la matrice de polymère d'hydrogel biocompatible à base de polyglycol.

**9.** Pré-formulation biocompatible à base de polyglycol, entièrement synthétique, comprenant :

(a) au moins une première composition à base de polyglycol, entièrement synthétique comprenant 8ARM PEG Acétate Amine (8ARM-20k-AA) et 8ARM PEG Amine (8ARM-20k-$NH_2$) ;
(b) au moins une deuxième composition à base de polyglycol, entièrement synthétique comprenant 4ARM PEG Glutaramide de Succinimidyle (4ARM-20k-SGA) ;
(c) un tampon phosphate ;
(d) un agent d'augmentation de la viscosité ;
(e) au moins une cellule ; et
(f) un milieu de culture qui supporte la croissance de l'au moins une cellule ;

la pré-formulation biocompatible à base de polyglycol polymérisant au moins en partie et/ou figeant pour former une matrice de polymère d'hydrogel biocompatible à base de polyglycol encapsulant la cellule en présence d'eau.

**10.** Pré-formulation biocompatible à base de polyglycol, entièrement synthétique selon la revendication 9, la cellule étant une cellule de mammifère ou une cellule souche ; et éventuellement, le milieu de culture comprenant un tampon.

**11.** Pré-formulation biocompatible à base de polyglycol, entièrement synthétique selon l'une quelconque des revendications 9 et 10, la pré-formulation biocompatible à base de polyglycol figeant pour former une matrice de polymère d'hydrogel biocompatible à base de polyglycol en un temps compris entre environ 20 secondes et 10 minutes.

**12.** Matrice de polymère d'hydrogel biocompatible à base de polyglycol, entièrement synthétique pour une utilisation dans un procédé de traitement d'une maladie, la matrice de polymère d'hydrogel biocompatible à base de polyglycol étant l'une quelconque parmi les revendications 1 à 8.

**13.** Matrice de polymère d'hydrogel biocompatible à base de polyglycol selon la revendication 1, l'agent d'augmentation de la viscosité étant l'HPMC.

**14.** Pré-formulation biocompatible à base de polyglycol, entièrement synthétique selon la revendication 9, l'agent d'augmentation de la viscosité étant l'HPMC.

**Figure 1**

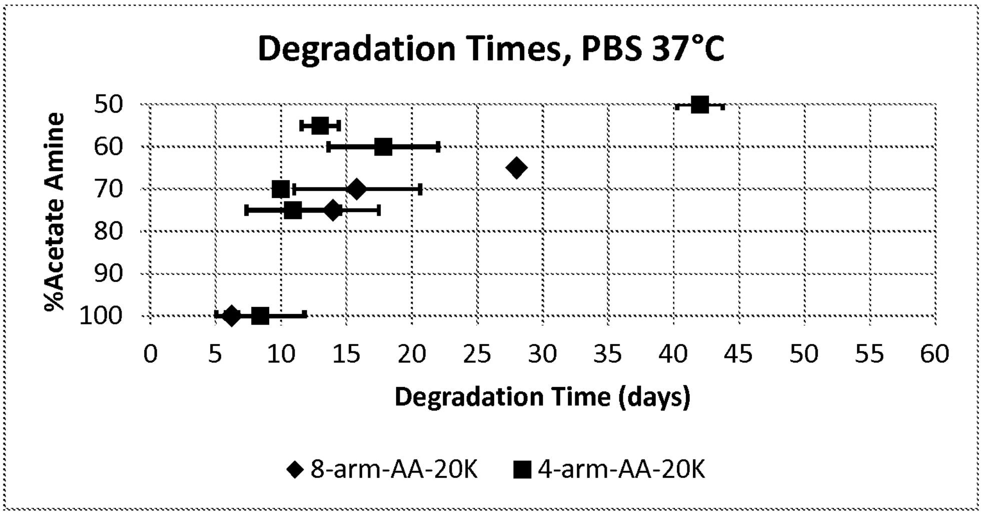


**Figure 2**

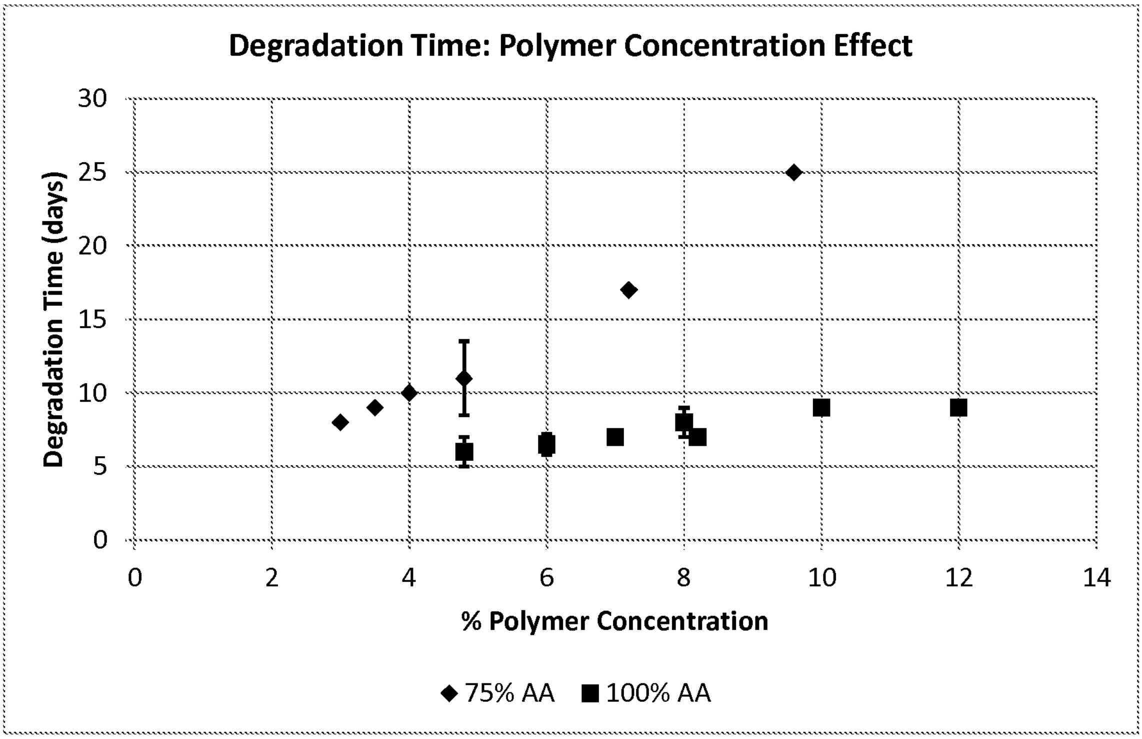

Figure 3

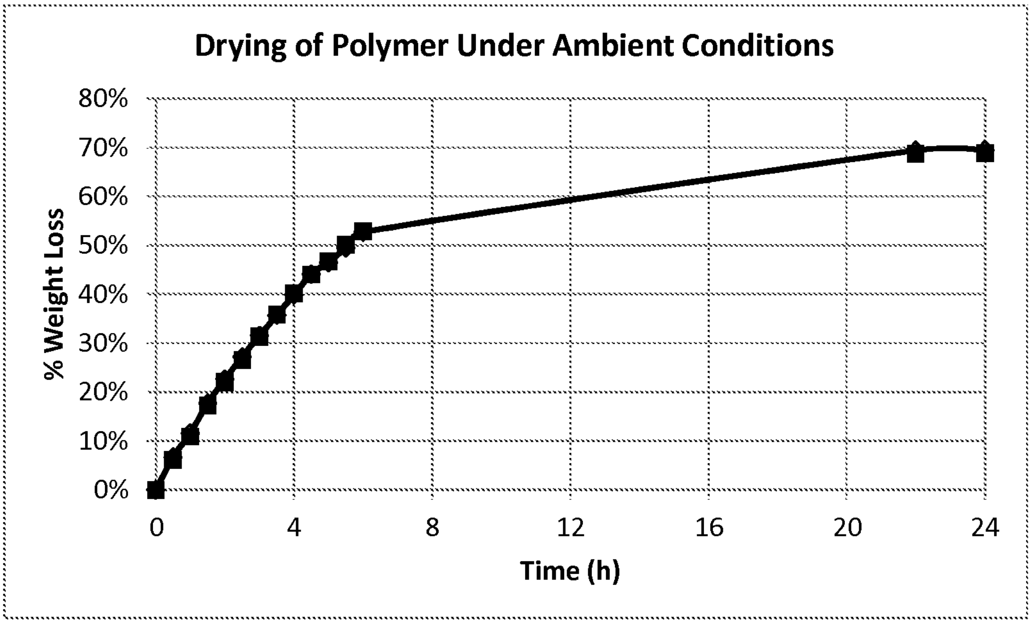


Figure 4

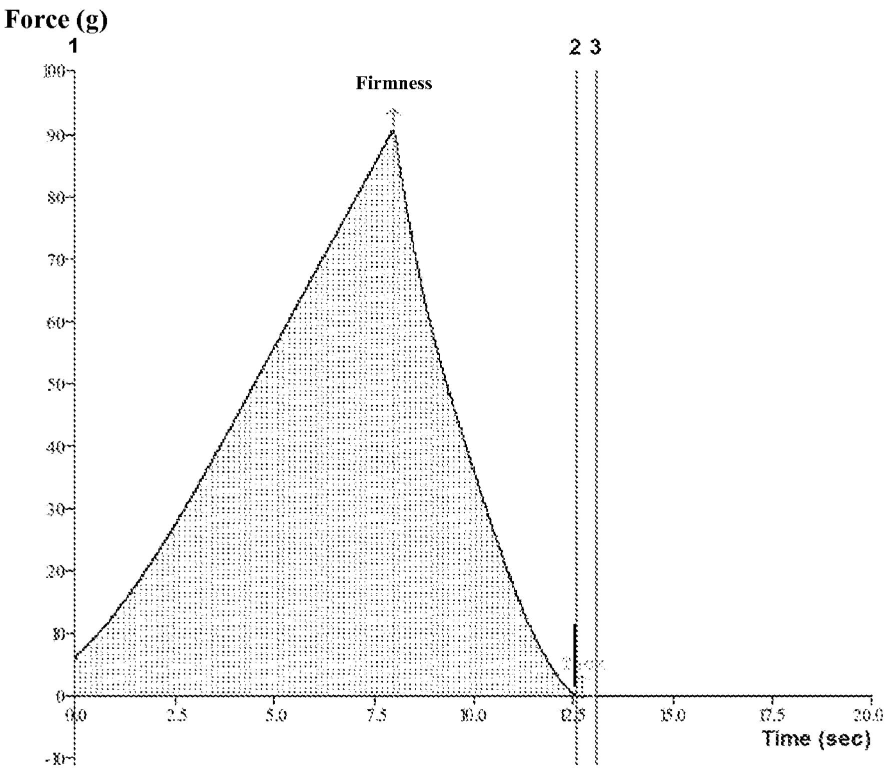

**Figure 5**

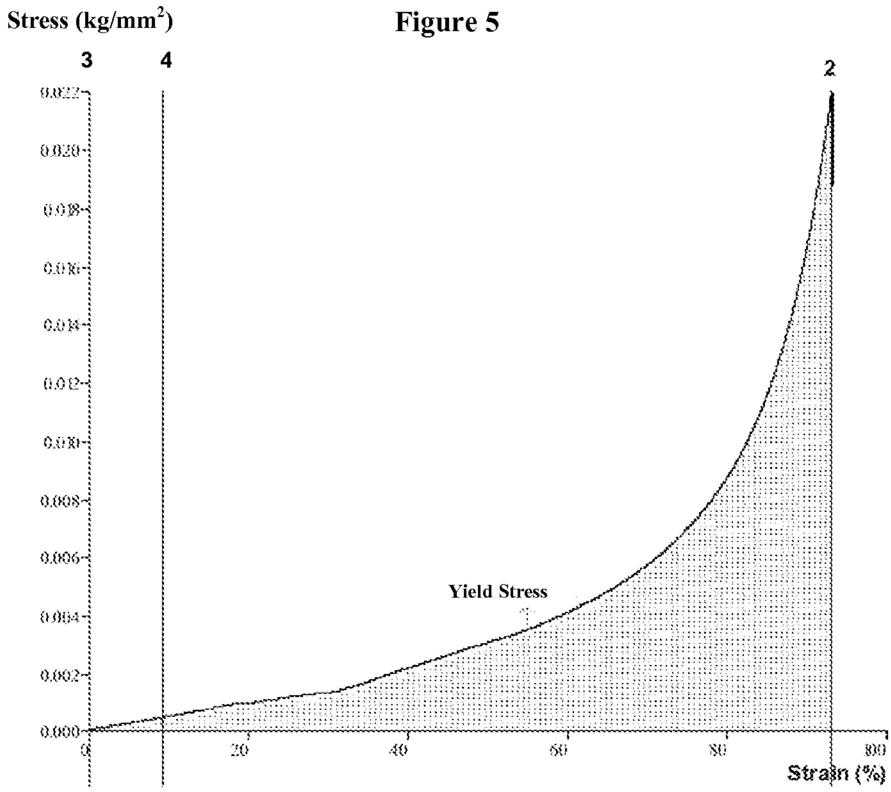


**Figure 6**

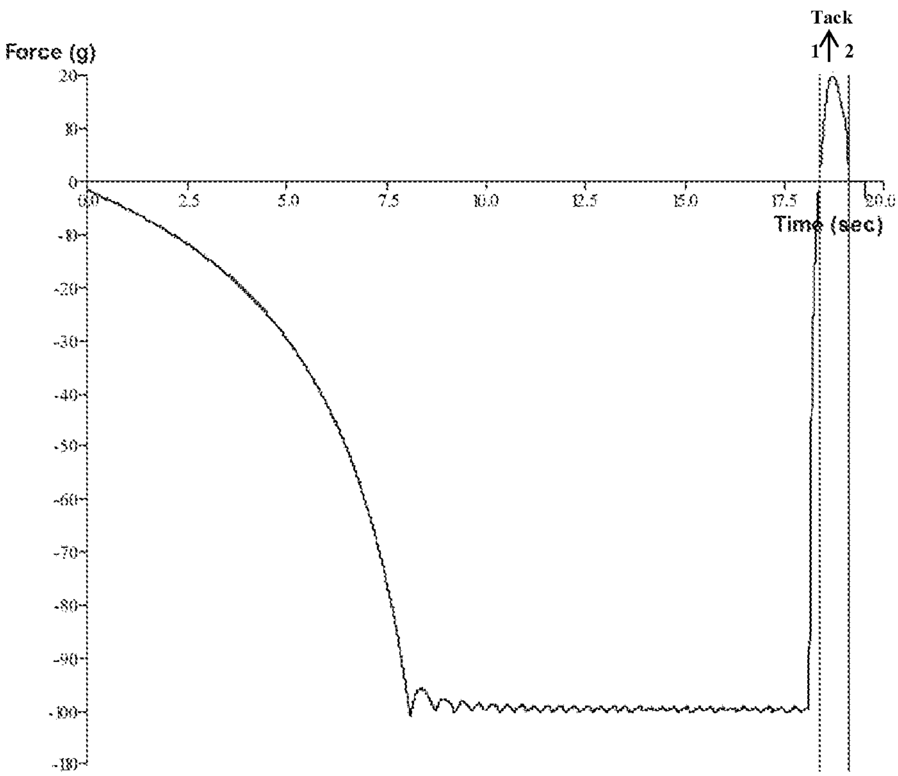

Figure 7

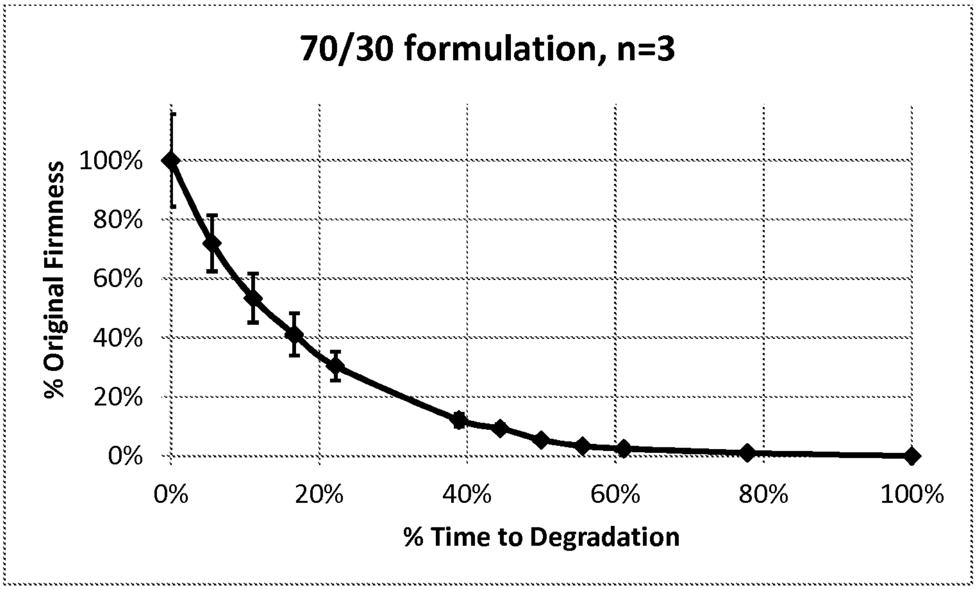


Figure 8

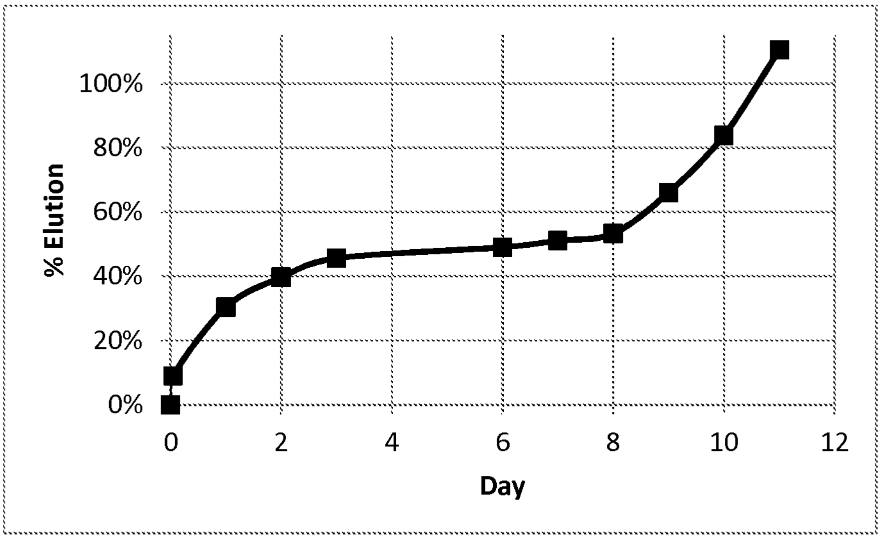

Figure 9

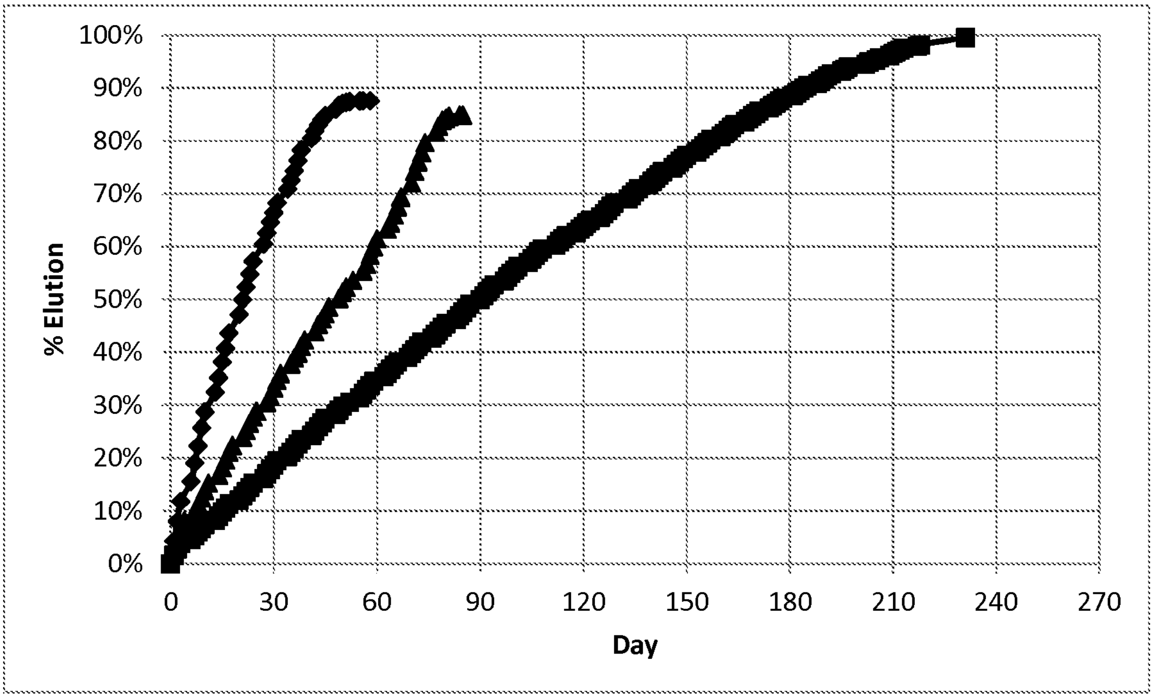
100%
90%
80%
70%
60%
50%
40%
30%
20%
10%
0%
% Elution
0
30
60
90
120
150
180
210
240
270
Day

Figure 10

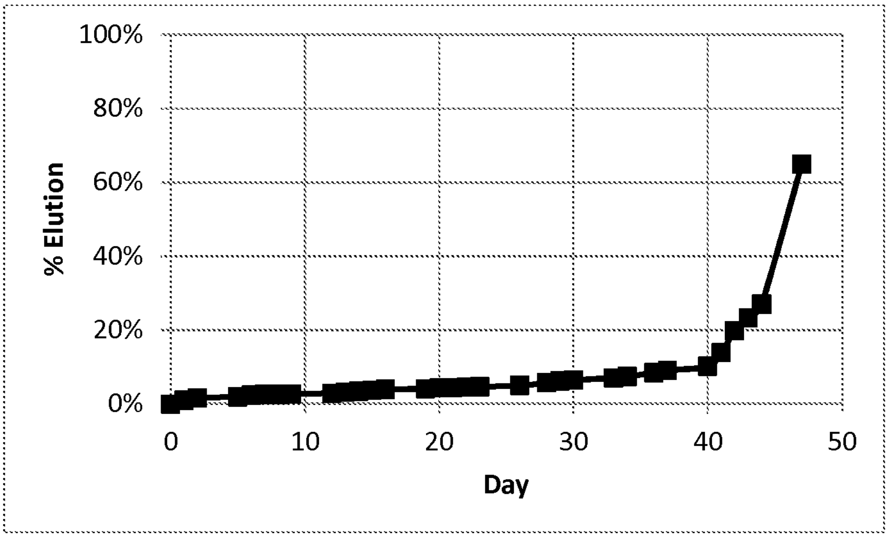


Figure 11

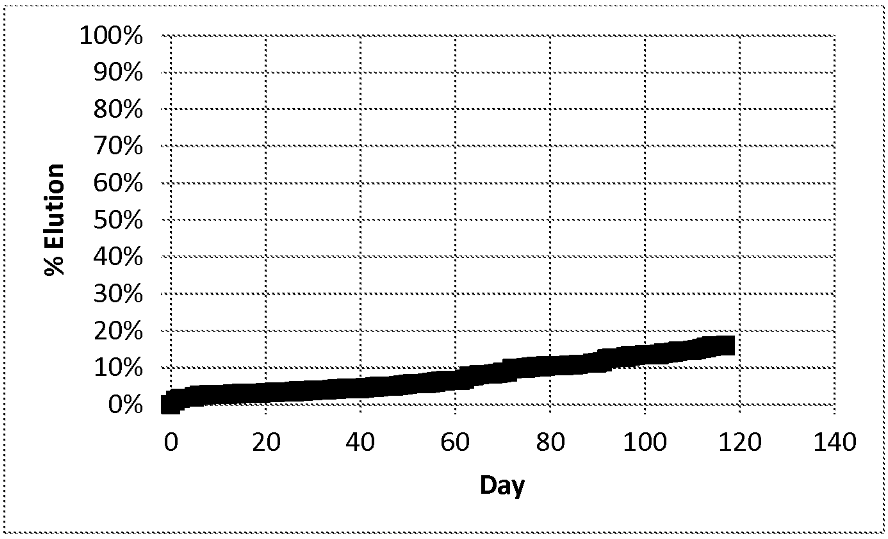

Figure 12A

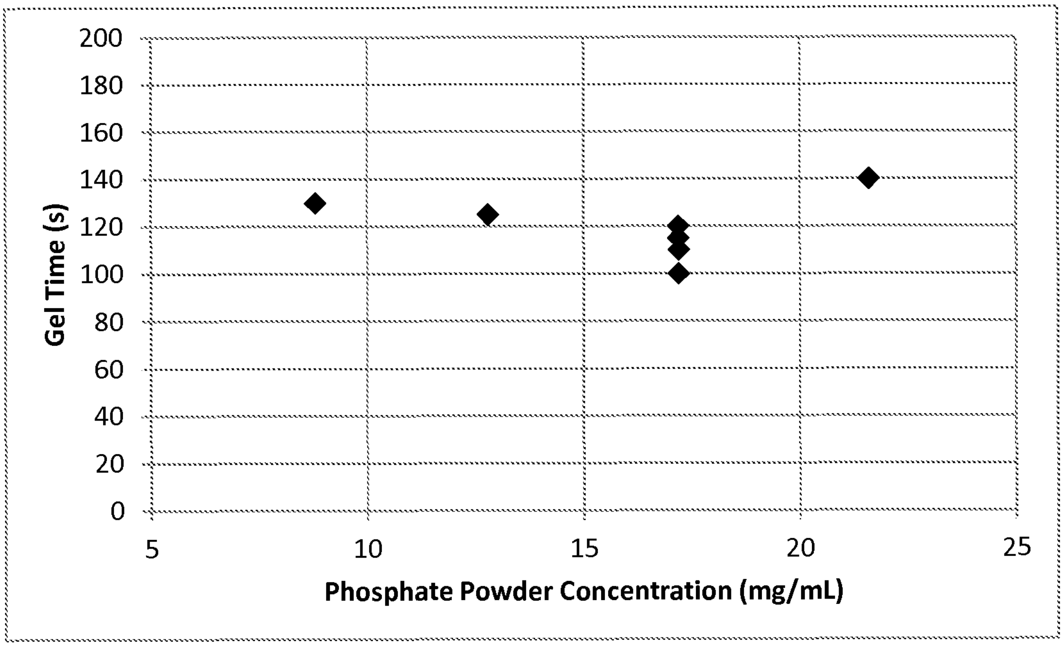


Figure 12B

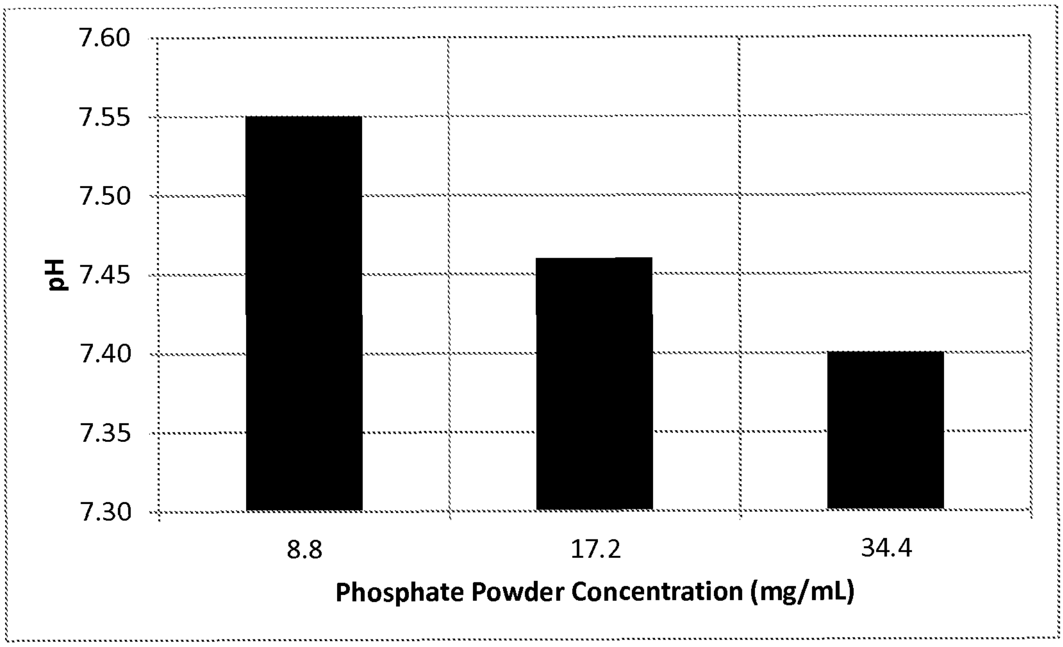

Figure 13A

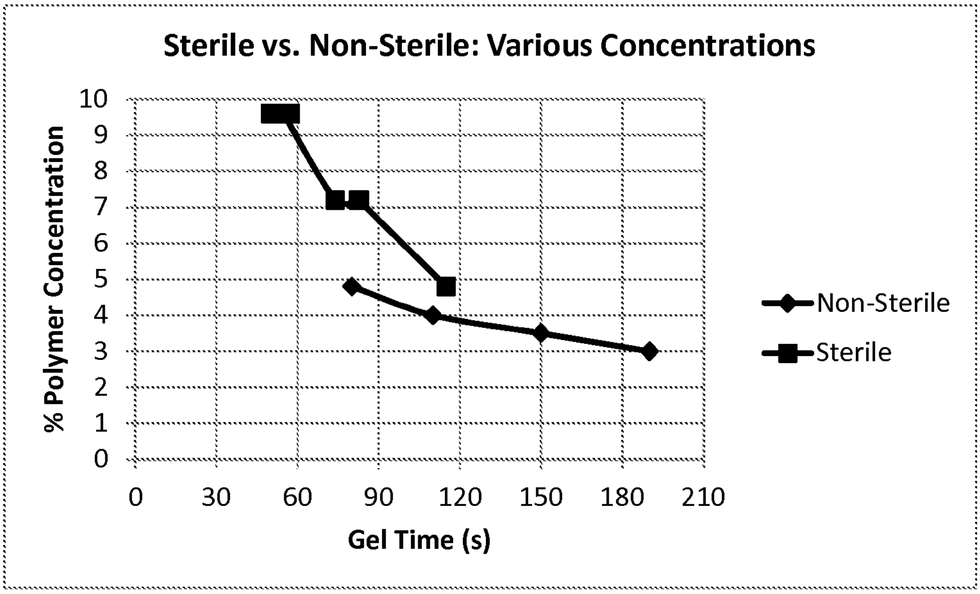


Figure 13B

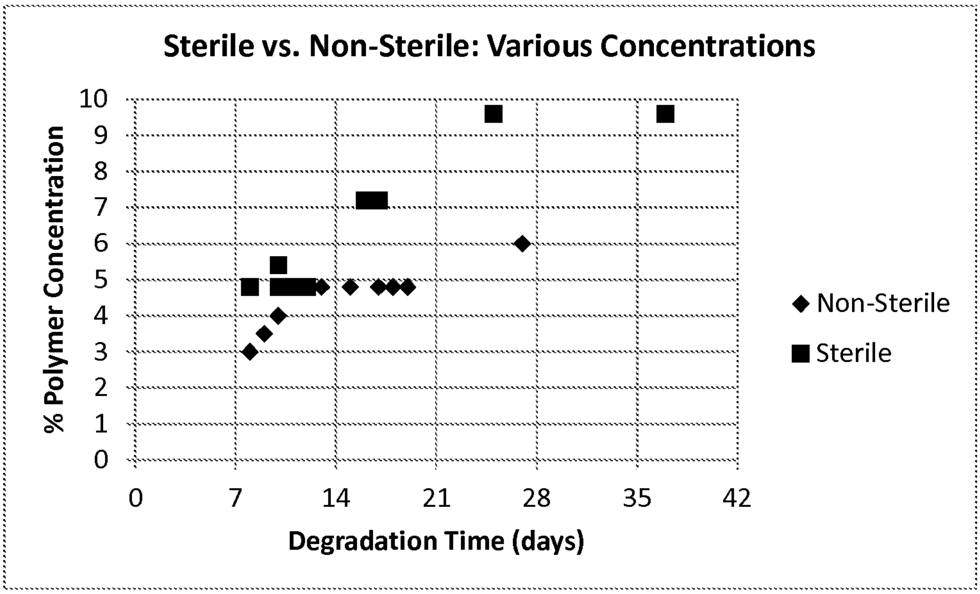

**Figure 14**

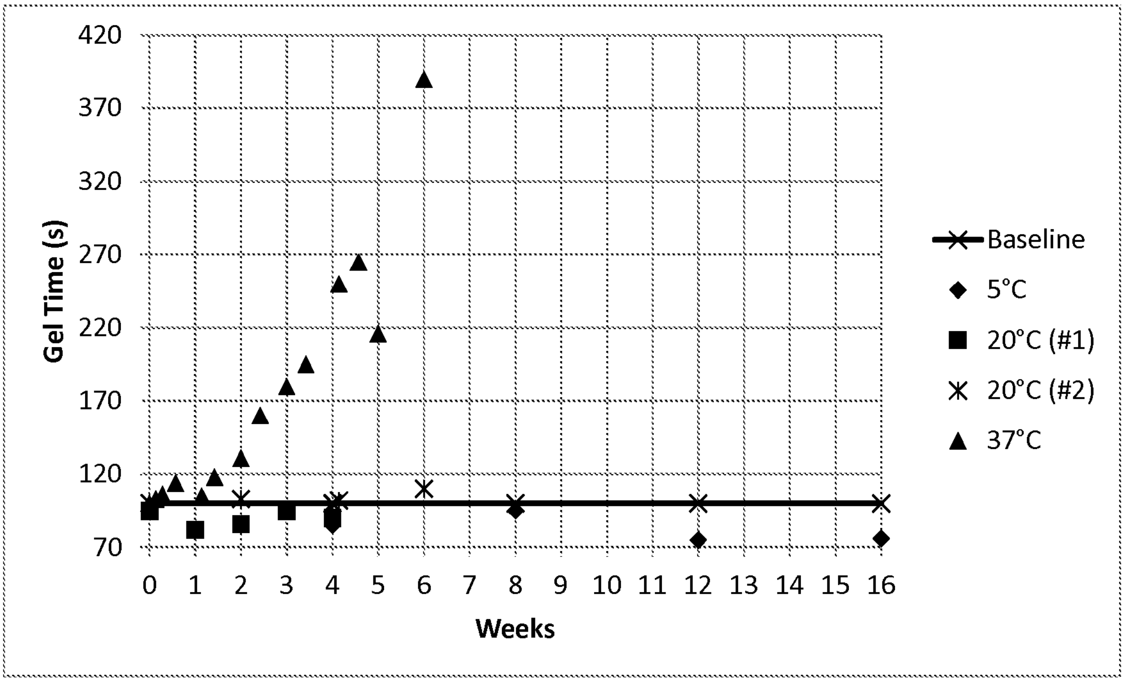

420
370
320
270
220
170
120
70
Gel Time (s)
0 1 2 3 4 5 6 7 8 9 10 11 12 13 14 15 16
Weeks
Baseline
5°C
20°C (#1)
20°C (#2)
37°C

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2011140519 A
- US 2001003125 A